# EUROPEAN PATENT SPECIFICATION

(11) **EP 4 505 470 B1**
(45) Date of publication and mention of the grant of the patent: **25.02.2026**
(21) Application number: 23716567.5
(22) Date of filing: 04.04.2023
(51) Int. Cl.: G16B 40/30, G16B 25/10

(54) **ANALYSIS OF TUMOUR SAMPLES**
ANALYSE VON TUMORPROBEN
ANALYSE D'ÉCHANTILLONS DE TUMEURS

(30) Priority: 05.04.2022 EP 22166809
(43) Date of publication of application: 12.02.2025
(73) Proprietor: F. Hoffmann-La Roche AG, 4070 Basel (CH)
(72) Inventor: AKTAS, Said, 8952 Zürich (CH); MARUYAMA, Toru, 4070 Basel (CH); POESCHL, Daniel Johannes, 8952 Zürich (CH)
(74) Representative: Mewburn Ellis LLP
(86) International application number: PCT/EP2023/058865
(87) International publication number: WO 2023/194392

(56) References cited:
- TORROJA CARLOS ET AL: "Digitaldlsorter: Deep-Learning on scRNA-Seq to Deconvolute Gene Expression Data", FRONTIERS IN GENETICS, vol. 10, 25 October 2019 (2019-10-25), XP055960261, DOI: 10.3389/fgene.2019.00978
- RASHID SABRINA ET AL: "Dhaka: variational autoencoder for unmasking tumor heterogeneity from single cell genomic data", vol. 37, no. 11, 12 July 2021 (2021-07-12), GB, pages 1535 - 1543, XP055960250, ISSN: 1367-4803, Retrieved from the Internet <URL:https://watermark.silverchair.com/btz095.pdf?token=AQECAHi208BE49Ooan9kkhW_Ercy7Dm3ZL_9Cf3qfKAc485ysgAAAvowggL2BgkqhkiG9w0BBwagggLnMIIC4wIBADCCAtwGCSqGSIb3DQEHATAeBglghkgBZQMEAS4wEQQMFpn9YFnA-Itbf6V3AgEQgIICrULYX8tup-amufpRlVvBxxxb6I7um_yVqlHamSkw8KCDxPsCL3Tx5wyUMSS9hvQNnPDX2fhnJ0OcitEz75n5nnRayedBR> DOI: 10.1093/bioinformatics/btz095
- WANG JINGSHU ET AL: "Data denoising with transfer learning in single-cell transcriptomics", NATURE METHODS, NATURE PUBLISHING GROUP US, NEW YORK, vol. 16, no. 9, 30 August 2019 (2019-08-30), pages 875 - 878, XP036875276, ISSN: 1548-7091, [retrieved on 20190830], DOI: 10.1038/S41592-019-0537-1
- CHEN SUJUN ET AL: "Single-cell analysis reveals transcriptomic remodellings in distinct cell types that contribute to human prostate cancer progression", NATURE CELL BIOLOGY, vol. 23, no. 1, January 2021 (2021-01-01), pages 87 - 98, XP037375544, ISSN: 1465-7392, DOI: 10.1038/S41556-020-00613-6
- LOTFOLLAHI MOHAMMAD ET AL: "Mapping single-cell data to reference atlases by transfer learning", NATURE BIOTECHNOLOGY, NATURE PUBLISHING GROUP US, NEW YORK, vol. 40, no. 1, 30 August 2021 (2021-08-30), pages 121 - 130, XP037667064, ISSN: 1087-0156, [retrieved on 20210830], DOI: 10.1038/S41587-021-01001-7
- MARIO FLORES ET AL: "Deep learning tackles single-cell analysis A survey of deep learning for scRNA-seq analysis", ARXIV.ORG, CORNELL UNIVERSITY LIBRARY, 201 OLIN LIBRARY CORNELL UNIVERSITY ITHACA, NY 14853, 25 September 2021 (2021-09-25), XP091058788
- QIAN JUNBIN ET AL: "A pan-cancer blueprint of the heterogeneous tumor microenvironment revealed by single-cell profiling", CELL RESEARCH, SPRINGER SINGAPORE, SINGAPORE, vol. 30, no. 9, 19 June 2020 (2020-06-19), pages 745 - 762, XP037234467, ISSN: 1001-0602, [retrieved on 20200619], DOI: 10.1038/S41422-020-0355-0

## Description

### Field of the disclosure

The present invention relates to methods for analysing tumour samples, using single cell gene expression profiles for cells in tumour samples. In particular, the present invention relates to the use of deep learning models to identify the cell type of cells from their gene expression profile, to methods for analysing single cell RNA sequencing data, to methods for providing a prognostic, therapeutic recommendation, patient selection, drug target identification and biomarker identification using such methods, and to related systems and devices.

### Background

Cancer is a highly complex and individual disease. While it has become apparent that the interaction between tumour cells and their microenvironment including stromal and immune cells plays a crucial part in disease aetiology, prognosis and response to therapy, the tumour microenvironment (TME) remains incompletely understood, partially due to inter and intra cancer heterogeneity.

In recent years, single cell RNA sequencing (scRNA-seq) has emerged as a new technology that provides high-throughput expression profiles of individual cells, providing an opportunity to overcome the challenges in studying the unique functions of individual cells in heterogeneous biological specimen, and greatly enhancing our ability to understand human diseases. However, accurate assignment of the types of the cells in a specimen is a critical step in any single cell data analysis. Traditionally, immunostaining techniques of the cell-type-specific surface marker proteins have been used to profile the composition of a specimen. Such cell type assignments, which can be obtained using technologies such as flow cytometry combined with labelled antibodies targeting surface marker proteins, are typically considered as a "gold standard". However, these methods are limited at least in that they require a priori knowledge about the epitopes of cell surface markers and are typically restricted to markers that are expressed on the cell surface, limiting the genes that can be used as cell type markers.

Studies have shown that there is usually a lack of correlation between the transcript and protein levels of the cell surface markers commonly used to profile immune cells. Therefore, the information obtained at the transcript level cannot be used as a direct equivalent of the information traditionally used at the protein level. Multiple methods for cell-type assignment using scRNA-seq data have been proposed. A common strategy for modelling the cellular heterogeneity in scRNA-seq data relies on clustering approaches using the mRNA expression levels of pre-defined, curated panels of cell-type markers to assign cell types to clusters. Deep learning based methods which can learn from large reference datasets have been proposed for cell type classification of query data (see e.g. Lotfollahi et al., 2022), and these have been demonstrated for the analysis of large multi-organ cell atlases and the mapping of disease samples to such multi-organ atlases.

However, no satisfactory solution for the analysis of single cell RNA sequencing has been proposed which enables to entangle the complexity in the tumour microenvironment.

Torroja and Sanchez-Cabo ("Digitaldlsorter: Deep-Learning on scRNA-Seq to Deconvolute Gene Expression Data", FRONTIERS IN GENETICS, 25 October 2019) present a deep learning based method to enumerate and quantify the immune infiltration in colorectal and breast cancer bulk RNA-Seq samples starting from scRNA-Seq.

### Summary of the disclosure

Broadly, the present inventors used deep learning models developed for the analysis of single cell RNA sequencing data to analyse the tumour microenvironment from a custom pan-cancer data set. For this purpose they developed a new approach for the separation of malignant cells from healthy cells, and for the identification of microenvironment cells including rare cell types by aggregation of data from purified samples, batch effect removal and improved cell type annotation in latent space. They demonstrated the reproducibility of the resulting annotation using a semi-supervised deep learning approach developed to assign cell type labels to unannotated data from annotated cells, providing a cell atlas enabling automated cell type annotation of novel studies in a pan cancer manner via architecture surgery (Lotfollahi et al., 2022). They further demonstrated that the latent variable representations learned according to their process enabled the generation of novel insights into the pan cancer tumour microenvironment (TME), which can be used for query mapping and TME based patient stratification for personalised medicine.

Accordingly, a first aspect provides a method of analysing a tumour sample comprising tumour cells and immune cells, the method comprising: (a) obtaining single cell gene expression profiles for a plurality of cells from the tumour sample, the single cell gene expression profiles comprising gene expression measurements for a set of genes; (b) using a deep learning model to identify a respective latent variable representation of the single cell gene expression profiles in the sample; and (c) identifying a respective one of one or more latent space clusters of cells that the cells in the sample belong to, wherein the clusters of cells correspond to cells from different cell types and wherein the one or more clusters of cells comprise at least a cluster corresponding to tumour cells and one or more clusters of cells corresponding to different cell types in the tumour microenvironment. The deep learning model has been obtained by: obtaining cell type labels associated with one or more clusters of cells in the latent space of a first deep learning model that has been trained to identify a latent variable representation of single cell gene expression profiles from cells in tumour samples that have not been purified to select tumour microenvironment cells, wherein the one or more clusters of cells comprise at least a cluster corresponding to tumour cells, obtaining cell type labels associated with one or more clusters of cells in the latent space of a second deep learning model that has been trained to identify a latent variable representation of single cell gene expression profiles from cells identified as non-malignant in tumour samples that have not been purified to select tumour microenvironment cells and/or cells from samples comprising purified tumour microenvironment cells, wherein the one or more clusters of cells correspond to different cell types in the tumour microenvironment, and training a third deep learning model to identify a latent variable representation of single cell gene expression profiles using cell type labels associated with the one or more clusters of cells in the latent space of the first deep learning model and the one or more clusters of cells in the latent space of the second deep learning model.

The present inventors have identified that in order to accurately analyse the tumour microenvironment it was beneficial to map single cell expression profiles using a different representation for tumour cells and microenvironment cells, wherein the former uses a representation adapted to identify tumour cells from their microenvironment and the latter uses a representation adapted to distinguish between different cell types in the microenvironment. Such representations are therefore learned from data that either includes both tumour and microenvironment cells or only includes cells from the microenvironment. This was unexpected as single cell expression atlases were previously thought to be most informative when integrating large and diverse sets of samples. To the best of the inventors knowledge, in the context of analysing single cell expression profiles for tumour samples it was never suggested that an improved characterisation of tumour microenvironment would be obtained by separately analysing profiles from (a) non-purified tumour samples and (b) from purified tumour microenvironment cells and cells from non-purified tumour samples identified as non-tumour cells, rather than analysing together as many diverse samples as possible.

The results of such an analysis (latent variable representation, cell type identification from clusters of cells in latent space) can be used to identify cell types in a tumour sample, to characterise a tumour sample in terms of subtypes of tumour that show various characteristics such as tumour burden or immune cell composition, to identify prognostic or diagnostic features of a tumour sample such as the presence or prevalence of specific cell types or the expression of particular genes in specific cell types, to normalise single cell gene expression data (e.g. removing batch effects) for further analysis, to select patients for a particular course of therapy based on any prognostic or diagnostic feature as described above, to select patients for a clinical trial based on features of samples from said patients that identify the patient as likely responsive to a therapy, to identify a drug target by analysis of expression in particular cell types in tumours, and generally for any purpose that benefits from improved cell type annotation and single cell RNA expression analysis in the context of cancer.

The methods according to the present aspect may have one or more of the following optional features.

The method may comprise training the first deep learning model using data comprising the single cell gene expression profiles for the plurality of cells from the tumour sample and single cell gene expression profiles for a plurality of cells from a plurality of tumour samples that have not been purified to select tumour microenvironment cells. The method may comprise training the second deep learning model using data comprising the single cell gene expression profiles for the plurality of cells from the tumour sample and single cell gene expression profiles for a plurality of cells that have been identified as non-malignant in a plurality of tumour samples that have not been purified to select tumour microenvironment cells and/or for a plurality of cells from samples comprising purified tumour microenvironment cells. The method may comprise training the third deep learning model using data comprising the single cell gene expression profiles for the plurality of cells from the tumour sample and single cell gene expression profiles for a plurality of cells associated with cell type labels associated with clusters of cells in the latent space of the first and/or second deep learning models.

The tumour sample that is being analysed may be a sample that has not been purified to select tumour microenvironment cells. The tumour sample that is being analysed may be a sample that has been purified to select tumour microenvironment cells.

The single cell gene expression profiles for a plurality of cells from a plurality of tumour samples that have not been purified to select tumour microenvironment cells may be referred to as "reference" profiles for the purpose of training the first deep learning model.

The single cell gene expression profiles for a plurality of cells that have been identified as non-malignant in a plurality of tumour samples that have not been purified to select tumour microenvironment cells and/or for a plurality of cells from samples comprising purified tumour microenvironment cells may be referred to as "reference" profiles for the purpose of training the second deep learning model.

Thus, a sample that has not been purified to select tumour microenvironment cells may be analysed together with a plurality of single cell expression profiles that have been identified as non-malignant in a plurality of tumour samples that have not been purified to select tumour microenvironment cells and/or for a plurality of cells from samples comprising purified tumour microenvironment cells (reference profiles) by training a second deep learning model. In such cases the sample that is being analysed together with the reference profiles may be referred to herein as "query sample".

Similarly, a sample that has not been purified to select tumour microenvironment cells may be analysed together with a plurality of single cell expression profiles from a plurality of tumour samples that have not been purified to select tumour microenvironment cells (reference profiles) by training a first deep learning model. In such cases the sample that is being analysed together with the reference profiles may be referred to herein as "query sample".

Further, a sample that has not been purified to select tumour microenvironment cells may be analysed together with a plurality of single cell expression profiles from a plurality of tumour samples that have not been purified to select tumour microenvironment cells (reference profiles) for the purpose of training a first deep learning model. Such a model can thereafter be used to analyse other tumour samples (whether purified or not), for example by transfer learning. In other words, profiles from the sample analysed may form part of the reference profiles. Similarly, a sample that has been purified to select tumour microenvironment cells may be analysed together with a plurality of single cell expression profiles that have been identified as non-malignant in a plurality of tumour samples that have not been purified to select tumour microenvironment cells and/or for a plurality of cells from samples comprising purified tumour microenvironment cells (reference profiles) for the purpose of training a second deep learning model. In other words, profiles from the sample analysed may form part of the reference profiles. Such a model can thereafter be used to analyse other tumour samples (whether purified or not), for example by transfer learning. In other words, profiles from the sample analysed may form part of the reference profiles. Similarly, single cell gene expression profiles from cells that have been identified as non-malignant (e.g. using a latent variable representation from a first deep learning model as described herein) in a sample that has not been purified to select tumour microenvironment cells may be analysed together with a plurality of single cell expression profiles that have been identified as non-malignant in a plurality of tumour samples that have not been purified to select tumour microenvironment cells and/or for a plurality of cells from samples comprising purified tumour microenvironment cells (reference profiles) for the purpose of training a second deep learning model. In other words, selected profiles from the sample analysed may form part of the reference profiles. Such a model can thereafter be used to analyse other tumour samples (whether purified or not), for example by transfer learning. In other words, selected profiles from the sample analysed may form part of the reference profiles.

Tumour microenvironment cells may be stromal cells and/or immune cells. Purification to select tumour microenvironment cells may refer to any immune cell enrichment process. For example, purification to select tumour microenvironment cells may refer to CD45+ cell sorting, orCD3D and TCR gamma delta dual sorting. The single cell gene expression profile may be one that has been obtained using a high-throughput transcriptomics technology. For example, the single cell gene expression profile may comprise gene expression measurements for a set of genes comprises at least 500 genes, at least 1000 genes, at least 2000 genes or at least 4000 genes. The single cell gene expression profile may be a substantially whole transcriptome gene expression profile. In some cases, the high-throughput transcriptomics technology is an untargeted transcriptomics technology, for example using next-generation sequencing. In other words, the single cell gene expression profile may have been obtained using a technology that aims to identify substantially all transcripts expressed by a cell. As the skilled person understands, not all transcripts that can theoretically be expressed from a cells genome will be expressed in any particular condition, and technologies such as next-generation sequencing typically sample the transcriptome of a cell such that not all transcripts expressed by the cell may in fact be detected. Suitably, the single cell gene expression profile has been obtained through single cell RNA sequencing. In particular, the single cell gene expression profiles may be acquired as part of the methods described herein or may have been acquired prior to performing the methods described herein using any single cell RNA sequencing technology known in the art, such as e.g. C1 SMARTer, SMART-Seq, RAGE-seq, STRT, Smart-seq2, MATQ-seq, MARS-seq, CEL-seq, Drop-seq, InDrop (CellBio), Chromium (10x Genomics), ddSEQ (Illumina-BioRad), SEQ-well, SPLIT-seq, etc.

The first and/or second and/or third deep learning models may have been trained (or may be used to analyse a tumour sample) using single cell gene expression profiles comprising gene expression measurements for a selected set of genes. The selected set of genes may be present in all single cell gene expression profiles used to train the deep learning model(s). The selected set of genes may comprise genes that are not present in all single cell gene expression profiles. Expression for such genes may be imputed, such as e.g. by imputing a count of 0, or an average count across all single cell gene expression profiles. The selected set of genes may be present in the single cell gene expression profile from the tumour sample that is analysed. The selected set of genes may comprise genes that are not present in the single cell gene expression profile from the tumour sample that is analysed. Expression for such genes may be imputed, such as e.g. by imputing a count of 0, or an average count across all single cell gene expression profiles. The selected set of genes may comprise genes that have highly variable expression between the single cell gene expression profiles used to train the deep learning model(s). The selected set of genes may comprise a predetermined number of genes (e.g. 1000 genes, 2000 genes, 3000 genes, 4000 genes) that have the most variable expression between the single cell gene expression profiles used to train the deep learning model(s). The selected set of genes may comprise genes that have been previously identified based on expert knowledge. For example, the selected set of genes may comprise genes that are marker of tumour and/or microenvironment cells. The selected set of genes may comprise one or more genes selected from: AASS, ACTA2, ACTC1, ADH7, AGR2, AIF1, ALDH1L1, APOBEC3A, APOC1, APOE, AQP4, ASAH1, ASGR1, ASPN, AXIN2, AXL, AZU1, BATF3, BCL3, BCL11A, C1QA, C1QB, C1QC, CA2, CALD1, CCL2, CCL3, CCL4, CCL5, CCL13, CCL17, CCL19, CCL21, CCL22, CCR1, CCR6, CCR7, CCR8, CD1C, CD1D, CD1E, CD2, CD3D, CD3E, CD3G, CD4, CD6, CD8A, CD8B, CD9, CD14, CD19, CD24, CD27, CD28, CD37, CD38, CD44, CD68, CD69, CD72, CD79A, CD79B, CD81, CD83, CD84, CD96, CD160, CD163, CD200, CD207, CD209, CD244, CD300E, CDK1, CHL1, CLC, CLDN4, CLDN7, CLEC1B, CLEC4C, CLEC4G, CLEC9A, CLEC10A, CNN1, COL1A1, COL1A2, COL5A1, CPA3, CRIP2, CRTAM, CSF1R, CSF3R, CSPG4, CST7, CTHRC1, CTLA4, CTNNA2, CTSG, CXCL8, CXCL13, CXCR3, CXCR4, CXCR5, DCN, DCT, DSG1, EGFR, ELANE, ELF3, ENHO, ENTPD1, EOMES, EPCAM, EXD3, F3, FABP4, FABP7, FAM3C, FAP, FBLN1, FCER1A, FCGR3A, FCGR3B, FCN1, FCN2, FDCSP, FEZ1, FHL1, FLNA, FLT3, FOLH1, FOXP3, FSCN1, FXYD3, GADD45A, GAP43, GATA2, GATA3, GDF15, GNLY, GPBAR1, GPR17, GZMA, GZMB, GZMH, GZMK, GZMM, HAVCR2, HBA1, HBB, HDC, HHIP, HLA-DQA1, HLA-DRA, HLA-DRB1, HMGB2, HPGDS, HSD11B1, HSPB1, ICAM4, ICOS, IDO1, IFI6, IFI16, IFI27, IFI35, IFI44, IFI44L, IFIH1, IFIT1, IFIT2, IFIT3, IFIT5, IFITM1, IFITM2, IFITM3, IFNG, IGHG1, IGHM, IGKC, IL1A, IL1B, IL2, IL2RA, IL3RA, IL4, IL6, IL6ST, IL7R, IL8, IL33, IRF4, IRF7, IRF8, IRF9, ISG15, ITGAM, ITGAX, KIT, KLK3, KLRB1, KLRC1, KLRD1, KLRF1, KLRK1, KRT1, KRT5, KRT8, KRT18, KRT19, L1CAM, LAG3, LAMP3, LAYN, LEF1, LGR5, LILRA4, LILRB2, LILRB4, LRRN4, LST1, LUM, MAGEA4, MBP, MCAM, MCEMP1, MDK, MEST, MGST1, MITF, MKI67, MLANA, MMP9, MMRN1, MPO, MS4A1, MS4A2, MS4A3, MTSS1, MYH11, NCAM1, NCR1, NEAT1, NFIB, NKG7, NOTCH3, NOVA1, NPNT, NTSR2, NUDT17, OPALIN, PCNA, PDCD1, PDGFRB, PERGL, PERP, PFN2, PI16, PILRA, PLA2G7, PLN, PLVAP, PMEL, PMP2, POSTN, PRDM1, PRF1, PROX1, PRTN3, PSPH, PTPRC, RALB, RGS5, RORC, RUNX2, S100A8, S100A9, S100A12, S100A16, SBDSP1, SDC1, SELL, SEPP1, SESN3, SHTN1, SIGLEC10, SMIM22, SMTN, SOX2, SOX4, SOX9, SOX10, SPI1, SPIB, SPN, SPP1, STAB1, STAT4, STMN1, TACSTD2, TAGLN, TBX21, TCF4, TCF7, TCF7L2, TFF3, TGFB1, TIGIT, TNF, TNFRSF4, TNFRSF9, TNFRSF17, TNFRSF18, TNFSF8, TOX, TOX2, TPSAB1, TPSB2, TPST1, TRAC, TRBC1, TRBC2, TRDC, TRGC1, TRGC2, TRIM9, TSPAN13, TUBA1B, TXN, TYMS, TYR, TYRP1, UPK3B, VCAN, VPS37B, VWA5A, VWF, WIF1, XCL1, XCL2, XCR1, and ZNF366.

The method may comprise training the first deep learning model using data comprising the single cell gene expression profiles for the plurality of cells from the tumour sample and parameters from a deep learning model that has been trained to identify a latent variable representation from single cell gene expression profiles for a plurality of cells from a plurality of tumour samples that have not been purified to select tumour microenvironment cells. The method may comprise training the second deep learning model using data comprising the single cell gene expression profiles for the plurality of cells from the tumour sample and parameters from a deep learning model that has been trained to identify a latent variable representation from single cell gene expression profiles for a plurality of cells that have been identified as non-malignant in a plurality of tumour samples and/or for a plurality of cells from samples comprising purified tumour microenvironment cells. The method may comprise training the third deep learning model using data comprising the single cell gene expression profiles for the plurality of cells from the tumour sample and parameters from a deep learning model that has been trained to identify a latent variable representation from single cell gene expression profiles for a plurality of cells associated with cell type labels associated with clusters of cells in the latent space of the first and/or second deep learning models. Thus, a query sample (whether purified or non-purified) may be analysed using transfer learning, based on parameters from a first, second and/or third deep learning model. Each of the first, second and/or third deep learning models may have been trained using respective reference profiles. The deep learning model may be a deep neural network comprising a set of nodes and weights between nodes, the parameters comprise the weights between the nodes, and training uses transfer learning of a model extending the deep learning models for which weights are provided by including one or more additional input nodes with trainable weights. The training may comprise fine tuning weights and training the trainable weights. The training may be performed using architecture surgery as described in Lotfollahi et al., 2022.

The single cell gene expression profiles used to train the first and/or second and/or third deep learning models may be from a plurality of samples at least some of which are from different types of cancers. The single cell gene expression profiles used to train the first and/or second and/or third deep learning models may be from a plurality of samples that do not include haematological malignancies. The present inventors have identified that properties of the tumour microenvironment are emergent properties that are associated with different types of tumours which can be identified in multiple cancer types, such that an improved characterisation of the tumour microenvironment can be obtained by enabling a deep learning algorithm to identify this common variability (which was found to be possible even in the presence of variability associated with cancer types). This was surprising because although the immune cells were better characterised separately from the cancer cells (even within the same cancer type or even the same sample), the characterisation of the microenvironment (composition and representation of the microenvironment in the tumour) was better characterised by combining information across samples and cancer types. The present inventors have further identified that it was beneficial to exclude haematological malignancies from the model training process in order to avoid confusing the classification between malignant cells and non-malignant cells, the latter being expected to comprise an important population of immune cells.

The single cell gene expression profiles used to train the first and/or second and/or third deep learning models may have been acquired using the same or different single cell RNA sequencing technologies. The single cell gene expression profiles for the tumour sample being analysed may have been acquired using the same or different single cell RNA sequencing technologies from the sequencing technologies used to acquire the single cell gene expression profiles used to train the first and/or second and/or third deep learning models. The single cell gene expression profiles used to train the first and/or second and/or third deep learning models may have been acquired using a plurality of different single cell RNA sequencing technologies. The use of data sets that vary in terms of experimental platforms and/or biological sample of origin may result in a more robust latent variable representation.

The tumour sample being analysed may be from a type of cancer that is represented in at least one sample from which the single cell gene expression profiles used to train the first and/or second and/or third deep learning models have been obtained. The tumour sample being analysed may be from a type of cancer that is not represented in the samples from which the single cell gene expression profiles used to train the first and/or second and/or third deep learning models have been obtained.

The samples from which the single cell gene expression profiles used to train the first and/or second and/or third deep learning models have been obtained and/or the tumour sample analysed may be from a cancer selected from: head and neck squamous cell carcinoma (HNSCC), hepatocellular carcinoma (HCC), colorectal cancer (CRC), different types of lung cancer (LC), clear cell renal cell carcinoma (ccRCC), prostate cancer (PC), breast cancer (BC), bladder urothelial carcinoma (BUC), esophageal squamous-cell carcinoma (ESCC), uveal melanoma (UV) and cutaneous squamous cell carcinoma (cSCC).

The tumour sample may be a sample comprising tumour cells or genetic material derived therefrom. The tumour sample may be a sample of cells or tissue that has been obtained directly from a tumour (e.g. a tumour biopsy). The samples from which the single cell gene expression profiles used to train the first and/or second and/or third deep learning models have been obtained and/or the tumour sample analysed may be from mammalian subjects. The samples from which the single cell gene expression profiles used to train the first and/or second and/or third deep learning models have been obtained and/or the tumour sample analysed may be from human subjects. The samples from which the single cell gene expression profiles used to train the first and/or second and/or third deep learning models have been obtained and/or the tumour sample analysed may be sample of cells or tissue that have been obtained directly from a tumour (e.g. a tumour biopsy).

The methods described herein are computer implemented unless context indicates otherwise. Indeed, the size of matched single cell gene expression data sets usable for the purpose of this method, in terms of the number of cells and/or the size of at least the single cell gene expression profiles, and the process of training deep learning models is of a complexity that places the methods described herein far beyond the capability of mental investigation.

In some cases, the methods comprise processing one or more samples of cells or tissues using a single cell transcriptomics protocol to obtain single cell gene expression profiles and/or obtaining the tumour sample from a subject. This step may not be computer implemented and may precede any computer implemented step performed on the single cell transcriptomics data acquired. Alternatively, all of the steps of the method may be computer-implemented and comprise receiving previously acquired single cell gene expression profiles.

The deep learning model may be a variational autoencoder or a generative adversarial net. The first and/or second deep learning models may be unsupervised models. The third deep learning model may be a semi-supervised deep learning model, The deep learning model may be a conditional variational autoencoder (CVAE), a conditional generative adversarial net (cGAN), a transfer variational autoencoder (trVAE), a single cell variational inference (scVI) model, or a single cell annotation using variational inference (scANVI) model. The third deep learning model may be a semi-supervised deep learning model adapted to assign labels to unlabelled single cell gene expression profiles using labels from the reference profiles.

The latent space clusters may be graph based clusters. The clusters may be obtained using the Leiden algorithm. A cell type may refer to a set of cells with a common morphology, physiology and/or function. A cell type may refer to any cell type selected from: malignant cells, non-malignant cells, immune cells, stromal cells, cytotoxic cells, proliferative cells, pro-inflammatory cells, T cells, CD4+ T cells, CD8+ T cells, gamma delta T cells, gamma delta 2 T cells, activated T cells, cd4+ follicular helper T cells, exhausted T cells, exhausted CD4+ T cells, exhausted CD8+ T cells, exhausted regulatory T cells, regulatory T cells, Th17 cells, naïve T cells, naïve CD4+ T cells, naïve CD8+ T cells, proliferative T cells, proliferative CD4+ T cells, proliferative CD8+ T cells, proliferative CD4+ T cells, proliferative CD8+ T cells, recently activated CD4+ T cells, naïve memory CD4+ T cells, terminally exhausted CD8+ T cells, effector memory CD8+ T cells, transitional memory CD4+ T cells, pre-exhausted CD8+ T cells, fibroblasts, B cells, naïve B cells, memory B cells, proliferative B cells, plasma cells, endothelial cells, lymphatic endothelial cells, liver sinusoidal endothelial cells, dendritic cells, plasmacytoid dendritic cells (pDC), cDC1 dendritic cells, dendritic cells expressing CLEC9A, cDC2 dendritic cells, dendritic cells expressing CD1C, cDC3 dendritic cells, dendritic cells expressing LAMP3, myeloid dendritic cells, langerin dendritic cells, follicular dendritic cells, mast cells, natural killer (NK) cells, monocytes, macrophages, tumour associated macrophages (TAM), SPP1 TAMs, M2 TAMs, alveolar macrophages, monocytes, CD14+ monocytes, CD16+ monocytes, erythrocytes, pericytes, keratinocytes, melanocytes, neuronal cells, smooth muscle cells. Malignant cells may also be referred to as "tumour cells", or "cancer cells". Non-malignant cells may also be referred to as "microenvironment cells", "normal cells", "non-tumour cells" or "non-cancer cells". In the context of a tumour sample, non malignant cells may be stromal cells or immune cells.

The cells identified as non-malignant in tumour samples that have not been purified to select tumour microenvironment cells may have been identified based on the latent variable representation from the first deep learning model. The method may comprise identifying cells as non-malignant in the tumour samples that have not been purified to select tumour microenvironment cells based on the latent representation from the first deep learning model. The method may comprise identifying one or more cells in the tumour sample as non-malignant cells based on the latent variable representation from the first deep learning model. Identifying one or more cells in a tumour sample as non-malignant cells based on the latent variable representation from the first deep learning model may comprise classifying one or more cells in the tumour sample between a first class corresponding to malignant cells and a second class corresponding to non-malignant cells by assigning cells to one of a plurality of clusters in the latent space of the first deep learning model, each cluster being associated with a malignant state or non-malignant state. Each cluster may be associated with a malignant state or non-malignant state based on a tumour score obtained from expression of a plurality of genes associated with cancer cells and a plurality of genes associated with immune or stromal cells. The plurality of genes associated with cancer cells may be genes overexpressed in cancer. The plurality of genes associated with cancer cells may comprise one or more of: EPCAM, MLANA and KRT8. The plurality of genes associated with immune or stromal cells may be markers of immune cells, and/or one or more types of stromal cells selected from collagen-producing cells, fibroblasts, pericyte, and/or endothelial origin. The plurality of genes associated with immune or stromal cells may comprise one or more of: a marker of immune cells such as PTPRC, markers of collagen producing cells selected from COL1A1, COL1A2, COL5A1 and LUM, a marker of fibroblasts such as FBLN1, markers of pericyte selected from RGS5, CNN1, MYH11, SMTN, ACTA2, TAGLN and CALD1, and markers of endothelial origin selected from VWF and PVLAP. Other possible genes associated with cancer cells include KRT18, and/or genes identified as differentially expressed between normal adjacent tissue and tumour tissue.

A tumour score as described herein may be obtained by: computing a single cell tumour score from expression of a plurality of genes associated with cancer cells and a plurality of genes associated with immune or stromal cells, obtaining a cluster tumour score as a summarised value of the single cell tumour scores for each cluster, identifying each cluster as malignant or non-malignant based on the cluster tumour score, obtaining a summarised latent space coordinate for the clusters identified as malignant and a summarised latent space coordinate for the clusters identified as non-malignant, and associating a cluster with a malignant state or non-malignant state based on a distance between the cluster and the summarised latent space coordinate for the clusters identified as malignant or non-malignant. The single cell tumour score may be obtained by computing, for each cell, the difference between a summarised expression value for the plurality of genes associated with cancer cells and a summarised expression value for the plurality of genes associated with immune or stromal cells. The summarised expression value may be the mean or the maximum mean for one of a plurality of subsets of genes, such as subsets of genes that are markers of immune cells or one or more types of stromal cells. The cluster tumour score may be the average of the single cell tumour scores for all the cells assigned to a cluster. The summarised latent space coordinate for the clusters identified as malignant / non-malignant may be the average latent space coordinate vector across clusters identified as malignant / non-malignant. Identifying each cluster as malignant or non-malignant based on the cluster tumour score comprises comparing the cluster tumour score to a threshold identified using the distribution of the single cell tumour scores. The threshold may be identified as the positive local minimum of a kernel density estimate of the distribution of single cell tumour scores. The distance may be a Euclidian distance. Other distance metrics may be used such as e.g. Manhattan distance. Associating a cluster with a malignant state or non-malignant state based on a distance between the cluster and the summarised latent space coordinate for the clusters identified as malignant or non-malignant may comprise computing the distance between: (i) the average latent space coordinate for the cluster and the summarised latent space coordinate for the clusters identified as malignant and (ii) the average latent space coordinate for the cluster and the summarised latent space coordinate for the clusters identified as non-malignant, and associating the cluster with a malignant state if the distance in (i) is smaller than the distance in (ii).

The method may comprise identifying cells as non-malignant in the tumour samples that have not been purified to select tumour microenvironment cells based on the latent representation from the first deep learning model, and identifying remaining malignant cells as cells with high or low tumour potential using a classifier trained to distinguish between normal and non normal cells based on one or more metrics derived from a RNAseq copy number variation analysis, optionally wherein the metrics derived from a RNA seq copy number variation analysis are selected from: a single cell CNV score, a single cell percentile CNV score, or a cluster donor entropy score for clusters obtained in single cell CNV score space. The CNV score may be obtained using inferCNVpy. The classifier may be a binary classifier using a single metric (e.g. a single split decision tree) derived from a RNA seq copy number variation analysis. The metric derived from a RNA seq copy number variation analysis may be a cluster donor entropy score for clusters obtained in single cell CNV score space. The clusters may be Leiden clusters in CNV score space. The classifier may be trained using training data comprising a binary labels for normal and non normal cells. The binary labels may be obtained by binarizing specimen pathology labels.

Obtaining single cell gene expression profiles for a plurality of cells from the tumour sample may comprise receiving the single cell gene expression profiles from a computing device, sequence analysis means, data store or user interface. The method may comprise sequencing a tumour sample previously obtained from a subject to obtain single cell gene expression profiles for a plurality of cells in the sample.

The method may comprise providing to a user, for example through a user interface, the results of the analysis or any information derived therefrom. A data store may be a public or private database. The results of the analysis may comprise one or more of: a normalised single cell expression profile for one or more cells obtained using the trained first, second and/or third deep learning models, a cluster label for one or more cells, a cell type label for one or more cells, a trained first, second and/or third deep learning models, the values of parameters (e.g. architecture and weights) of the trained first, second and/or third deep learning models. Information derived from the results of the analysis may comprise one or more of: a tumour sample group assignment derived from a cell type composition obtained using the first, second and/or third deep learning models, a prognostic indication derived from a cell type composition or expression profile obtained using trained first, second and/or third deep learning models, a therapeutic indication derived from a cell type composition or expression profile obtained using trained first, second and/or third deep learning models, an indication of suitability for taking part in a clinical trial derived from a cell type composition or expression profile obtained using trained first, second and/or third deep learning models, etc.

The third deep learning model is trained to identify a latent variable representation of single cell gene expression profiles using cell type labels associated with clusters of cells in the latent space of the first deep learning model identified as malignant cells, and cell type labels associated with clusters of cells in the latent space of the second deep learning model, wherein the clusters of cells comprise at least a cluster corresponding to tumour cells and one or more clusters of cells corresponding to different cell types in the tumour microenvironment. The cell type labels may have been identified by: training a first deep learning model to identify a latent variable representation of single cell gene expression profiles from cells in tumour samples that have not been purified to select tumour microenvironment cells, identifying non-malignant cells and malignant cells based on the latent variable representation from the first deep learning model, associating a cell type label to any cell identified as a malignant cell, training a second deep learning model to identify a latent variable representation of single cell gene expression profiles from cells identified as non-malignant cells based on the latent variable representation from the first deep learning model and/or cells from samples comprising purified tumour microenvironment cells, clustering the latent space representation of single cell gene expression profiles from the second deep learning model, and associating a cell type label to one or more of the clusters, optionally wherein associating a cell type label is performed based on the level and/or frequency of expression of one or more markers for each cell type label in the cells of a cluster, and/or wherein associating a cell type label to one or more of the clusters comprises re-clustering the one or more clusters to identify further clusters such that the expression of one or more markers is more homogeneous within the further clusters than in the original cluster(s).

Associating a cell type label to one or more of the clusters may comprise defining one or more criteria that apply to the expression of one or more genes in the single cell gene expression profiles. The one or more criteria may apply to the expression values for one or more genes that are known markers of cell types (i.e. markers known to be associated with a cell type). For example, a cluster may be assigned a cell type label if the proportion of cells in the cluster expressing one or more markers associated with the cell type is above a threshold, wherein a cell may be considered to express a marker if expression of the marker is above a predetermined threshold. Alternatively, a cluster may be assigned a cell type label if the average or median expression measurements for one or more markers associated with the cell type across cells in the cluster is above a threshold. Without wishing to be bound by theory, it is believed that a population of cells even from a single cell type cluster may not all express a particular known marker or combination of markers.

The method may comprise step (a) of classifying the tumour sample between a plurality of classes associated with different tumour burdens, wherein the tumour burden refers to the proportion of cells that are malignant cells vs non-malignant cells in the tumour sample, based on the proportion of cells in the tumour sample assigned to one or more latent space clusters from the first deep learning model corresponding to tumour cells and optionally the proportion of cells in the tumour samples assigned to one or more latent space clusters from the second deep learning model, or based on the proportion of cells in the tumour sample assigned to a latent space cluster from the third deep learning model corresponding to tumour cells. The plurality of classes may comprise a class with a higher tumour burden than all other classes, and a class with a lower tumour burden than all other classes. The plurality of classes may comprise a class with a high tumour burden and a class with a low tumour burden. The plurality of classes may comprise a class with a high tumour burden, a class with an intermediate tumour burden and a class with a low tumour burden. The plurality of classes may have been defined by clustering cell type profiles for a plurality of samples, each cell type profile comprising the proportion of cells assigned to one or more latent space clusters from the first deep learning model corresponding to tumour cells and optionally the proportion of cells assigned to one or more latent space clusters from the second deep learning model, wherein one or more of the clusters correspond to the plurality of classes, and classifying the tumour sample comprises clustering a cell type profile for the tumour sample together with cell type profiles for the plurality of samples, or selecting the class associated with the cluster that is closest to the cell type profile for the tumour sample. The method may comprise step (b) of identifying the cell type composition of the tumour sample by associating cell type labels with one or more cells in the tumour sample using the third deep learning model. Associating cell type labels may comprise obtaining a cell type label and prediction confidence for each latent space cluster or cell using the third deep learning model and associating a cell type label to any cell for which the prediction confidence is above a predetermined threshold or to any cell that belongs to a cluster for which the prediction confidence is above a predetermined threshold. The method may comprise step (c) of comparing the gene expression values of one or more genes in one or more latent space clusters of the first, second and/or third deep learning model. The method may comprise step (d)(i) of using the first, second and/or third deep learning models to obtain batch-corrected single cell gene expression profiles for the sample. The method may comprise step (d)(ii) of identifying a gene as a biomarker of treatment response, a biomarker of prognosis or a therapeutic target based on the gene expression values of the gene in one or more latent space clusters of the first, second and/or third deep learning model. Identifying a gene as a biomarker of treatment response or prognosis may comprise correlating expression of the gene in one or more latent space clusters with a metric of treatment response or prognosis The method may comprise step (e) of identifying a therapy for the subject from which the tumour sample has been obtained based on the cell type composition in (b), the expression of one or more genes identified as a biomarker of treatment response in (d)(ii) and/or the tumour burden classification in (a). The method may comprise step (f) of selecting a subject from which the tumour sample has been obtained for participation in a clinical trial based on the cell type composition in (b), the expression of one or more genes identified as a biomarker of treatment response in (d)(ii) and/or the tumour burden classification in (a). The method may comprise step (g) of providing a prognosis for the subject from which the tumour sample has been obtained based on the cell type composition in (b), the expression of one or more genes identified as a biomarker of treatment response in (d)(ii) and/or the tumour burden classification in (a).

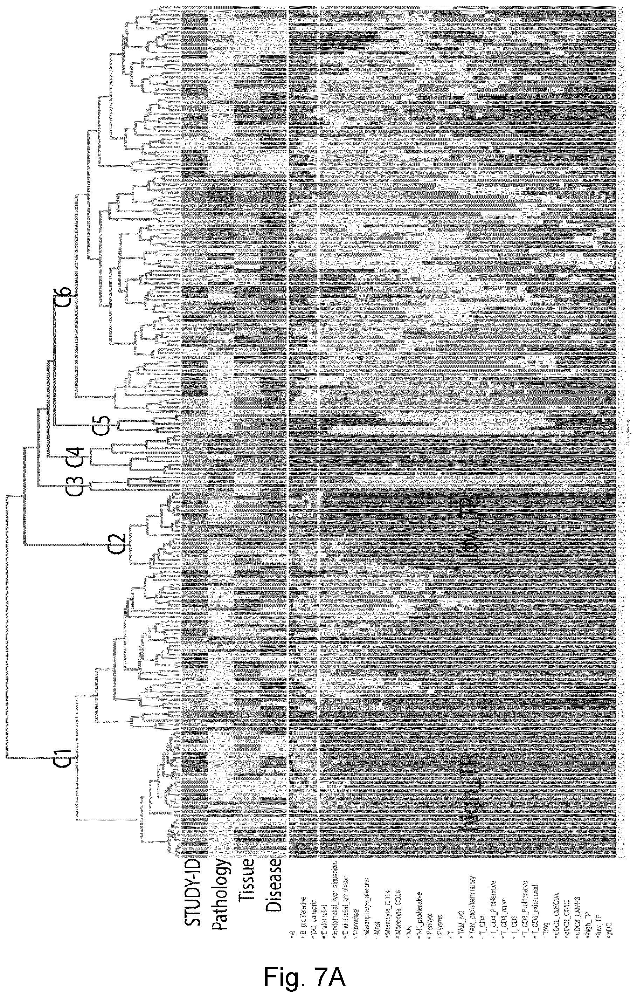

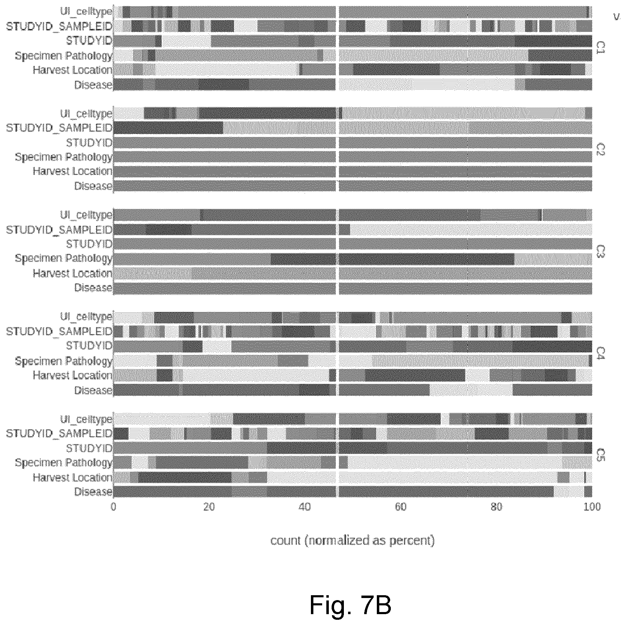

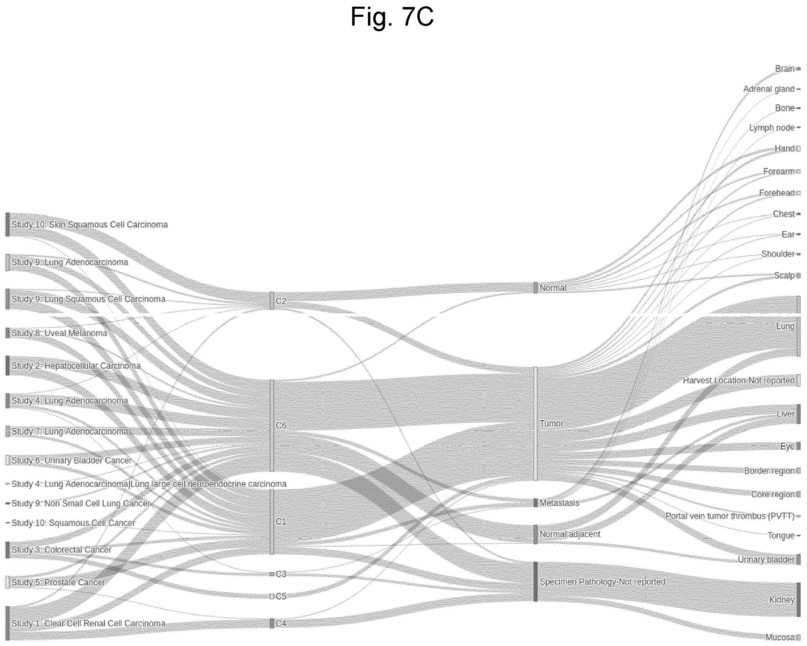

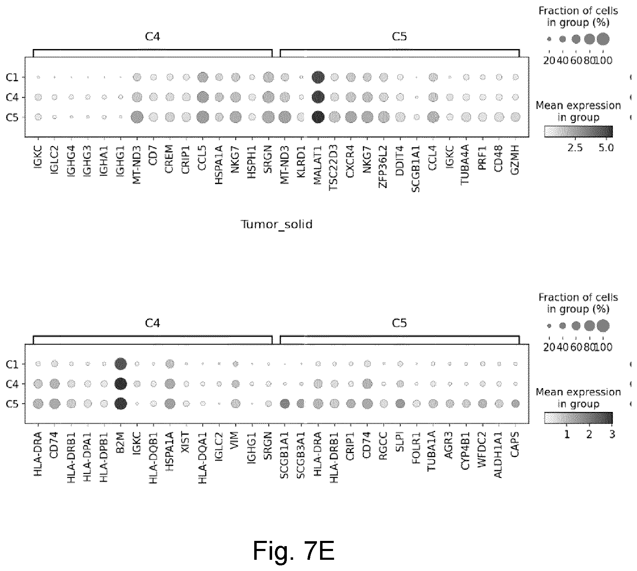

For example, a cell type composition identified as described herein can be used to identify a subject as having a high proportion of immunosuppressive cells in their tumour microenvironment (e.g. many cells assigned to clusters identified as regulatory T cells or myeloid cells). Such a subject may be selected for treatment with a checkpoint inhibitor therapy. As another example, the present inventors have identified robust clusters of tumours with different tumour burdens, such as e.g. clusters C1 and C4-C5 investigated in the examples (see Figure 7A). These were shown to be robust as they involved samples from different diseases, studies, and harvest locations (see Figure 7B). The inventors have further shown that cluster 5 was associated with a significantly higher ratio of CD8+ T cells to regulatory T cells than the medium tumour cluster 4 or the high tumour burden cluster 1 (see Figure 7C). This has been previously shown to correlate with poor clinical outcome. Thus, a subject may be identified as having a poor prognosis if a tumour sample from the subject samples assigned to a class associated with low tumour burden. As another example, the present inventors identified that Type II HLA molecules had stronger expression in cluster C5 than in cluster C1 (see Figure 7E). Thus, a subject may be identified as having a tumour that is likely subject to immune evasion if a tumour sample from the subject samples assigned to a class associated with low tumour burden.

The method may further comprise defining the plurality of classes by clustering cell type profiles for a plurality of samples, each cell type profile comprising the proportion of cells assigned to one or more latent space clusters from the first deep learning model corresponding to tumour cells, wherein one or more of the clusters correspond to the plurality of classes. Each cell type profile may comprise the proportion of cells assigned to one or more latent space clusters from the first deep learning model corresponding to tumour cells and the proportion of cells assigned to one or more latent space clusters from the second deep learning model. The clustering may be any clustering method known in the art. For example, the clustering method may be a linkage-based clustering (e.g. hierarchical clustering), a centroid based clustering (e.g. k-means), a distribution-based clustering (e.g. Gaussian mixture models), a density-based clustering, a graph-based clustering (e.g. clique analysis), or an unsupervised neural network (e.g. a self-organising map). The clustering may be hierarchical clustering.

Thus, also described herein is a method of determining the tumour burden in a tumour sample wherein the tumour burden refers to the proportion of cells that are malignant cells vs non-malignant cells in the tumour sample, the method comprising: analysing the tumour sample using the method of any embodiment of the first aspect; and classifying a tumour sample between a plurality of classes associated with different tumour burdens based on the proportion of cells in the tumour sample assigned to one or more latent space clusters from the first deep learning model corresponding to tumour cells and optionally the proportion of cells in the tumour samples assigned to one or more latent space clusters from the second deep learning model, or based on the proportion of cells in the tumour sample assigned to a latent space cluster from the third deep learning model corresponding to tumour cells. The method according to the present aspect may have any of the following optional features.

The plurality of classes may comprise a class with a higher tumour burden than all other classes, and a class with a lower tumour burden than all other classes. The plurality of classes may comprise a class with a high tumour burden and a class with a low tumour burden. The plurality of classes may comprise a class with a high tumour burden, a class with an intermediate tumour burden and a class with a low tumour burden. The plurality of classes may have been defined by clustering cell type profiles for a plurality of samples, each cell type profile comprising the proportion of cells assigned to one or more latent space clusters from the first deep learning model corresponding to tumour cells and optionally the proportion of cells assigned to one or more latent space clusters from the second deep learning model, wherein one or more of the clusters correspond to the plurality of classes. Classifying the tumour sample may comprise clustering a cell type profile for the tumour sample together with cell type profiles for the plurality of samples. Alternatively, classifying the tumour sample may comprise selecting the class associated with the cluster that is closest to the cell type profile for the tumour sample.

Also described is a method of identifying the cell type composition of a tumour sample, the method comprising: analysing the tumour sample using the method of any embodiment of the first aspect; and associating cell type labels with one or more cells in the tumour sample using the third deep learning model. Associating cell type labels may comprise obtaining a cell type label and prediction confidence for each latent space cluster or cell using the third deep learning model and associating a cell type label to any cell for which the prediction confidence is above a predetermined threshold or to any cell that belongs to a cluster for which the prediction confidence is above a predetermined threshold.

Also described but not claimed is a method of analysing single cell gene expression data from a tumour sample, the single cell gene expression data comprising single cell gene expression profiles for a plurality of cells from the tumour sample, the single cell gene expression profiles comprising gene expression measurements for a set of genes, the method comprising: using a deep learning model to identify a respective latent variable representation of the single cell gene expression profiles in the sample; and identifying a respective one of one or more latent space clusters of cells that the cells in the sample belong to, wherein the clusters of cells correspond to cells from different cell types, wherein the deep learning algorithm is selected from: a first deep learning model that has been trained to identify a latent variable representation of single cell gene expression profiles from cells in tumour samples that have not been purified to select tumour microenvironment cells, wherein the one or more clusters of cells comprise at least a cluster corresponding to tumour cells, a second deep learning model that has been trained to identify a latent variable representation of single cell gene expression profiles from cells identified as non-malignant in tumour samples that have not been purified to select tumour microenvironment cells and/or cells from samples comprising purified tumour microenvironment cells, wherein the one or more clusters of cells correspond to different cell types in the tumour microenvironment, and a third deep learning model that has been trained to identify a latent variable representation of single cell gene expression profiles using cell type labels associated with clusters of cells in the latent space of the first and/or second deep learning model, wherein the one or more clusters of cells comprise at least a cluster corresponding to tumour cells and/or clusters of cells corresponding to different cell types in the tumour microenvironment. The method according to the present aspect may have any of the features described in relation to any aspect described herein and in particular to any embodiment of the first aspect. The method may further comprise comparing the gene expression values of one or more genes in one or more latent space clusters of the first, second and/or third deep learning model. The method may further comprise using the first, second and/or third deep learning models to obtain batch-corrected single cell gene expression profiles for the sample.

Also described is a method of identifying a gene as a biomarker of treatment response, a biomarker of prognosis or a therapeutic target, the method comprising: analysing a plurality of tumour samples using the method of any embodiment of the first aspect; and identifying a gene as a biomarker of treatment response, a biomarker of prognosis or a therapeutic target based on the gene expression values of the gene in one or more latent space clusters of the first, second and/or third deep learning model. Identifying a gene as a biomarker of treatment response or prognosis may comprise correlating expression of the gene in one or more latent space clusters with a metric of treatment response or prognosis. For example, differential expression of a gene in one or more clusters compared to other clusters may be associated with poor or good prognosis, or with treatment response in subjects. In other words, subjects with significantly higher or lower expression of the gene in a particular cluster compared to other clusters or compared to other samples may be associated with better or worse prognosis or treatment response than subjects that do not have said significantly higher or lower expression of the gene. Identifying a gene as a therapeutic target may comprise identifying the gene as significantly upregulated or downregulated in a selected cluster compared to other clusters, wherein the selected cluster corresponds to cells that are beneficially targeted by therapy.

Also described is a method of identifying a therapy for a subject that has been diagnosed as having cancer, the method comprising: identifying the cell type composition of a tumour sample from the subject using a method of the third aspect, and identifying a therapy for the subject based on the cell type composition, and/or analysing single cell gene expression data from a tumour sample from the subject using the method of the fourth aspect, determining the expression of one or more genes identified as a biomarker of treatment response optionally using the method of the fifth aspect, and identifying the subject for treatment with the therapy associated with the biomarker(s) based on the expression of the one or more genes in one or more clusters of cells from the sample; determining the tumour burden in a tumour sample from the subject using the method of the second aspect and identifying a therapy for the subject from which the tumour sample has been obtained based on the tumour burden classification of the sample.

Also described is a method of providing a prognosis for a subject that has been diagnosed as having cancer, the method comprising: identifying the cell type composition of a tumour sample from the subject using a method of the third aspect, and identifying a prognosis for the subject based on the cell type composition, and/or analysing single cell gene expression data from a tumour sample from the subject using the method of the fourth aspect, determining the expression of one or more genes identified as a biomarker of prognosis optionally using the method of the fifth aspect, and identifying the subject as associated with a poor or good prognosis depending on the expression of the biomarker(s) in one or more clusters of cells from the sample; determining the tumour burden in a tumour sample from the subject using the method of the second aspect and determining a prognosis for the subject based on the tumour burden classification of the sample. For example, a tumour sample being classified in a class with low tumour burden may be associated with poor prognosis compared to a tumour sample classified in a class with high tumour burden.

Also described is a method of selecting a subject that has been diagnosed as having cancer for participation in a clinical trial, the method comprising: identifying the cell type composition of a tumour sample from the subject using a method of the third aspect, and selecting or excluding the subject from participation in the clinical trial depending on the cell type composition, optionally wherein samples associated with a cell type composition comprising a proportion of cells in one or more clusters that satisfies one or more predetermined criteria is selected for participation in the clinical trial (for example, the one or more clusters may correspond to cell types that are targeted by the therapy in the clinical trial), and/or analysing single cell gene expression data from a tumour sample from the subject using the method of the fourth aspect, determining the expression of one or more genes identified as a biomarker of treatment response optionally using the method of the fifth aspect, and selecting or excluding the subject from participation in the clinical trial depending on the expression of the biomarker(s) in one or more clusters of cells from the sample (for example, the one or more clusters may correspond to cell types in which expression of the biomarker is associated with response to the therapy in the clinical trial); and/or determining the tumour burden in a tumour sample from the subject using the method of the second aspect and selecting or excluding the subject from the clinical trial based on the tumour burden classification of the sample. For example, a tumour sample being classified in a class with low tumour burden may be associated with poor response to the therapy in the clinical trial compared to a tumour sample classified in a class with high tumour burden, and the subject may thus be excluded from participation in the clinical trial. Conversely, a tumour sample being classified in a class with high tumour burden may be associated with good response to the therapy in the clinical trial compared to a tumour sample classified in a class with low tumour burden, and the subject may thus be selected for participation in the clinical trial. The particular criteria and associated treatment response may vary depending on the biomarker, therapy, etc, and the invention is not limited in this regard.

According to a further aspect, there is provided a system comprising: a processor; and a computer readable medium comprising instructions that, when executed by the processor, cause the processor to perform the (computer-implemented) steps of the method of the first aspect. Also described is a non-transitory computer readable medium or media comprising instructions that, when executed by at least one processor, cause the at least one processor to perform the method of any embodiment of any aspect described herein. Also described is a computer program comprising code which, when the code is executed on a computer, causes the computer to perform the method of any embodiment of any aspect described herein.

Embodiments of the present invention will now be described by way of example and not limitation with reference to the accompanying figures. However various further aspects and embodiments of the present invention will be apparent to those skilled in the art in view of the present disclosure.

The present invention includes the combination of the aspects and preferred features described except where such a combination is clearly impermissible or is stated to be expressly avoided. These and further aspects and embodiments of the invention are described in further detail below and with reference to the accompanying examples and figures.

### Brief Description of the figures

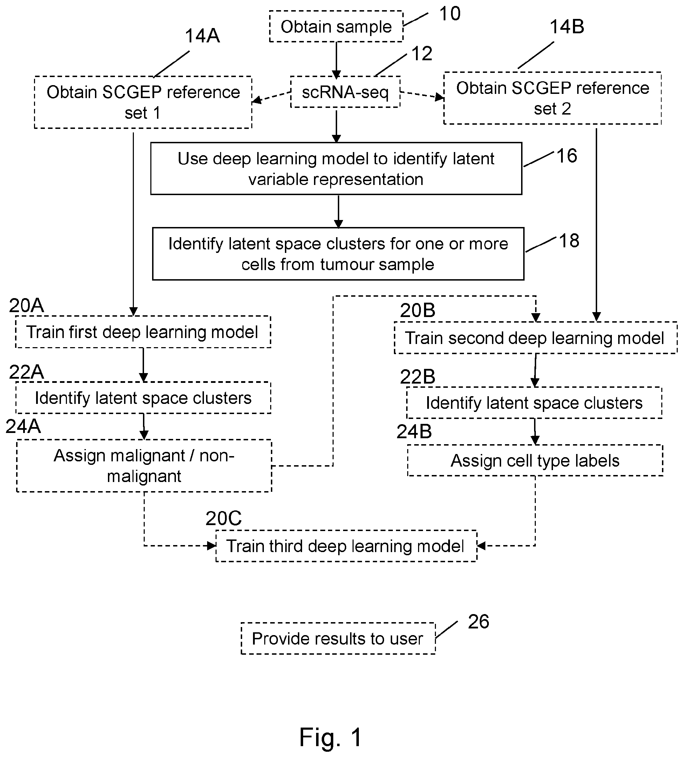

**Figure 1** is a flowchart illustrating a method of analysing a tumour sample as described herein.

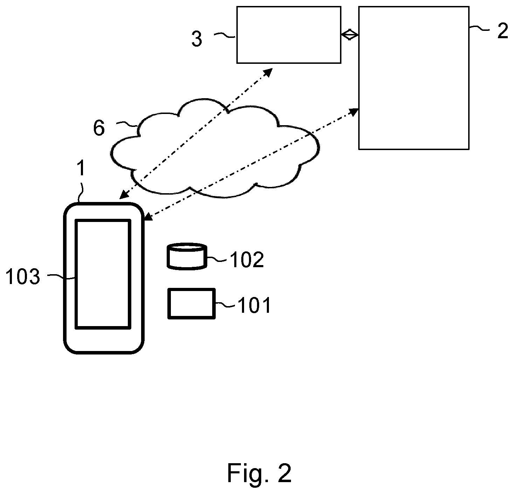

**Figure 2** shows an embodiment of a system for characterising a tumour.

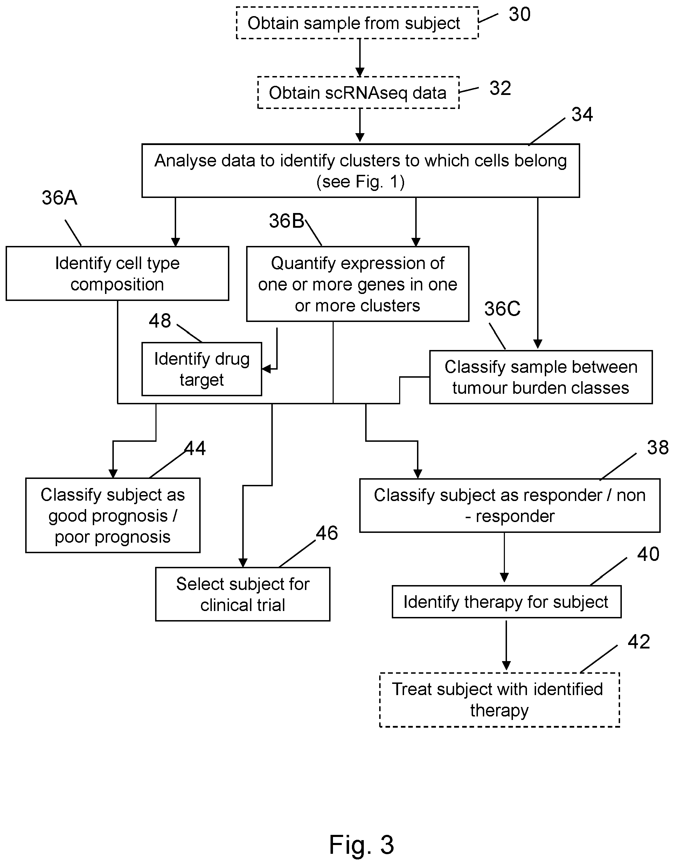

**Figure 3** is a flow diagram illustrating schematically a method using the methods of analysing a tumour sample according to embodiments of the present disclosure.

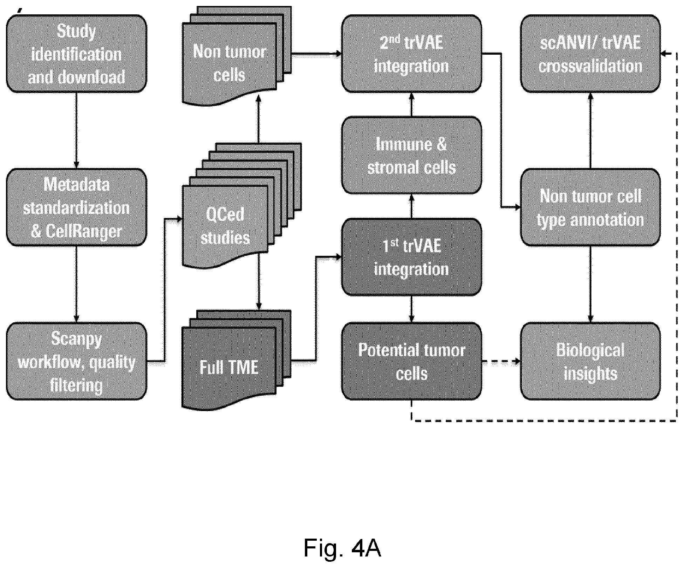

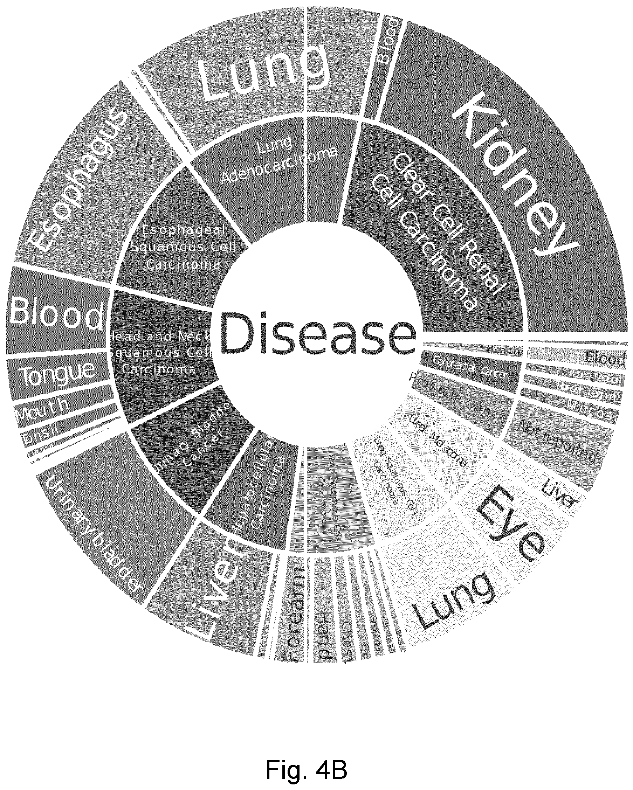

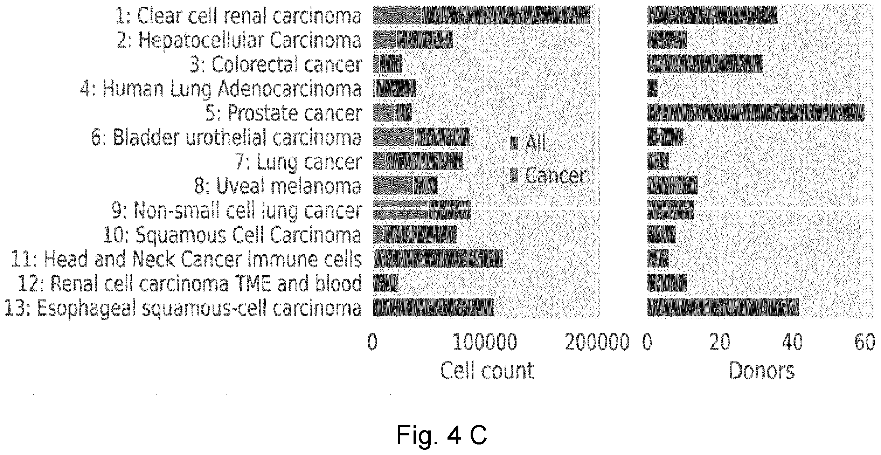

**Figure 4** shows the analysis pipeline and integrated data used in the examples. A. High level schematic of analysis pipeline used. B. Sunburst plot showing the distribution of analysed studies over different cancer indications. C. Cell count (left) and donor count (right) distribution over analysed studies. The cell count (number of cancer cells) on the left is based on cancer identity assessed through the process of malignant cell identification described I the examples (1^{st} trVAE integration in A).

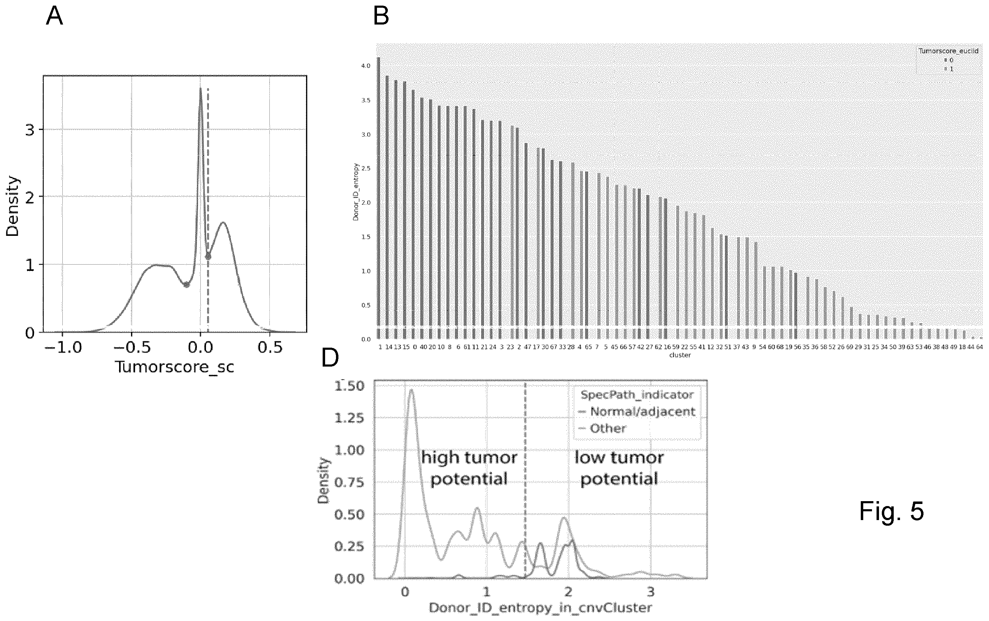

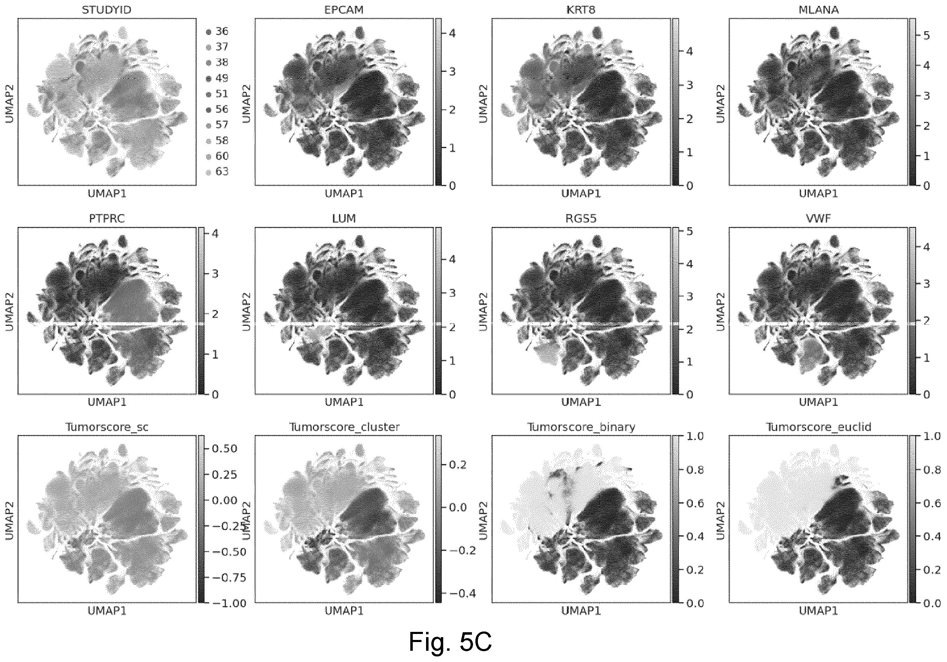

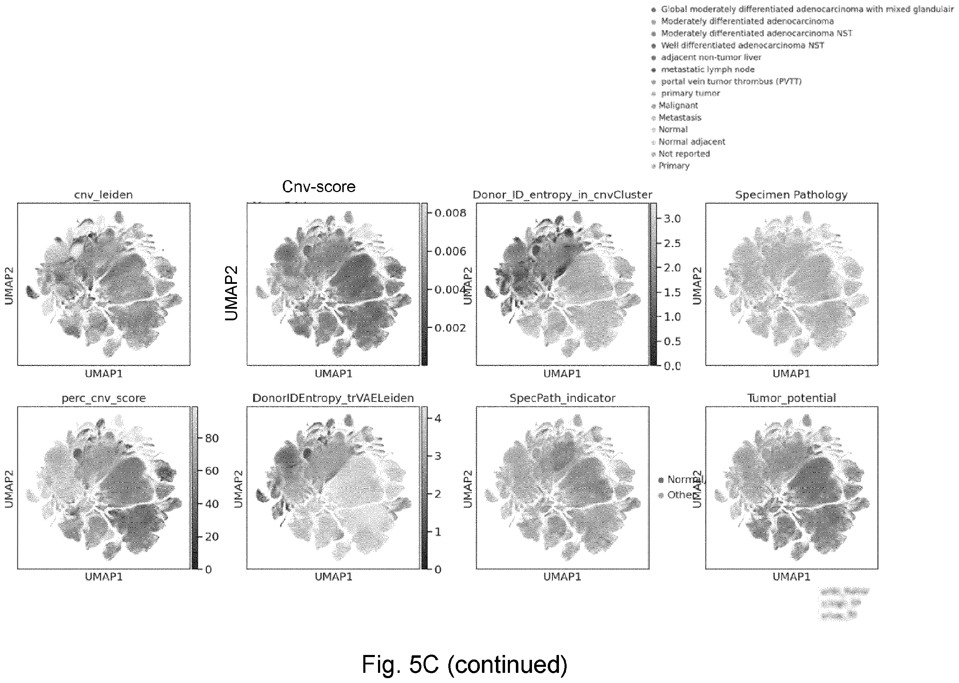

**Figure 5** shows the results of a process of malignant cell identification. A. Kernel density estimation of single cell tumour scores. Red dots indicate minima of the density distribution. The red dashed line shows the positive minima, used as a threshold for classifying clusters as (non) malignant. B. Cluster-wise donor ID entropy scores. Blue / orange bars indicate healthy / malignant clusters. C. UMAP representation of integrated full TME studies. The colouring highlights the distribution of different studies (top left) and marker gene expression values across the Leiden clusters (rows 1 and 2). The last row on the first page (row 3) indicates the initial single cell tumour score, the cluster level tumour score (average of the single cell tumour score for cells in the cluster), its binary equivalent (a 0 or 1 value depending on whether the cluster-level tumour score falls on the left or right of the threshold indicated by the dashed line on A) and the final tumour score after Euclidean distance smoothing, respectively. The first row on the second page (row 4) shows Leiden clusters identified in CNV score space, the CNV scores from inferCNVpy, the donor ID entropy for each Leiden cluster in CNV space, and the known specimen pathology from the study. The second row on the second page (row 5) shows a percentile ranked CNV score with increased comparability between studies (i.e. each cell is assigned the percentile of its ranked score across CNV scores in the study), the donor entropy per cluster in the trVAE Leiden space, and specimen pathology indicator which is a binarized version of the specimen pathology, and the result of classification of cells in the malignant cell cluster identified using the final tumour score above (Figure 5C, firs page, bottom right) between cells with high tumour potential and low tumour potential using a single split decision tree based on donor entropy per cluster in CNV space. D. Kernel Density Estimation showing further separation of 'Other' from A into low/ high tumour potential cells (normal (adjacent)/ malignant) using the donor ID entropy in inferred copy number variation (cnv) cluster.

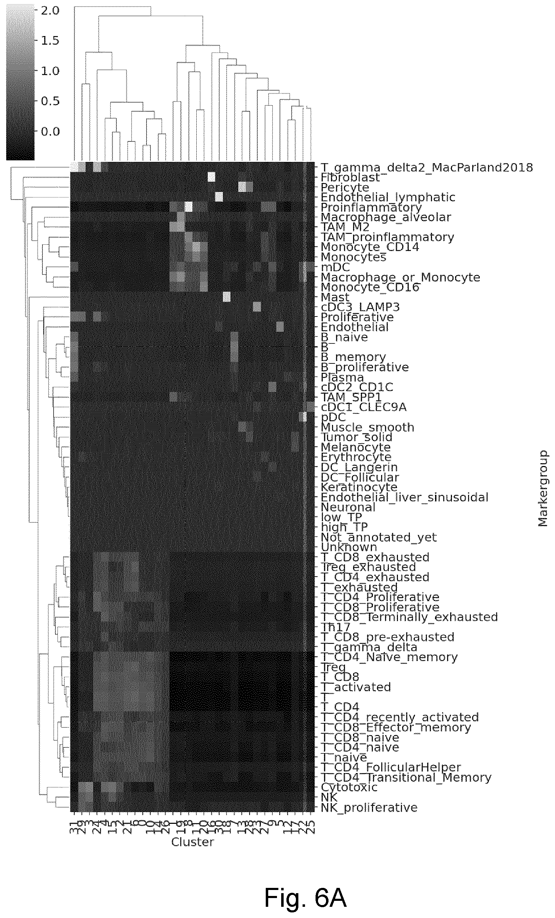

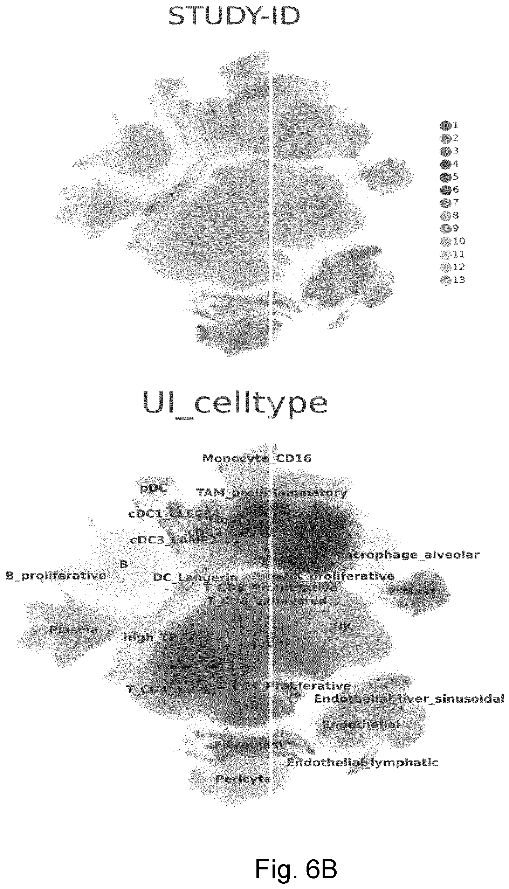

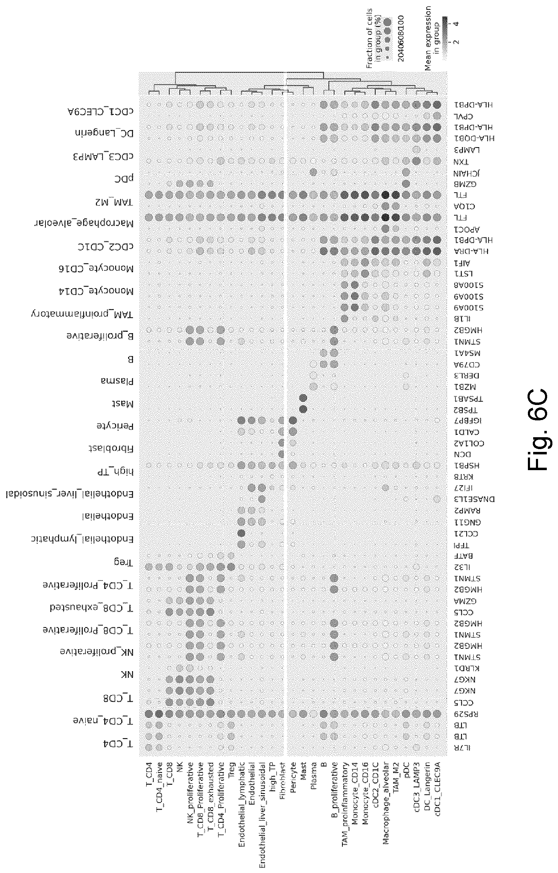

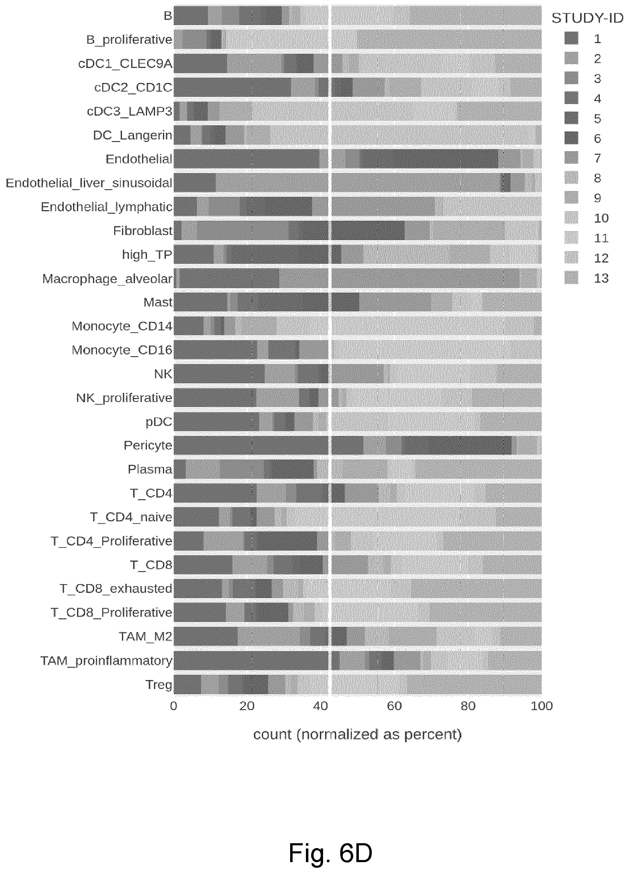

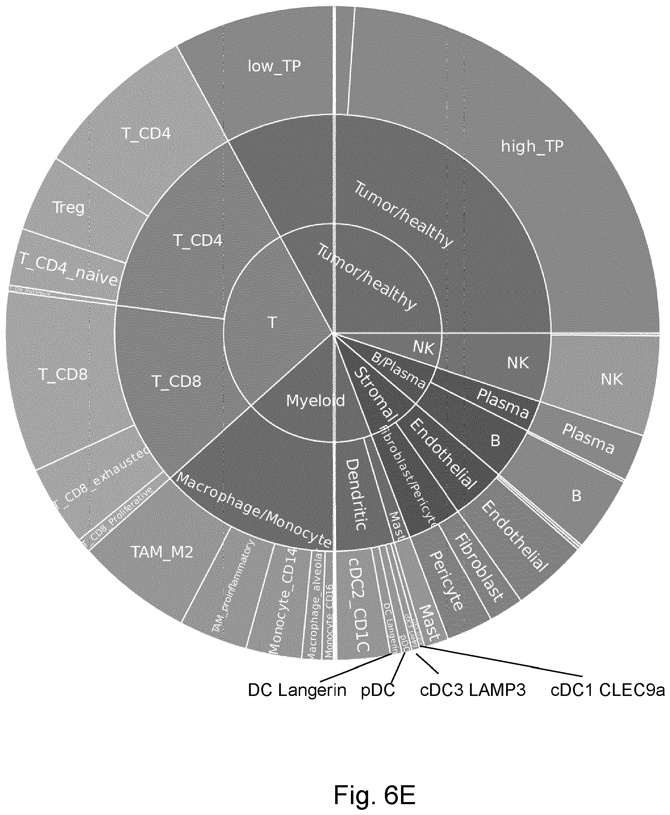

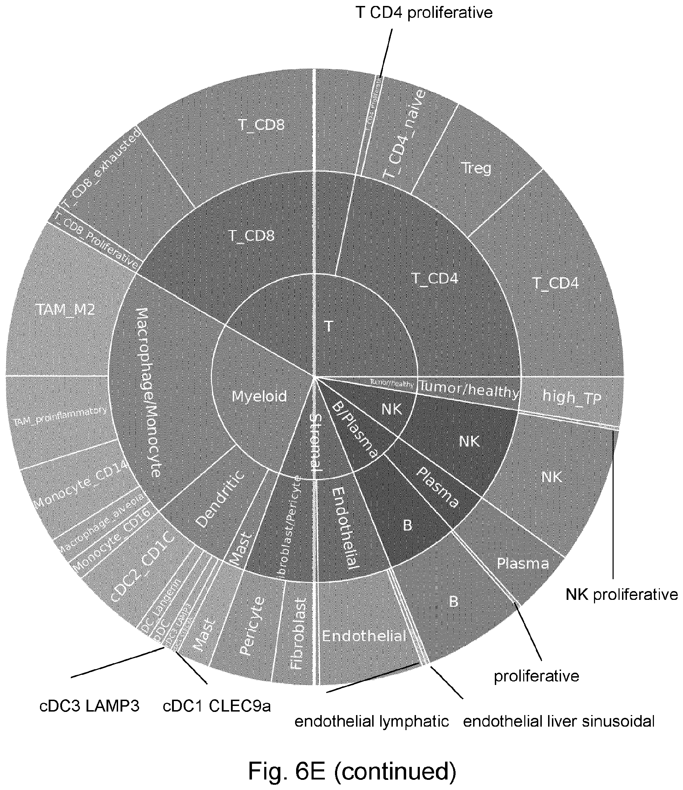

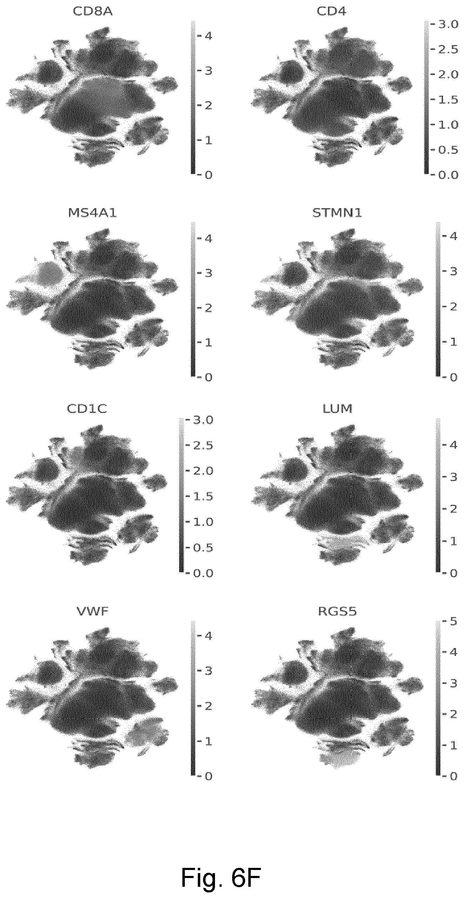

**Figure** 6 shows the results of a process of identifying cell types in human tumour microenvironment. A. Heatmap of hierarchically clustered cell type signature scores per Leiden cluster. The cell signature scores are used as a guidance to analyse the results of the process. They are obtained through a process comprising, for each Leiden cluster: (i) for each marker group in Table 2, compute the mean expression of each marker gene in the marker group over all cells of the cluster, (ii) subtract background mean (in all other clusters) from mean in current cluster, (iii) form median over the subtracted gene expression values. B. UMAP visualization of the trVAE-generated latent space, coloured by assigned cell type (bottom) and study ID (top), showing striking intermixing of studies while keeping cell types separated. Cell types were assigned semi-manually using a combination of factors comprising: the calculated signature scores in A, maker gene expression, identity of neighbouring clusters, diversity of studies forming the cluster, disease types forming the cluster (single disease or mixed), and top differentially expressed genes in the cluster. This assessment may be performed on initial Leiden clustering and again after re-clustering if re-clustering is performed. C. Dotplot of cell type marker genes. The circle radius corresponds to the fraction of cells expressing a marker gene, whereas colour intensity is proportional to marker gene mean expression. D. Distribution of studies used in this analysis per assigned cell types. Counts are normalized as percentages for each cell type. This plot shows the relative study / disease specificity of some cell types (e.g. endothelial_liver_sinusoidal) while other cell types are represented in many studies. In other words, the plot shows how much each study contributes to each cell type. E. Sunburst representation of the relative fraction and hierarchical composition of cell types of the tumour microenvironment, in the combined dataset (first graph shows all cells, second graph shows the composition excluding tumour cells). F. Selected cell type marker genes show strong localization in UMAP plot of latent space.

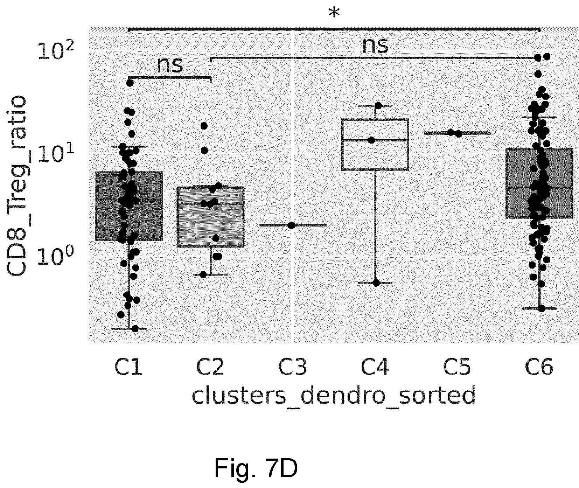

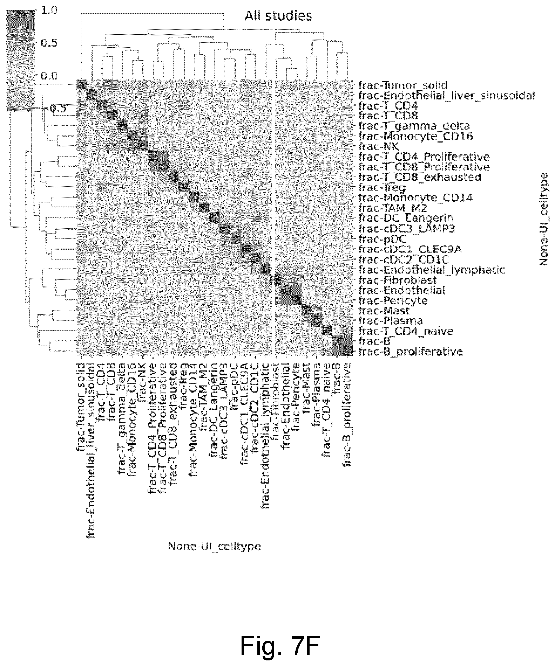

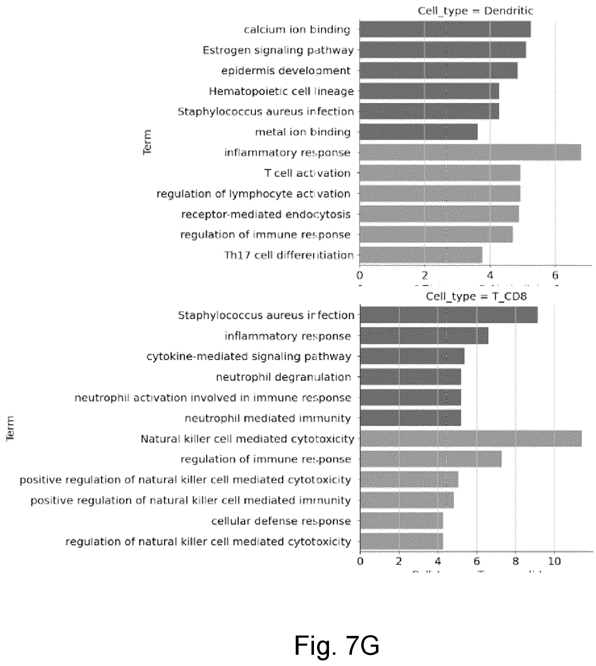

**Figure 7** shows the results of a process of identifying the pan cancer composition of human tumour microenvironments. A. Hierarchically clustered, stacked bar chart, displaying the relative contribution of each cell type to each sample analysed. B. Covariates for each cluster. C. Sankey plot illustration of the quantitative relationship of several covariates and their contribution to the computed immune clusters. Flow width represents the number of samples involved in the respective connection. The plot shows the composition of the dendrogram clusters, indicating that clustering is firstly by TME composition, but also indicating whether some clusters contain an over-representation of e.g. normal samples or tumour samples, which clusters are small and study specific, etc. D. Ratio of CD8+ to Treg cells within each cluster (ns=p value>0.05, *=p value >0.01, two-sided Mann-Whitney test). E. Top overexpressed genes in cluster C4, C5 using cluster C1 as reference. F. Co-appearance/ mutual exclusion of immune cells in the pan cancer TME as determined by relative count correlation across samples (Pearson correlation between the relative counts profiles across samples). G. Selected top pathways in cluster 1 and Cluster 5. Pathways are selected from KEGG 2021 Human pathways, Panther 2016, GO Molecular Function 2021, GO Biological Process 2021 and the differentially expressed genes used per cell type have an adjusted p-value<0.01 and log fold change > 1.

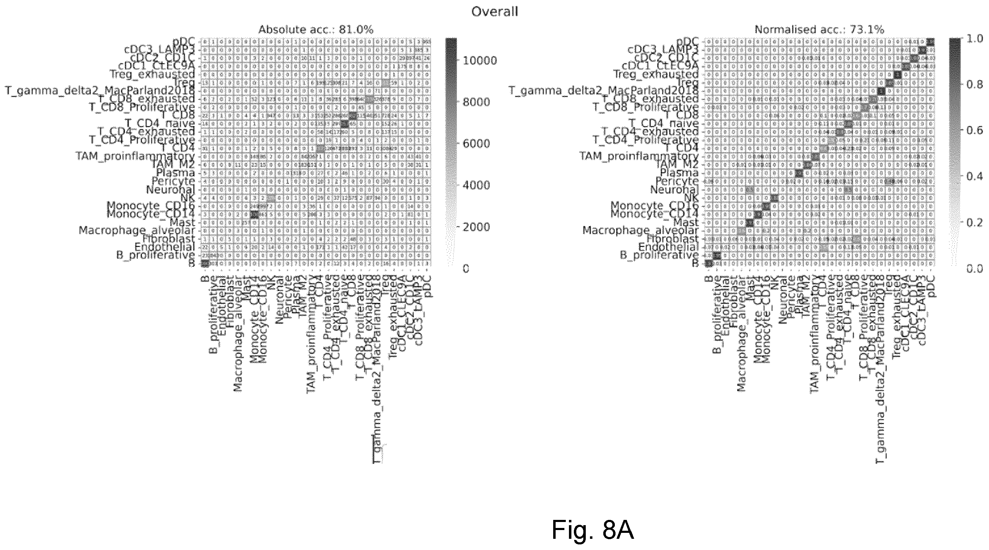

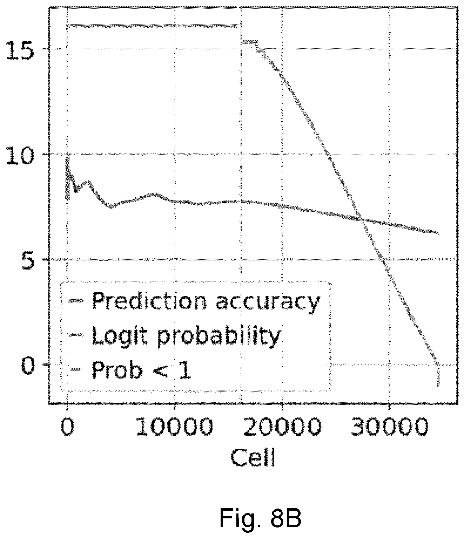

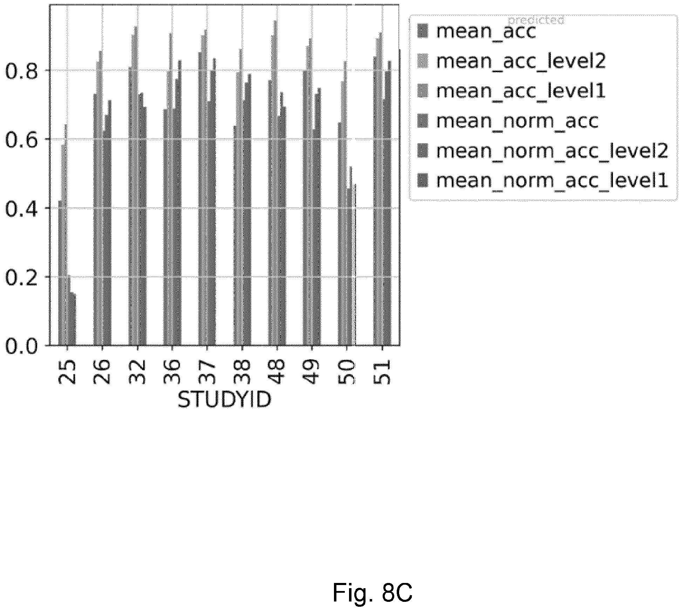

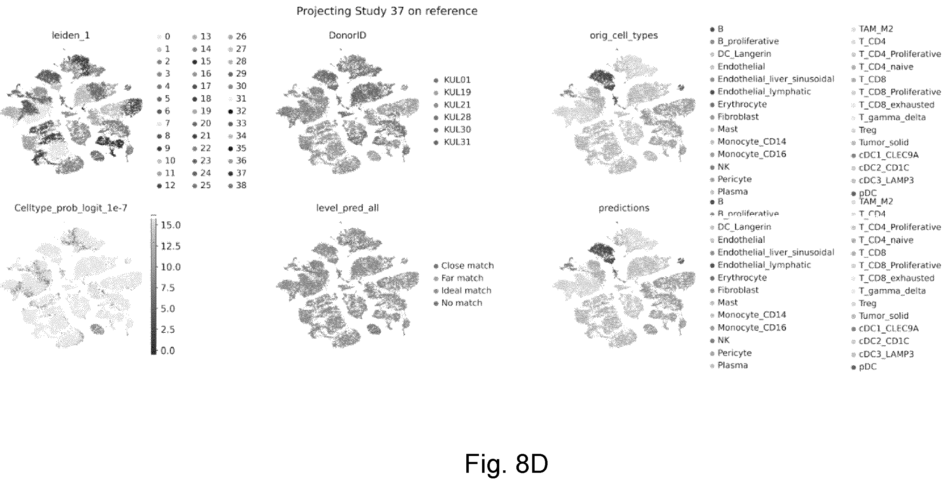

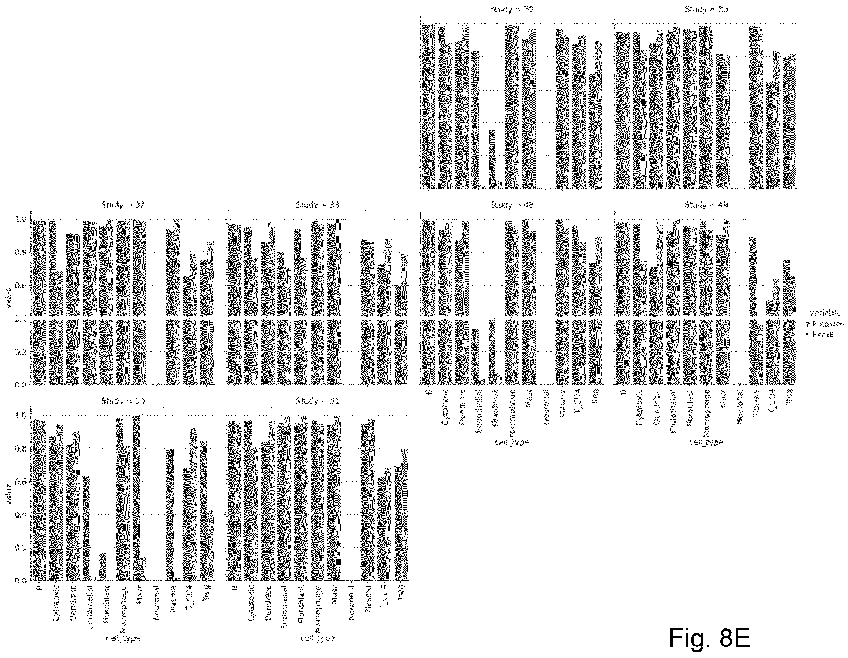

**Figure 8** shows the results of a process for transferring cell type knowledge. A. Confusion matrix showing the overall absolute (left) and normalized (right) prediction accuracy for the mapping of predicted (x-axis) to actual (y-axis) cell types. B. Relationship between empirical cell type prediction accuracy and logit-transformed prediction probability. Cells are sorted by probability with cells of probability of 1 left of the red, dashed line and cells of lower probability on the right. C. Absolute and normalized cell type prediction accuracy for each study and 3 hierarchical levels of cell type annotation. D. UMAP visualization of scANVI prediction on a single example study. The first row shows the distribution of Leiden clusters, donor IDs and original cell types (cell type labels from the semi-manual annotation of trVAE1 clusters). In the second row the logit-transformed prediction probabilities are displayed, together with prediction success and the predicted cell type. For cell type prediction success, 3 separate hierarchical levels of cell type labelling were tested, corresponding to the levels from subfigure C. For prediction success, the predictions were classified as a "close", "far" or "ideal" match to account for cases where trVAE cell type is e.g. T_CD4, but T_CD4_naive is predicted, which would be classified as an incorrect prediction but is not necessarily wrong. This was achieved by mapping cell types to different hierarchies of nomenclature as explained in Table 2. An ideal match would for example be T_CD4_naive predicted by scANVI and semi-manually annotated by trVAE. A close match would be T_CD4 predicted T_CD4_naive actual, and far match would be T_CD4_naive predicted, T_CD8 actual, no match would be any T predicted, any myeloid actual etc. Thus, the close matches are those that are direct hierarchical subsets of each other, ideal matches are exact matches and far matches are matches in different subsets of the same hierarchical branch. E. Precision left bar of each pair of bars) and recall (right bar of each pair of bars) of identification of selected cell types in selected studies. Study IDs see Table 1.

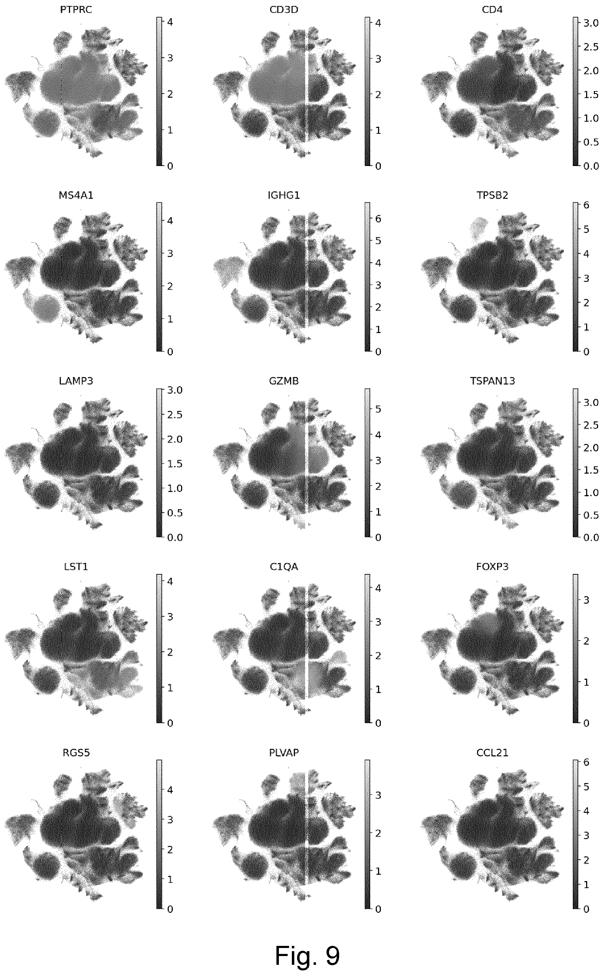

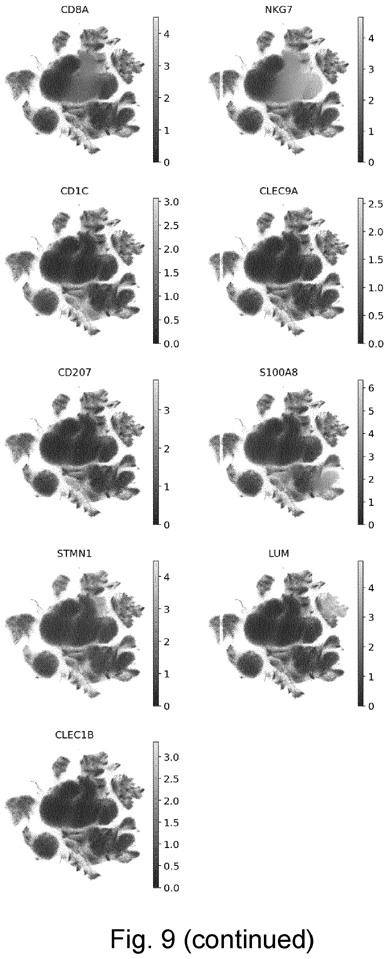

**Figure 9** shows the results of a pan cancer analysis of TME cell type. UMAP plots are shown coloured by selected marker genes. These plots show that canonical cell type marker gene expression is highly localised and thus pan cancer data integration as described herein worked as it is supposed to. For example, the plots show that clusters that were annotated as T_CD8 indeed express CD3 and CD8A etc.

### Detailed description

In describing the present invention, the following terms will be employed, and are intended to be defined as indicated below.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

A "sample" as used herein may be a cell or tissue sample (e.g. a biopsy), or an extract from which genomic material can be obtained for single cell analysis, such as transcriptome sequencing (whole transcriptome sequencing, or targeted (also referred to as "panel") sequencing). In particular, the sample is a tumour sample. The sample may be one which has been freshly obtained from a subject or may be one which has been processed and/or stored prior to making a determination (e.g. frozen, fixed or subjected to one or more purification, enrichment or extractions steps). In particular, the sample may be a cell or tissue culture sample that has been derived from a tumour. As such, a sample as described herein may refer to any type of sample comprising cells or genomic material derived therefrom, whether from a biological sample obtained from a subject, or from a sample obtained from e.g. a cell line. The sample is preferably from a mammalian (such as e.g. a mammalian cell sample or a sample from a mammalian subject, including in particular a model animal such as mouse, rat, etc.), preferably from a human (such as e.g. a human cell sample or a sample from a human subject). Further, the sample may be transported ad/or stored, and collection may take place at a location remote from the genomic sequence data acquisition (e.g. sequencing) location, and/or the computer-implemented method steps may take place at a location remote from the sample collection location and/or remote from the genomic data acquisition (e.g. sequencing) location (e.g. the computer-implemented method steps may be performed by means of a networked computer, such as by means of a "cloud" provider). A "tumour sample" refers to a sample that contains tumour cells and immune cells or genetic material derived therefrom. The tumour sample may be a cell or tissue sample (e.g. a biopsy) obtained directly from a tumour.

As used herein "treatment" and "therapy" refer to reducing, alleviating or eliminating one or more symptoms of the disease which is being treated, relative to the symptoms prior to treatment.

The systems and methods described herein may be implemented in a computer system, in addition to the structural components and user interactions described. As used herein, the term "computer system" includes the hardware, software and data storage devices for embodying a system or carrying out a method according to the above-described embodiments. For example, a computer system may comprise a processing unit such as a central processing unit (CPU) and/or graphics processing unit (GPU), input means, output means and data storage, which may be embodied as one or more connected computing devices. Preferably the computer system has a display or comprises a computing device that has a display to provide a visual output display. The data storage may comprise RAM, disk drives or other computer readable media. The computer system may include a plurality of computing devices connected by a network and able to communicate with each other over that network. It is explicitly envisaged that computer system may consist of or comprise a cloud computer.

The methods described herein may be provided as computer programs or as computer program products or computer readable media carrying a computer program which is arranged, when run on a computer, to perform the method(s) described herein. As used herein, the term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Embodiments of the present disclosure relate to the identification of the cell type of cells present in the tumour microenvironment, including e.g. stromal cells and immune cells, and to distinguishing tumour cells from tumour microenvironment cells. References to cell types refer to phenotypically and/or functionally distinct cell forms within an organism. Within the context of the present disclosure, a cell type refers to any class of cell that can be distinguished on the basis of expression of one or more gene markers. Embodiments of the present disclosure relate to the identification of the cell type of immune and stromal cells. Immune cells are commonly classified into phenotypically and/or functionally distinct classes including natural killer (NK) cells, B cells, monocytes, cytotoxic T cells (also referred to as CD8+ T cells), helper T cells (also referred to as CD4+ T cells), regulatory T cells (CD4+, CD25+ T cells), effector T cells, etc. Multiple subclassifications also exists such as e.g. naïve CD8+ T cells, naïve helper T cells, activated T cells, etc.

Reference to determining the expression level of a gene refers to determination of the expression level of an expression product of the gene. Within the context of the present disclosure, unless indicated otherwise, references to gene expression levels refer to gene expression determined at the nucleic acid level (i.e. at the transcript level). As such, within the context of the present disclosure, gene expression data may also be referred to as transcriptomics data. The gene expression levels determined may be considered to provide a gene expression profile. By "gene expression profile" is meant a set of data relating to the level of expression of one or more of the relevant genes in a cell, in a form which allows comparison with comparable expression profiles (e.g. from cells for whom the cell type is already known), in order to assist in the identification of the cell type of the cell. For example, a gene expression profile may comprise "counts" for each of a plurality of reads. The counts may quantify the number of transcripts from a particular gene observed in a particular cell. Processes for the analysis of scRNA-seq data to obtain counts are known in the art and in particular may depend on the scRNA-seq technology at hand. Examples include SCANPY (Wolf et al., Genome Biol. 2018 Feb 6;19(1):15).

The methods described herein relate in particular to single cell gene expression data. As such, the determination of gene expression levels may involve determining the presence or amount of mRNA in a sample of one or more cells, such that the presence or amount of mRNA in each cell can be determined individually. Methods for doing this are well known to the skilled person. Single cell gene expression levels may be determined in a sample of cells using any conventional method, for example using single cell RNA sequencing (scRNAseq or scRNA-seq) or single cell quantitative PCR (sc-qPCR). Single cell RNA sequencing typically involves a series of steps including single cell isolation (e.g. using micromanipulation, fluorescence activated cell sorting (FACS), laser capture microdissection, microfluidic technology, antibody coated magnetic particle capture, etc.), single cell library preparation (in which single cells are lysed, RNA is reverse transcribed to generate cDNAs including cell-specific barcodes - typically within a single cell droplet, and cDNAs are amplified), and sequencing (which can include 5 end sequencing, 3 end sequencing and/or sequencing of unique molecular identifiers or barcodes introduced in the reverse transcription step). Protocols for single cell RNA sequencing protocols may differ in the way each of the cell isolation, library preparation and sequencing steps performed. A variety of single cell RNA sequencing technologies are available, all of which may be used within the context of the present invention. For example, references to scRNAseq data may refer to data that has been acquired using any of the following protocols: Drop-Seq (Macosko et al., Cell vol. 161, issue 5, p1202-1214, 2015), 10x Genomics Chromium technology, GemCode (Zheng et al., Nature Communications volume 8, Article number: 14049 (2017)) technology, Tang et al. (Nat Methods. 2009 May;6(5):377-82.), STRT (Islam e al., Genome Res. 2011. 21: 1160-1167), SMART-seq (Ramskold et al., Nat Biotechnol. 2012 Aug;30(8):777-82.), CEL-seq (Hashimshony et al., Cell Rep. 2012 Sep 27;2(3):666-73.), RAGE-seq (Singh et al., Nature Communications volume 10, Article number: 3120 (2019)), Quartz-seq (Sasagawa et al., Genome Biology volume 14, Article number: 3097 (2013)), and C1-CAGE (Kouno et al., Nature Communications volume 10, Article number: 360 (2019)). Single cell quantitative PCR typically involves a series of steps including single cell isolation (e.g. using microfluidic technologies, single cell printing, flow cytometry, etc.), followed by cell lysis and amplification of target gene expression products using gene specific primers. Genes whose expression is expected to be constant in the experimental conditions (also referred to as "housekeeping genes") are commonly used for normalisation. Fluorescent dyes are used as reporter molecules to monitor the amplification, from which the initial quantity of the target gene expression products can be inferred.

Methods described herein relate in particular to the analysis of single cell gene expression data using deep learning models. Deep learning is a machine learning technique that trains a model to learn features present in data, using a deep learning model. A deep learning model is an artificial neural network (ANN) that comprises multiple hidden layers. In the context of the present disclosure, the deep learning models are typically unsupervised models or semi-supervised models. Unsupervised models are able to learn features of data without making use of labels associated with data. Semi-supervised models can make use of partially labelled training data. Examples of unsupervised deep learning models include autoencoders and generative adversarial networks (GANs). When analysing single cell gene expression data as described herein, the data that is used as input to the deep learning model is a plurality of gene expression profiles for a respective plurality of single cells. Thus, the data comprises a plurality of data points each comprising a single cell gene expression profile. A single cell gene expression profile is typically a vector comprising a plurality of gene expression measurements for a respective plurality of genes or transcripts. An autoencoder is an ANN that use used to learn efficient encodings of unlabeled data. Encodings refer to values of variables that capture the variability in the data in a more compact manner than the original input data. Autoencoders learn encodings from which the input data can be reconstructed. A GAN is a generative model that learns features of input data (embeddings) in such a way that the model can be used to generate new data with similar characteristics (in particular, data drawn from the same distribution). The variables that are learned by a deep learning model and that capture variability in the input data (encodings or embeddings) can also be referred to as "latent variables". Thus, the deep learning models used herein learn a latent variable representation for single cell gene expression profiles, also referred to as "latent representation" or "latent space representation". A latent variable representation of a single cell gene expression profile comprises a set of values for each of a plurality of latent variables learned by a deep learning model. These can also be seen as coordinates in a new space defined by the latent variables. These coordinates can be used to cluster single cell gene expression profiles in latent space, calculate distances between single cell gene expression profiles in latent space, and generally do any type of analysis that is available to multidimensional data set. A latent space cluster refers to a cluster of data points (e,g, single cell gene expression profiles, also referred to as cells as each such data point relates to a single cell) that has been obtained based on latent space coordinates for these points.

Embodiments of the methods described herein make use of an approach called "transfer learning", which is a machine learning method where a pre-trained model is used as a starting point for training a model to perform a new task. Recently, transfer learning has been applied to single cell RNA-seq data for cell type classification. In particular, Lotfollahi et al. (2022) proposed an approach called "single cell architecture surgery" (also referred to herein as "architecture surgery") wherein a reference deep learning model trained on single cell RNA seq data is extended and fine-tuned to include one or more query samples. In particular, an autoencoder is trained on multiple reference data sets and then the trained weights are transferred with minor weight adaptation (fine tuning) and adding a condition node to map a new sample into the reference.

The wording "reference profile", "reference data" refer to single cell gene expression profiles and collections thereof that are used to train a model for subsequent use. These may also be referred to as "training data".

"Clustering" refers to the process of grouping or segmenting data sets with shared attributes. In other words, clustering typically aims to identify subgroups (also referred to as "clusters") within a data set, where the data points in a subgroup are more similar to each other than they are to data in other subgroups. Clustering does not rely on data that has been labelled, classified or categorised, although labels, categories or classes can be assigned to clusters after the clusters have been identified. Various types of clustering methods are known in the art. For example, a clustering method may be a linkage based clustering (also referred to as connectivity-based clustering e.g. hierarchical clustering) which connects data that are close to each other, a centroid based clustering (e.g. k-means) that represents clusters using a single representative vector, a distribution-based clustering (e.g. Gaussian mixture models) that represents clusters using statistical distributions, a density-based clustering which defines clusters as connected dense regions in the data space, a graph-based clustering (e.g. clique analysis) which represents data points as nodes and similarity as edges and identifies structures such as cliques (a subset of nodes in a graph such that every two nodes in the subset are connected by an edge), or an unsupervised neural network (e.g. a self-organising map).

### Methods of Analysing a Tumour

**Figure 1** is a flow diagram showing, in schematic form, a method of analysing a DNA sample according to the disclosure. At optional step 10, a sample is obtained from a tumour of a subject. At optional step 12, the tumour sample is sequenced using a single cell RNA sequencing protocol to obtain single cell gene expression profiles for a plurality of cells from the tumour sample. At optional step 14A, reference single cell gene expression profiles (SCGEP) for a plurality of cells from a plurality of tumour samples that have not been purified to select tumour microenvironment cells (reference set 1) are obtained. At optional step 14B, reference single cell gene expression profiles for a plurality of cells from a plurality of tumour samples that have been purified to select tumour microenvironment cells (also referred to as single cell gene expression profiles for a plurality of cells from samples comprising purified tumour microenvironment cells) are obtained (reference set 2). At step 16, the single cell gene expression profiles for a plurality of cells from the tumour sample are provided as input to a deep learning model to identify a respective latent variable representation for the single cell gene expression profiles. At step 18, a respective one of one or more latent space clusters of cells is identified for the one or more cells. In other words, each cell is assigned to one of one or more clusters of cells in latent space. The deep learning model comprises a first, second and third deep learning model.

The first deep learning model may optionally be trained at step 20A using the reference profiles obtained at step 14A and optionally also the single cell gene expression profiles from the tumour sample being analysed obtained at step 12. Alternatively, at optional step 20A the first deep learning model may be trained using the reference profiles obtained at step 14A, and partially re-trained using the single cell gene expression profiles from the tumour sample being analysed. The latent variable representation from the first deep learning model is used to identify clusters at step 22A. The latent clusters identified at step 22A may be identified as malignant or non-malignant at step 24A. This may comprise computing a single cell tumour score from expression of a plurality of genes associated with cancer cells and a plurality of genes associated with immune or stromal cells, obtaining a cluster tumour score as a summarised value of the single cell tumour scores for each cluster, identifying each cluster as malignant or non-malignant based on the cluster tumour score, obtaining a summarised latent space coordinate for the clusters identified as malignant and a summarised latent space coordinate for the clusters identified as non-malignant, and associating a cluster with a malignant state or non-malignant state based on a distance between the cluster and the summarised latent space coordinate for the clusters identified as malignant or non-malignant.

The second deep learning model may optionally be trained at step 20B using the reference profiles obtained at step 14B, optionally also the single cell gene expression profiles from the tumour sample being analysed obtained at step 12, and optionally the reference profiles obtained at step 14A for cells belonging to clusters identified as non-malignant at step 24A. Alternatively, at optional step 20B the second deep learning model may be trained using the reference profiles obtained at step 14B and optionally the reference profiles obtained at step 14A for cells belonging to clusters identified as on-malignant at step 24B, and partially re-trained using the single cell gene expression profiles from the tumour sample being analysed. The latent variable representation from the second deep learning model is used to identify clusters at step 22B. The latent clusters identified at step 22B are associated with respective cell type labels at step 24B. This may be based on the level and/or frequency of expression of one or more markers for each cell type label in the cells of a cluster. This may comprise re-clustering the one or more clusters to identify further clusters such that the expression of one or more markers is more homogeneous within the further clusters than in the original cluster(s).

The third deep learning model may optionally be trained at step 20C. The third deep learning model may be trained using any of the reference profiles obtained at step 14A and/or 14B, and associated labels obtained at step 24B for any non-malignant cell and at step 24A for any malignant cell, and the single cell gene expression profiles from the tumour sample being analysed obtained at step 12. Alternatively, at optional step 20C the third deep learning model may be trained using the reference profiles obtained at step 14B and/or step 14C, and partially re-trained using the single cell gene expression profiles from the tumour sample being analysed. The third deep learning model may be a semi-supervised deep learning model adapted to assign labels to unlabelled single cell gene expression profiles using labels from the reference profiles.

At optional step 26, results of any one or more of steps 16 to 24 may be provided to a user.

### Use of Analysis Results

The results of such an analysis (latent variable representation, cell type identification from clusters of cells in latent space) can be used to identify cell types in a tumour sample, to characterise a tumour sample in terms of subtypes of tumour that show various characteristics such as tumour burden or immune cell composition, to identify prognostic or diagnostic features of a tumour sample such as the presence or prevalence of specific cell types or the expression of particular genes in specific cell types, to normalise single cell gene expression data (e.g. removing batch effects) for further analysis, to select patients for a particular course of therapy based on any prognostic or diagnostic feature as described above, to select patients for a clinical trial based on features of samples from said patients that identify the patient as likely responsive to a therapy, to identify a drug target by analysis of expression in particular cell types in tumours, and generally for any purpose that benefits from improved cell type annotation and single cell RNA expression analysis in the context of cancer.

**Figure** 3 illustrates a method of providing a prognosis and/or treating a subject that has been diagnosed with cancer and/or selecting a subject for inclusion in a clinical trial and/or identify a drug target or a biomarker, according to examples described herein. The method may comprise optional step 30 of obtaining a sample from a tumour of a subject. The step of obtaining a sample from a subject may comprise physically obtaining the sample from the subject. Alternatively, the sample may have been previously obtained and no interaction with the subject may be required. In other words, obtaining a sample may comprise receiving a previously acquired sample. At optional step 32, sequence data is obtained from the tumour sample. The step of obtaining sequence data from a sample may comprise sequencing the sample using any single cell RNA sequencing protocol known in the art. Alternatively, sequence data may have been previously obtained. Thus, obtaining sequence data may comprise receiving the data from one or more databases, or from a user through a user interface. At step 34, the single cell gene expression profiles for one or more cells in the sample are analysed using methods described herein such as e.g. by reference to Figure 1. At step 36A, the cell type composition of the tumour sample is identified by associating cell type labels with one or more cells in the tumour sample using the third deep learning model. At step 36B, the gene expression values of one or more genes in one or more latent space clusters of the first, second and/or third deep learning model is analysed and optionally compared. At step 36C, the tumour sample is classified between a plurality of classes associated with different tumour burdens based on the proportion of cells in the tumour sample assigned to one or more latent space clusters from the first deep learning model corresponding to tumour cells and optionally the proportion of cells in the tumour samples assigned to one or more latent space clusters from the second deep learning model, or based on the proportion of cells in the tumour sample assigned to a latent space cluster from the third deep learning model corresponding to tumour cells.

Based on the determinations at steps 36A, 36B and/or 36C, the subject may be classified as having a good or poor prognosis at step 44. Instead or in addition to this, based on the determinations at steps 36A, 36B and/or 36C, the subject may be selected for participation in a clinical trial at step 46. Instead or in addition to this, the subject may be classified at step 38 as being likely to respond or unlikely to respond to a particular course of treatment, where responder/non-responder status is known to be associated with the determinations at steps 36A, 36B and/or 36C. Thus, the determinations at steps 36A, 36B and/or 36C may also be used to identify biomarkers / stratification criteria for prognosis and/or treatment response. At optional step 40, a particular course of treatment (which may comprise one or more different individual therapies) may be identified based on the results of step 38. For example, a subject that has been identified at step 38 as unlikely to respond to the particular course of therapy may be identified as likely to benefit from a therapy that is different from the particular course of therapy. Alternatively, a subject that has been identified at step 38 as likely to respond to the particular course of therapy may be identified as likely to benefit from a therapy that includes the particular course of therapy. At optional step 42, the subject may be treated with the therapy identified at step 40. The determinations at step 36B may also be used to identify a drug target at step 48.

Whether a prognosis is considered good or poor for a tumour sample that satisfies one or more predetermined criteria may vary between cancers and stage of disease. In general terms a good prognosis is one where the overall survival (OS), disease free survival (DFS) and/or progression-free survival (PFS) is longer than that of a comparative group or value, such as e.g. the average for that stage and cancer type, or the average for a comparative group of cancers that do not satisfy one or more criteria. A prognosis may be considered poor if OS, DFS and/or PFS is lower than that of a comparative group or value, such as e.g. the average for that stage and type of cancer, or the average for a comparative group of cancers that do not satisfy one or more criteria. Thus, in general terms, a "good prognosis" is one where survival (OS, DFS and/or PFS) and/or disease stage of an individual patient can be favourably compared to what is expected in a population of patients within a comparable disease setting. Similarly, a "poor prognosis" is one where survival (OS, DFS and/or PFS) of an individual patient is lower (or disease stage worse) than what is expected in a population of patients within a comparable disease setting.

The subject is preferably a human patient. The cancer may be ovarian cancer, breast cancer, endometrial cancer (uterus/womb cancer), kidney cancer (renal cell), lung cancer (small cell, non-small cell and mesothelioma), brain cancer (gliomas, astrocytomas, glioblastomas), melanoma, merkel cell carcinoma, clear cell renal cell carcinoma (ccRCC), lymphoma, gastrointestinal cancer (e.g. colorectal cancer), small bowel cancers (duodenal and jejunal), leukemia, pancreatic cancer, hepatobiliary tumours, liver cancer (e.g. hepatocellular carcinoma), germ cell cancers, prostate cancer, head and neck cancers, bladder cancer, thyroid cancer, oesophagal cancer, melanoma (e.g. uveal melanoma), cutaneous squamous cell carcinoma and sarcomas. For example, the cancer may be head and neck squamous cell carcinoma (HNSCC), hepatocellular carcinoma (HCC), colorectal cancer (CRC), different types of lung cancer (LC), clear cell renal cell carcinoma (ccRCC), prostate cancer (PC), breast cancer (BC), bladder urothelial carcinoma (BUC), esophageal squamous-cell carcinoma (ESCC), uveal melanoma (UV) and cutaneous squamous cell carcinoma (cSCC). All of these have been tested with the methods described herein.

### Systems

**Figure 2** shows an embodiment of a system for analysing a tumour sample and/or for providing a prognosis or treatment recommendation, according to the present disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. The computing device 1 is communicably connected, such as e.g. through a network, to sequence data acquisition means 3, such as a sequencing machine, and/or to one or more databases 2 storing sequence data. The one or more databases 2 may further store one or more of: one or more deep learning algorithm, training data, parameters (such as e.g. parameters of a deep learning model used to identify latent variable representations of single cell gene expression profiles, e.g. weights of an autoencoder model, architecture and parameters of a deep neural network classifier, etc.), clinical and/or sample related information, etc. The computing device may be a smartphone, tablet, personal computer or other computing device. The computing device is configured to implement a method for analysing a tumour sample, as described herein. In alternative embodiments, the computing device 1 is configured to communicate with a remote computing device (not shown), which is itself configured to implement a method of analysing a tumour sample, as described herein. In such cases, the remote computing device may also be configured to send the result of the method of analysing a tumour sample to the computing device. Communication between the computing device 1 and the remote computing device may be through a wired or wireless connection, and may occur over a local or public network 6 such as e.g. over the public internet. The sequence data acquisition means may be in wired connection with the computing device 1, or may be able to communicate through a wireless connection, such as e.g. through WiFi and/or over the public internet, as illustrated. The connection between the computing device 1 and the sequence data acquisition means 3 may be direct or indirect (such as e.g. through a remote computer). The sequence data acquisition means 3 are configured to acquire sequence data from a nucleic acid sample, preferably a nucleic acid sample obtained from single cells or maintaining single cell traceability. The sequence data is RNA expression data (also known as single cell transcriptomics) and may be acquired using any single cell RNA sequencing technology known in the art, such as e.g. C1 SMARTer, SMART-Seq, RAGE-seq, STRT, Smart-seq2, MATQ-seq, MARS-seq, CEL-seq, Drop-seq, InDrop (CellBio), Chromium (10x Genomics), ddSEQ (Illumina-BioRad), SEQ-well, SPLIT-seq, etc. In some embodiments, the sample may have been subject to one or more preprocessing steps such as fixation, dissociation, DNA purification, fragmentation, library preparation, target sequence capture (such as e.g. exon capture and/or panel sequence capture). Any sample preparation process that is suitable for use in the determination of a single cell expression profile may be used within the context of the present invention. The sequence data acquisition means preferably comprises a next generation sequencer.

The following is presented by way of example and is not to be construed as a limitation to the scope of the claims.

### Examples

Cancer is the second leading cause of death in the United States, with a projected, global burden of 28.4 million cases by 2040. Despite substantial progress in diagnostics and treatment, the tumour microenvironment (TME) remains incompletely understood, partially due to inter and intra cancer heterogeneity. The TME comprises not only tumour cells, but also varying proportions of tumour infiltrating immune cells and stromal cells, both of which can interfere with or promote tumour growth. Accordingly, single cell (sc) approaches are required to shed light on the complex interplay between the above. As such, scRNA-Seq offers single cell resolution, and can be considered unbiased since no marker panels are involved. However, cell type annotation, the bottleneck step in scRNA-Seq analysis, is typically carried out at least semi-manually, thereby introducing potential bias. That is, manual cell type annotation traditionally relies on few marker genes, disregarding the larger transcriptome. In addition,
study-intrinsic batch effects and non-harmonised cell type/ state nomenclature further complicate cross study comparisons and data integration.

In these examples, the inventors propose a new method for the analysis of single cell genomic data from tumour samples which is specifically designed to be able to accurately capture the complexity of the tumour microenvironment.

In particular, they demonstrate a new approach (Figure 4A) that uses transfer variational autoencoder (trVAE, described in Lotfohlli et al., 2020) and single-cell Annotation using Variational Inference (scANVI, described in Xu et al., 2021) as implemented in the single-cell architectural surgery (scArches, described in Lotfollahi, M. et al. 2022) approach, applied to publicly available pan-cancer scRNA-Seq data. A new approach using trVAE integration specifically adapted for the study of the TME allowed for separation of malignant cells from healthy cells, rare cell type identification by aggregation, batch effect removal and therewith improved cell type annotation in latent space. Subsequent scANVI-mediated cross validation highlights the reproducibility of the annotation described here and yields an atlas which enables automated cell type annotation of novel pan cancer studies via architecture surgery.

Taken together, this work provides novel insights into the pan cancer tumour microenvironment, enabled by state of the art integration approaches. Additionally, cancer-spanning cell type gene signatures are derived and a TME reference atlas is generated, allowing for query data mapping, automated cell type annotation and iterative atlas extension by the scientific community. Moreover, they demonstrate how such approaches can be deployed to perform TME composition-based patient stratification and therewith further personalize clinical practice. Indeed, it has become increasingly apparent that different patients who suffer from the same cancer type might have strikingly dissimilar TME cell compositions affecting the success of immunotherapy and requiring more personalized medicine approaches (Duan et al., 2020). The approach described herein provides further support for such TME diversity and at least elements of it verify across multiple cancer types such that an improved characterisation can be obtained by leveraging information across multiple cancer types.

### Example 1 - Full TME integration for identification of cancer cells, integration and annotation of non-malignant cells

### Methods

### Study selection

Studies integrated via the first trVAE (full TME, see Figure 4A and below) had to fulfil several inclusion criteria. That is, only raw count cancer studies of human origin encompassing multiple cell types, including solid tumour cells, and more than 1000 T cells were considered. Hematological malignancies were excluded to avoid ambiguities between immune and tumour cells. Data included in the second trVAE integration comprises all non tumour cells of the first trVAE integration together with studies that fulfil all the above criteria, except that the absence of tumour cells was required (see Figure 4A).

### Study curation

Individual scRNA-Seq studies were downloaded from publicly accessible repositories and metadata annotation was stored in a curated format. If available, fastq files were downloaded and processed via an in-house cellranger pipeline to yield count matrices in mtx format. An exhaustive list of all public scRNA-Seq studies used in this analysis can be found in Table 1.

### Processing of raw count data

Each study was separately pre-processed using scanpy (https://scanpy.readthedocs.io/en/stable/index.html). To remove unreliable observations, only cells which expressed at least 200 genes and only genes expressed in at least 3 cells were retained. Subsequently, damaged and dying cells were removed by discarding cells with mitochondrial gene count above 10% of the total gene expression count, or discarding the 1% of cells with highest mitochondrial count, if the 99% quantile was below 10%. If required, the cutoff was adjusted manually. Further outlier removal was performed by filtering out cells in which the number of expressed genes exceeded the minimum of the value of the 99% quantile or 10000 genes. UMI counts per gene and cell were normalized (divided) by the gene count per cell and log-transformed (log1p values). Integration used 4000 highly variable genes and the custom set of marker genes below selected from publicly available data (see Table 2 and Nieto et al., 2021) and prior knowledge of the biology modelled.

*Custom marker genes:* AASS, ACTA2, ACTC1, ADH7, AGR2, AIF1, ALDH1L1, APOBEC3A, APOC1, APOE, AQP4, ASAH1, ASGR1, ASPN, AXIN2, AXL, AZU1, BATF3, BCL3, BCL11A, C1QA, C1QB, C1QC, CA2, CALD1, CCL2, CCL3, CCL4, CCL5, CCL13, CCL17, CCL19, CCL21, CCL22, CCR1, CCR6, CCR7, CCR8, CD1C, CD1D, CD1E, CD2, CD3D, CD3E, CD3G, CD4, CD6, CD8A, CD8B, CD9, CD14, CD19, CD24, CD27, CD28, CD37, CD38, CD44, CD68, CD69, CD72, CD79A, CD79B, CD81, CD83, CD84, CD96, CD160, CD163, CD200, CD207, CD209, CD244, CD300E, CDK1, CHL1, CLC, CLDN4, CLDN7, CLEC1B, CLEC4C, CLEC4G, CLEC9A, CLEC10A, CNN1, COL1A1, COL1A2, COL5A1, CPA3, CRIP2, CRTAM, CSF1R, CSF3R, CSPG4, CST7, CTHRC1, CTLA4, CTNNA2, CTSG, CXCL8, CXCL13, CXCR3, CXCR4, CXCR5, DCN, DCT, DSG1, EGFR, ELANE, ELF3, ENHO, ENTPD1, EOMES, EPCAM, EXD3, F3, FABP4, FABP7, FAM3C, FAP, FBLN1, FCER1A, FCGR3A, FCGR3B, FCN1, FCN2, FDCSP, FEZ1, FHL1, FLNA, FLT3, FOLH1, FOXP3, FSCN1, FXYD3, GADD45A, GAP43, GATA2, GATA3, GDF15, GNLY, GPBAR1, GPR17, GZMA, GZMB, GZMH, GZMK, GZMM, HAVCR2, HBA1, HBB, HDC, HHIP, HLA-DQA1, HLA-DRA, HLA-DRB1, HMGB2, HPGDS, HSD11B1, HSPB1, ICAM4, ICOS, IDO1, IFI6, IFI16, IFI27, IFI35, IFI44, IFI44L, IFIH1, IFIT1, IFIT2, IFIT3, IFIT5, IFITM1, IFITM2, IFITM3, IFNG, IGHG1, IGHM, IGKC, IL1A, IL1B, IL2, IL2RA, IL3RA, IL4, IL6, IL6ST, IL7R, IL8, IL33, IRF4, IRF7, IRF8, IRF9, ISG15, ITGAM, ITGAX, KIT, KLK3, KLRB1, KLRC1, KLRD1, KLRF1, KLRK1, KRT1, KRT5, KRT8, KRT18, KRT19, L1CAM, LAG3, LAMP3, LAYN, LEF1, LGR5, LILRA4, LILRB2, LILRB4, LRRN4, LST1, LUM, MAGEA4, MBP, MCAM, MCEMP1, MDK, MEST, MGST1, MITF, MKI67, MLANA, MMP9, MMRN1, MPO, MS4A1, MS4A2, MS4A3, MTSS1, MYH11, NCAM1, NCR1, NEAT1, NFIB, NKG7, NOTCH3, NOVA1, NPNT, NTSR2, NUDT17, OPALIN, PCNA, PDCD1, PDGFRB, PERGL, PERP, PFN2, PI16, PILRA, PLA2G7, PLN, PLVAP, PMEL, PMP2, POSTN, PRDM1, PRF1, PROX1, PRTN3, PSPH, PTPRC, RALB, RGS5, RORC, RUNX2, S100A8, S100A9, S100A12, S100A16, SBDSP1, SDC1, SELL, SEPP1, SESN3, SHTN1, SIGLEC10, SMIM22, SMTN, SOX2, SOX4, SOX9, SOX10, SPI1, SPIB, SPN, SPP1, STAB1, STAT4, STMN1, TACSTD2, TAGLN, TBX21, TCF4, TCF7, TCF7L2, TFF3, TGFB1, TIGIT, TNF, TNFRSF4, TNFRSF9, TNFRSF17, TNFRSF18, TNFSF8, TOX, TOX2, TPSAB1, TPSB2, TPST1, TRAC, TRBC1, TRBC2, TRDC, TRGC1, TRGC2, TRIM9, TSPAN13, TUBA1B, TXN, TYMS, TYR, TYRP1, UPK3B, VCAN, VPS37B, VWA5A, VWF, WIF1, XCL1, XCL2, XCR1, and ZNF366.

### Training trVAE

Transfer variational autoencoders were trained for 50 epochs, respectively. To avoid overfitting, early stopping was enabled using val_unweighted_loss as criterion, threshold of 0, patience of 20, reducing Ir, Ir patience of 13, and Ir factor of 0.1. Hidden layer sizes were set to [128, 128] and alpha_epoch_anneal to 200. Exclusively working with raw count data, the trVAE loss function was set to nb.

### Tumour cell identification

Following trVAE integration of tumour containing scRNA-Seq raw count data, marker genes for tumour-(EPCAM11-14, MLANA15, KRT816), immune- (PTPRC) and endothelial cells (VWF, PLVAP) and fibroblasts (COL1A1, COL5A1, COL1A2, LUM, FBLN1) and pericytes (RGS5, CNN1, MYH11, SMTN, ACTA2, TAGLN, CALD1) were defined using prior knowledge and literature mining. For each cell, the mean, log1p-transformed marker expression within each of the above cell type groups was computed. Next, a single cell tumour score was calculated by subtracting the maximum mean expression of genes corresponding to healthy cell types from the mean expression of tumour genes. Subsequently, the tumour score was scaled by division with its absolute maximum and kernel density estimation (KDE) was applied to identify a tumour score threshold suitable for cancer cell separation. The latter was set to the positive minimum of the tumour score density distribution. In the following, single cell tumour scores were averaged within each trVAE-integration derived Leiden cluster. Clusters with mean tumour score below the threshold were labelled as healthy, whereas clusters with mean tumour score above the cutoff were considered cancerous. To not solely rely on the expression of few indicator genes for malignant cluster calling, the 10-dimensional latent space mean of all healthy cluster cells and all cancer cluster cells identified so far was computed. Finally, the latent space Euclidean distance from each cluster mean to the healthy- and malignant mean was computed, respectively and each cluster was assigned to the closer match.

### Manual cell type annotation and subclustering

trVAE integrated Leiden clusters of stromal and immune cells were manually annotated using several indicators. For each cluster, signature scores were computed as the median difference between mean marker gene expression within the cluster and all other clusters for each cell type listed in Table 2. The highest scores per cluster were considered as indicators of overall cluster identity. In a complementing, unbiased approach, top ranked genes resulting from a wilcoxon test on cluster level were reviewed to aid cluster annotation. In addition, distribution of donors and studies over each cluster was considered to identify potentially missed tumour or tissue specific clusters. Whenever an intracluster marker gene distribution heterogeneity was identified, subclustering was performed with custom leiden resolution. In case unsupervised subclustering agreed with observed marker gene expression patterns, subclusters were annotated separately to maximize annotation accuracy.

### Results

### Full TME study integration and cancer cell identification

13 cancer studies of varying cell counts were downloaded and processed, including head and neck squamous cell carcinoma (HNSCC), hepatocellular carcinoma (HCC), colorectal cancer (CRC), different types of lung cancer (LC), clear cell renal cell carcinoma (ccRCC), prostate cancer (PC), breast cancer (BC), bladder urothelial carcinoma (BUC), esophageal squamous-cell carcinoma (ESCC), uveal melanoma (UV) and cutaneous squamous cell carcinoma (cSCC), as described in Table 1 and Figure 4B. After pre-processing according to the method section, the data comprised 757967 cells from 157 patients (207 samples from 10 studies) that differed in cancer type, harvest location, specimen pathology and FACS sorting strategy (Fig. 4C,D). A two-step data integration procedure was designed to deal with the mixture of cell sorted and non-cell sorted samples (Figure 4A).

**Table 1. Studies details. Seq. tech.= sequencing technology. Ext=external ID. Int=internal ID.**

| **Study ID (ext. / int.)** | **Seq. tech.** | **Study Title** | **Cancer type** | **Source** |
|---|---|---|---|---|
| GSE1 39324 / 32 | 10x Genomics | Immune landscape of viral and carcinogen-derived head and neck cancer | Head and Neck Cancer Immune cells | Ruffin et al., 2021 |
| GSE1 49614 / 36 | 10x Genomics | A Single-Cell Atlas of the Multicellular Ecosystem of Primary and Metastatic Hepatocellular Carcinoma | Hepatocellular Carcinoma | Li et al., 2021 https://www.ncbi.nl m.nih.gov/geo/quer y/acc.cgi |
| GSE1 44735 / 37 | 10x Genomics | Single cell 3 RNA sequencing of 6 Belgian colorectal cancer patients | Colorectal cancer | Lee et al., 2020 https://www.ncbi.nl m.nih.gov/geo/quer y/acc.cgi?acc=GS E144735 |
| GSE1 23902 /38 | 10x Genomics | The single cell transcriptional landscape of human lung adenocarcinoma | Human Lung Adenocarcinoma | Laughney et al., 2020 |
| GSE1 21638 / 48 | 10x Genomics | Single-cell sequencing of peripheral blood and tumour-infiltrating immune cells in renal clear cell carcinoma | Renal cell carcinoma TME and blood | Borcherding et al., 2021 |
| GSE1 41445 / 49 | 10x Genomics | Single cell analysis reveals onset of multiple progression associated transcriptomic remodellings in prostate cancer | Prostate cancer | Chen et al., 2021 |
| GSE1 41665 / 50 | 10x Genomics | Single-cell RNA-sequencing of gd-T Cells from Peripheral Blood and Breast | Breast and blood cancer | Boufea et al., 2021 |
| PRJN A6620 18/51 | 10x Genomics | Single-cell RNA sequencing highlights the role of inflammatory cancer-associated fibroblasts in bladder urothelial carcinoma | bladder urothelial carcinoma | Chen et al., 2020 |
| GSE1 60269 / 54 | 10x Genomics | Dissecting esophageal squamous-cell carcinoma ecosystem by single-cell transcriptomic analysis | Esophageal squamous-cell carcinoma | Zhang et al., 2021 https://ngdc.cncb.a c.cn/gsa-human/browse/HR A000195 |
| PRJN A7739 87 / 56 | 10x Genomics | Early Lung Carcinogenesis and Tumour Microenvironment Observed by Single-Cell Transcriptome Analysis | Lung cancer | Kim et al., 2022 |
| GSE1 39829 / 57 | 10x Genomics | Single-cell analysis reveals new evolutionary complexity in uveal melanoma | Uveal melanoma | Durante et al., 2020 |
| GSE1 48071 / 58 | Singleron | Single-cell profiling of tumour heterogeneity and the microenvironment in advanced non-small cell lung cancer | Non-small cell lung cancer | Wu et al., 2021 |
| GSE1 44240 / 60 | 10x Genomics | Multimodal Analysis of Composition and Spatial Architecture in Human Squamous Cell Carcinoma | Squamous Cell Carcinoma | Ji et al., 2020 |

Initially, pre-processed, tumour cell containing studies were jointly integrated via scArches trVAE using the expression values of 4000 highly variable genes together with known marker genes (see methods above; Figure 4A full TME branch). The scArches package documentation recommends the use of 2000-5000 genes usually perform well. The inventors tested 2000, 3000 and 4000 genes and the latter was found to be marginally better. The genes were identified using the provided functionality in scanpy. Integration resulted in a 10-dimensional, latent representation of each cell. Further dimensionality reduction was performed using neighbourhood graph computation and Leiden clustering, followed by Uniform Manifold Approximation and Projection (UMAP30). This approach yielded 69 Leiden clusters, which could be harbouring malignant cells, healthy cells or a mix of the above.

To distinguish malignant cells from healthy cells, several canonical marker genes of healthy stromal and immune cells were visualized together with genes frequently overexpressed in cancer (Figure 5C). The expression of distinctive marker genes showed strong localisation and minimum overlap, thereby justifying a cluster level annotation of malignant cells, since no intermixing was apparent. Initially, a single cell tumour score was computed by subtracting the maximum mean expression of canonical marker genes of healthy stromal and immune cells from the mean of genes typically overexpressed in cancer (mean(EPCAM, MLANA, KRT8) - max(PTRC, mean(COL1A1, COL1A2, COL5A1, LUM, FBLN1), mean(RGS5, CNN1, MYH11, SMTN, ACTA2, TAGLN, CALD1), mean(VWF, PVLAP))). The tumour genes used encompass EPCAM, MLANA and KRT8. PTPRC was used as a marker of immune cells. The collagen genes COL1A1, COL1A2, COL5A1 and LUM and FBLN1 served as fibroblast indicators. Pericyte identity was assessed via the expression of RGS5, CNN1, MYH11, SMTN, ACTA2, TAGLN and CALD1, whereas VWF and PVLAP were used as markers of endothelial origin.

Next, kernel density estimation was applied to visualize the single cell tumour score distribution and identify a suitable local-minimum-density tumour score (chosen as the minimum on the positive side of the tumour score distribution, Figure 5A). The latter served as a threshold for cluster-averaged tumour scores, enabling the assignment of healthy / malignant status to clusters below / above the threshold. To further refine this high level cancer cluster annotation and avoid solely relying on few marker genes, the latent space mean vector of healthy and cancerous clusters was computed, respectively. Finally, each cluster was assigned to the malignancy state its latent space mean vector had the smaller Euclidean distance to. This assignment enabled a clear distinction between healthy and malignant cells with minimal intermixing (Figure 5C). As an additional confirmation of meaningful cancer cluster assignment, the Donor ID entropy was computed for each cluster, hypothesizing that tumour heterogeneity would hinder integration with other cell types and clusters. As expected, the majority of assigned cancerous clusters was highly donor-specific and thus showed low entropy, whereas clusters assigned as healthy were overly characterized by high Donor ID entropy (Figure 5B).

### Identification of subsets of malignant cells

Having identified malignant cells, the inventors set out to further divide the potentially malignant cells into cells which are likely tumorous and cells which are likely healthy keratinocytes, melanocytes, epithelial cells or others. Thus, single cell copy number variations (CNVs) were inferred from scRNA-Seq data for each study independently, using inferCNVpi (see https://bioconductor.org/packages/devel/bioc/vignettes/inferenv/inst/doc/inferCNV.html). This approach computes a score that quantifies expression intensity of genes across positions of the genome in comparison to the average or a set of reference 'normal' cells. High scores are indicative of cells that are likely affected by copy number aberrations. Thus, a score is obtained for each single cell. These scores were then averaged on the level of samples and cell types to obtain sample and cell type CNV scores (although the single cell CNV features were used to obtain CNV clusters, see below). In addition, principal component analysis (PCA), neighborhood graph computation, Leiden clustering and UMAP projection were applied to the data in CNV space. The data in CNV space comprises a CNV estimate for each of a plurality of positions (implemented as a sliding window, e.g. averaging expression over 100 gene stretches). The donor entropy was calculated for each Leiden cluster in CNV space. For the separation of potentially tumorous cells into cells with high and low tumor potential, a single-split decision tree was trained to discover the most informative feature and associated threshold for the prediction of normal / normal adjacent sample origin. This was used because some studies did not contain normal / normal adjacent samples as reference and even samples of malignant origin could still harbor a fraction of healthy cells. Thus, a decision tree was trained on the inferred CNV score, a percentile ranked CNV score with increased comparability between studies, and the donor ID entropy for each cluster in the trVAE latent- and CNV space, respectively (see Figure 5C, second page). The tree was trained to predict which cells are normal / normal adjacent tissue, or anything else (i.e. a binarized version of the specimen pathology - see Figure 5C, second page). Due to the high heterogeneity of cancer, the donor specificity of expression patterns was hypothesized to be a potential indicator of normal / cancerous origin. That is, clusters with low donor ID entropy should tend to be malignant while clusters with shared expression patterns between multiple donors should tend to be healthy cells. Decision tree overfitting was avoided by training on 70% of the data and testing on the remaining 30%, while only allowing a single split. The most informative feature was the donor ID entropy in CNV space, which yielded an accurate prediction of specimen pathology type in 81% of cases. Hence, the decision tree derived threshold on donor ID entropy in CNV space was used to classify all potentially tumorous cells into cells with high / low tumor potential, guided by the use of normal (adjacent) reference during classifier training.

### Integration and annotation of non-malignant cells

To facilitate healthy cell type / state annotation, previously identified, malignant cells were removed and the remaining, healthy immune and stromal cells jointly trVAE-integrated with cells from cancer studies that underwent immune cell enrichment (primarily CD45+ sorting) prior to sequencing (studies in Table 1 except studies 36, 37, 38, 49, 51, 56, 57,58, 60, 63 which were the unsorted studies used in the previous step). Afterwards, neighbourhood graph computation, Leiden clustering and UMAP visualization were performed in the latent space. Each cluster was annotated based on a multitude of indicators, including study- and donor contribution (to identify clusters that are donor-specific or study-specific, as donor specific clusters are often cancer clusters due to the high heterogeneity in cancer, and study specific clusters can be disease and thus cancer specific, but could also be tissue specific, e. g. alveolar macrophages were identified as distinct, partially because they are enriched in lung studies), top differentially expressed genes (DEGs; Wilcoxon test; examined manually with expert knowledge) and expression of marker genes taken from literature and prior knowledge (Table 2, Figure 6A, Figure 6C). Additionally, re-clustering of clusters was performed whenever a heterogenous, intracluster marker gene distribution could be identified. In case unsupervised re-clustering of individual clusters yielded subclusters which aligned well with marker gene expression patterns, the subclusters were annotated as distinct cell types / states, despite belonging to the same high level cluster. This means that loss of biologically relevant information due to the choice of clustering resolution was minimized.

During annotation, 29 different cell types / states could be identified, including regular and proliferative B cells, Plasma cells, Mast cells, 5 subsets of dendritic cells, CD14+ and CD16+ Monocytes, M2 and alveolar Macrophages and different states of CD4+ and CD8+ T cells and NK cells. Moreover, several stromal cells such as Fibroblasts, Pericytes and different endothelial subtypes were identified. Of note, some cell types are indication specific, e.g. alveolar macrophages in lung malignancies (such as adenocarcinoma) or neuronal cells in Glioblastoma. The model does not capture the indication information in training. However, as demonstrated above, they were nonetheless able to identify these cells as separate clusters.

B cells showed strong MS4A1 and CD79A expression, while Plasma cells had high expression levels of Immunoglobulin chain genes, including IGKC, IGHG1, IGHG3 and IGHG4. Mast cells were identified by their expression of TPSB2, TPSAB1 and KIT. The most prevalent, conventional dendritic cell (cDC) subset was cDC2 dendritic cells, characterized by expression of CD1C. However, cDC1 and cDC3 were also present, as indicated by the expression of CLEC9A and LAMP3, respectively. Plasmacytoid dendritic cells expressed GZMB, LILRA4 and TSPAN13, while langerin dendritic cells were CD207+. CD14+ Monocytes showed strong proinflammatory properties, as indicated by high expression of the S100 genes S100A8 and S100A9, whereas CD16+ monocytes expressed LST1, FCGR3A and LILRB2. M2 Macrophages displayed high levels of C1QA, C1QB, C1QC. The unifying feature of T cells was CD3 expression (CD3D, CD3E, CD3G), whereas NK cells presented the cytotoxicity markers NKG7, GNLY, GZMA and GZMB. Regulatory CD4+ T cells expressed FOXP3 and naive CD4+ T cells ISG15 and MX1. Exhausted CD8+ T cells displayed high levels of the exhaustion markers LAG3 and TIGIT.Proliferative subset were identified for B, CD4+ and CD8+ T cells, which co-expressed their respective cell type markers with proliferation markers such as STMN1, HMGB2, MKI67 and TUBA1B. In the group of stromal cells, fibroblasts exhibited high expression levels of collagen genes including COL1A1, COL1A2 and COL3A1 as well as DCN and LUM, whereas Pericytes expressed CALD1, MYL9 and RGS5. VWF and PVLAP served as markers for regular endothelial cells, whereas lymphatic endothelial cells had high levels of CCL21, TFF3, MMRN1, and PROX1. Liver-specific endothelial cells on the other hand were enriched in FCN2, CLEC1B and CLEC4G.

**Table 2. Hierarchical description of cell types annotated.**

| **Cell type** | **Parent** | **Genes** | **Source** | **Type** |
|---|---|---|---|---|
| Cytotoxic | | CCL5, GZMA, GZMK, NKG7, GNLY, GZMH, GZMM, PRF1, CXCR3, GZMB, CCL4, IFNG | | cell_state |
| Proliferative | | STMN1, MKI67,CDK1, TUBA1B, PCNA | | cell_state |
| Proinflammatory | | IL1B, CXCL8, NEAT1 | Vanderbeke etal. 2021 | cell_state |
| T | | TRAC,TRBC1,TRBC2,CD 3D, CD3E, CD3G | | immune |
| T_CD4 | T | CD4 | | immune |
| T_CD8 | T | CD8A,CD8B | | immune |
| Treg | T_CD4 | FOXP3, IL2RA | | immune |
| T_gamma_delta | | TRDC,TRGC1,TRGC2, CD3E | | immune |
| T_gamma_delta2 _MacParland201 8 | | STMN1,HMGB2,TYMS | MacParland et al., 2018, Figure 2 | immune |
| T activated | T | CD69,IL2RA | | immune |
| T_CD4_Follicular Helper | T_CD4 | CD200, TOX, TOX2, CXCR5, ICOS | | immune |
| T_exhausted | T | PDCD1, LAG3, HAVCR2, TIGIT, SPN, CTLA4, ENTPD1, TNFRSF4 | | immune |
| T_CD4_exhauste d | T_CD4, T_exhausted | | | immune |
| T_CD8_exhauste d | T_CD8, T_exhausted | | | immune |
| Treg_exhausted | Treg, T_exhausted | | | immune |
| Th17 | T_CD4 | TNFSF8, IL7R, CXCR4, VPS37B, GZMK, STAT4, TGFB1, XCL1, XCL2, CCR7, CRTAM | | immune |
| T_naive | T | IL7R, TCF7, CCR7, LEF1, SELL | | immune |
| T_CD4_naive | T_CD4, T_naive | | | immune |
| T_CD8_naive | T_CD8, T_naive | | | immune |
| T_CD4_Proliferati ve | T_CD4, Proliferative | | | immune |
| T_CD8_Proliferati ve | T_CD8, Proliferative | | | immune |
| T_CD4_recently_ activated | T_CD4 | CCL4, IFITM1, CD69, PRF1, BCL3, IL7R,IFITM3, TCF7, CD81, CXCR4, GZMK, GZMM, IFITM2 | | immune |
| T_CD4_Naive_m emory | T_CD4 | IL7R, TCF7, CCL5, IFITM1 | | immune |
| T_CD8_Terminall y_exhausted | T_CD8 | CXCL13, LAG3, GZMB, CCL5, NKG7, IFNG, GZMA, HAVCR2, GNLY, PDCD1, TIGIT, TNFRSF9, ENTPD1,CTLA4, PRF1, TOX, GZMH, GZMK | | immune |
| T_CD8_Effector_ memory | T_CD8 | GZMM, IFITM1, GZMK, IFNG, CCL5 | | immune |
| T_CD4_Transitio nal_Memory | T_CD4 | CD2, CD28, CD44, CD6, CD69, CD96, CTLA4, IL6ST, IL7R, FLNA, SESN3 | | immune |
| T_CD8_pre-exhausted | T_CD8 | ISG15, IFI44L, IFI6, IFIT3, IFIT1, IFI44, IFI35, IRF7, IFIT2, LAG3,IFITM1, IFI16, IFI27, IFNG, GZMB, GZMK, PRF1, HAVCR2,IFIH1, GZMA, IRF9, CXCL13, GZMH, IFIT5, PDCD1 | | immune |
| Fibroblast | | COL1A1, COL5A1,COL1A2, LUM, FBLN1 | | non_imm une |
| B | | MS4A1, CD27, CD79A, CD79B,CD83, CD37, CD19 | | immune |
| B_naive | B | IGHM, CD72 | | immune |
| B_memory | B | IGHG1, CD27 | | immune |
| B_proliferative | B, Proliferative | | | immune |
| Plasma | | IGKC, IGHG1, IGHM, TNFRSF17, SDC1, CD38 | | immune |
| Endothelial | | VWF, PLVAP | | non_imm une |
| Endothelial_lymp hatic | | CCL21, TFF3, MMRN1, PROX1 | Arora & Pal, 2021 | non_imm une |
| Endothelial_liver_ sinusoidal | | FCN2, STAB1, CLEC1B,CLEC4G | Andrews et al., 2021 | non_imm une |
| | | | Wang et al., 2021 | |
| pDC | | IL3RA, GZMB,TSPAN13, LILRA4,TCF4, IRF8, IRF4, BCL11A, SPIB, CLEC4C, RUNX2 | | immune |
| cDC1 CLEC9A | | CLEC9A, FLT3,IDO1 | | immune |
| cDC2_CD1C | | CD1C, FCER1A,HLA-DQA1 | | immune |
| cDC3_LAMP3 | | LAMP3, CCR7,FSCN1 | | immune |
| mDC | | HLA-DRA, HLA-DRB1, SPI1, CD68, CD83, ITGAX, CD1D | | immune |
| DC_Langerin | | CD207 | Merad, Ginhoux & Collin, 2008 | immune |
| DC_Follicular | | FDCSP | Wu et al.,2021, see supplementary material. | immune |
| Mast | | TPSB2, TPSAB1, CPA3, GATA2, KIT, MS4A2 | | immune |
| NK | | PRF1, GNLY, KLRD1, KLRF1, GZMH, GZMB, KLRB1, GZMA, GZMM,CD160, CD244, KLRC1, NCR1 | | immune |
| Monocytes | | S100A8, S100A9, FCN1, VCAN, AIF1, SPI1, CD14, APOBEC3A, CSF1R, ASAH1 | | immune |
| Macrophage_or_ Monocyte | | CD14, CD68 | | immune |
| TAM_SPP1 | Macrophage _or_Monocyt e | SPP1, APOE, SEPP1, MMP9, CD163 | | immune |
| TAM_M2 | Macrophage _or_Monocyt e | C1QB, APOE, C1QA, C1QC, APOC1, SEPP1, SPP1, CD163 | | immune |
| TAM_proinflamm atory | Macrophage _or_Monocyt e | CXCL8, IL1B, S100A9, S100A8, CCL2, IL8, CD68, IL6, IL1A | | immune |
| Macrophage_alve olar | Macrophage _or_Monocyt e | FABP4, LSAMP, ATP10A | Poli et al.,2021.see supplementary-material | immune |
| Monocyte_CD14 | Macrophage _or_Monocyt e | CD14,FCN1,S100A8, S100A9 | Cheng et al., 2021 | immune |
| Monocyte_CD16 | Macrophage _or_Monocyt e | FCGR3A, LST1, LILRB2 | Cheng et al., 2021 | immune |
| Erythrocyte | | HBB,HBA1 | | non_imm une |
| Pericyte | | RGS5,CNN1, MYH11, SMTN, ACTA2, TAGLN, CALD1 | | non_imm une |
| Keratinocyte | | KRT1, DSG1, KRT5 | | non_imm une |
| Melanocyte | | SOX10, MITF, DCT, MLANA, PMEL, TYR, TYRP1 | Wang et al., 2020 | non_imm une |
| Unknown | | | | |
| Neuronal | | NCAM1,MBP,OPALIN,GP R17, L1CAM, ALDH1L1,WIF1, NTSR2 | Guslund et al., 2020 | non_imm une |
| Not_annotated_y et | | | | |
| Tumour_solid | | KRT18, KRT8, MGST1, ELF3, CLDN4, PERP, FXYD3, KRT19, CD9, TACSTD2, HSPB1, TXN, AGR2, MDK, GDF15, CD24,SMIM22, CLDN7, SOX4, EPCAM | | tumour |
| Muscle_smooth | | PERGL,PLN,MYH11 | Rubenstein et al.,2020. | non_imm une |

Having finalized the cell type annotation, the inventors computed differentially expressed genes, comparing the transcriptome of each cell type (in particular, identifying differentially expressed genes in each cluster, their expression levels and the fraction of cells expressing each gene identified as differentially expressed) to all other cell types in the data (Wilcoxon test). The results agreed with and extend previous knowledge on marker genes for scRNA-Seq and can be leveraged for improved cell type annotation (Figure 6C). UMAP plots coloured by selected marker genes are shown in Figure 9. Holistically viewed, the majority of all annotated cells were T cells, followed by myeloid cells, B/ Plasma cells, stromal and NK cells (Figure 6B, Figure 6E). T CD8 cells tended to rather show an exhaustion phenotype, while CD4+ T cells rather had naive properties.

### Example 2 - Characterisation of the pan cancer TME

### Methods

### See Example 1.

### Signatures

Cell type signatures are obtained by identifying differentially expressed genes in each annotated cluster compared to other clusters (p value and log fold change).

### Results

To investigate commonalities and differences between the TME immune composition across studies, samples, indications, pathologies and harvest locations, as well as the co-appearance/ mutual exclusion of cell types, the relative contribution of each cell type to its sample was computed. For this purpose, FACS pre-sorted studies were removed prior to the analysis. Hierarchical clustering of the pan-cancer TME composition yielded 5 clusters (Figure 7A) of different sizes. Cluster 1 consisted of 57 samples from 9 studies and diseases, 17 harvest locations and 27 cell types, where the vast majority were tumour cells (Figure 7B). Thus, cluster 1 resembles the pan-cancer TME subset with lowest immune cell infiltration. Cluster 2/ 3 encompasses merely 4/ 5 colorectal cancer samples, with cluster 2 hosting exclusively normal mucosa and cluster 3 hosting core and border region samples. While cluster 2 was dominated by B and Plasma cells, cluster 3 was dominated by stromal cells, most of which were fibroblasts. The largest cluster, cluster 4, contained 70 samples from 9 studies, 10 diseases and 17 different harvest locations and was dominated by tumour cells. However, cluster 4 showed considerable immune invasion compared to cluster 1, as evident by the relatively higher frequency of various subsets of T, B, myeloid and Plasma cells. Finally, cluster 5 consisted of 34 samples from 7 studies of 6 diseases from 10 harvest locations. Cluster 5 contained merely 10% tumour cells and therefore has the least tumour content across all samples. Additionally, it comprised largely tumour-adjacent samples, especially of lung and hepatocellular carcinoma and was enriched in T cells, NK cells and M2-like tumour associated macrophages (TAMs).

Taken together, the overall clustering reflects the sample tumour burden with cluster 1 having an average tumour burden of 85%, cluster 4 of 35% and cluster 5 of 11%. With the exception of the smallest clusters 2 and 3, which were indication-specific, all clusters contained multiple indications and harvest locations, thereby highlighting that the composition of the TME and degree of immune infiltration cannot readily be inferred from the cancer indication, and highlighting that stratification according to tumour burden is a property that is common across multiple cancer types. Interestingly, the low tumour cluster 5 showed a significantly higher ratio of CD8+ T cells to regulatory T cells than the medium tumour cluster 4 or the high tumour burden cluster 1 (Figure 7C). The CD8/Treg ratio has been previously demonstrated to correlate with clinical outcome, with a higher ratio being linked to enhanced outcome (Preston et al., 2013). This is in alignment with expectation, that hot (inflamed) tumours tend to show lower relative representation of immunosuppressive, regulatory T cells compared with cold (low immune infiltration) tumours.

To further understand generic, molecular drivers associated with varying degrees of immune infiltration, cell type specific gene expression comparison between the different clusters was performed (Wilcoxon test). It became evident that the fraction of CD8+ T cells expressing cytotoxicity markers such as NKG7, KLRD1, and GZMH as well as their mean expression of these was lower in the tumour rich cluster 1, medium in cluster 4 and the highest in the immune rich cluster 5. The same holds true for the expression of the proinflammatory CC chemokine ligands 4 and 5 (CCL4, CCL5), which are being evaluated for cancer therapy (Liang et al., 2016). By analogy and despite the apparent challenge of unravelling pan-cancer patterns due to cancer heterogeneity, several human leukocyte antigens (HLA) genes were consistently lower in mean and fraction of expressing cells in cluster 1, compared to cluster 4, which again showed lower expression than cluster 5. Consequently, active immune escape mechanisms which downregulate the cellular antigen presentation machinery can be considered a pan cancer strategy to help minimize immune infiltration. Among the most differentially expressed macrophage genes across clusters were FABP4 and MARCO, which were higher in frequency and mean expression in cluster 5 than in cluster 4, which had higher expression than cluster 1. The above genes are indicative of alveolar macrophages, which corresponds well with the overrepresentation of lung tissue in cluster 5, thus suggesting an antitumour activity of alveolar macrophages, however pro-tumour functions have also been previously observed (Almatroodi et al., 2014).

Next, significantly differentially expressed genes per cell type were subjected to a gene set enrichment analysis (GSEA). Genes significantly overexpressed in cluster 5 vs the immune excluded cluster 1 indicated to strong activity of inflammatory response and T cell activation pathways in dendritic cells, and elevated cytotoxicity by NK and CD8+ T cells, which were virtually absent in the immune-excluded cluster 1.

### Example 3 - Knowledge transfer and reproducibility assessment via scANVI

### Methods

### Training scANVI

SCVI was trained using 2 layers, disregarding covariates, using "both" as layer norm and no batch norm. Study ID was used as batch key. trVAE-integration derived cell type annotation was used as label key. scVI (Lopez et al., Nature Methods volume 15, pages1053-1058 (2018)) is a framework for normalisation and analysis of gene expression in single cells. It uses stochastic optimisation and conditional variational autoencoders to aggregate information across similar cells and genes and to approximate the distributions that underlie observed expression values while accounting for batch effects. The implementation in the scArches package (Lotfollahi et al., 2022) was used in this work.

scANVI (Xu et al.) is a semi-supversied method that builds upon a scVI model, using any cell type annotations available during autoencoder training to improve latent representation of the data. Thus, it can be used to leverage knowledge for a subset of cells in a dataset to annotate a data set of unlabelled cells. The implementation in the scArches package (Lotfollahi et al., 2022) was used in this work. scANVAE was trained based on SCVI with a maximum of 20 epochs, using a minimum of 500 samples per label to balance the data injected into the network in each epoch and mitigate any effect caused by unbalanced input data. In case no cell type was less frequent than 500, the minimum cell type frequency was used. Atlases were trained on the union of 4000 highly variable genes and custom marker genes (listed in Table 2), leaving out one study for later validation, respectively. Cells with unidentified type, merely state (e.g. "proliferative" cells that show clear proliferation marker, but cannot be assigned to a clear cell type) or high level annotation (e.g. "T" instead of "T_cd4" or "T_cd4_naive" which could cause problems in distinguishing T from T_cd4) were removed prior to atlas training and testing to avoid artifacts and restrict training/ testing to high quality data. Query studies were mapped to the gene space of their corresponding atlas by imputing missing genes with 0 and discarding genes absent in the atlas. Next, query studies were projected onto their atlas, via training for a maximum of 100 epochs, freezing dropouts, using a weight decay of 0 and checking values every 10 epochs.

### Results

Raw count studies were loaded, normalized, log1p transformed and annotated with the previously established, trVAE-based cell type annotation. Next, a gene subset was constructed, containing 4000 highly variable genes and additional, canonical cell type marker genes. (Figure 7A). After sparsity removal, a scVI network was trained, from which a scANVI network was constructed and trained. Cell type prediction was performed and evaluated using several performance metrics. A neighbourhood graph was constructed on the latent representation, followed by Leiden clustering and UMAP visualization (Figure 8D).

The inventors evaluated the performance of scANVI on multiclass cell type prediction using leave-one-out cross validation. That is, 12 atlases were generated based on 11 training studies and used to predict the cell types of the remaining test study, respectively. This was performed using architecture surgery as described in Lotfollahi et al., 2022 to project each reference study in the scANVI leave one out cross-validation onto the atlas and derive cell type predictions for the test study from the model trained on n-1 training studies. Prediction accuracy was analysed on different, hierarchical levels of cell type nomenclature to capture the effect of annotation detail on model performance (e.g. level 1 = all T cells, level 2=cd4+ T cells, level 3= exhausted cd4+ T cell). On the most coarse-grained level (Level1 - Levels in Table 2 that do not have a parent, e.g. T), good prediction accuracy was obtained, whereas more detailed annotation (Level2 - Levels in Table 2 that have as parent an annotation that does not have a parent, e.g. T_CD4) yielded accuracies as shown on Figure 8C. The most precise annotation (Level3, levels in Table 2 that have a parent that has a parent, e.g. T_CD4_exhausted) still resulted in good prediction accuracies from. Additionally, normalized accuracies were computed by averaging the absolute prediction accuracies for each cell type to reduce potential reporting metric bias due to nonuniform cell type distribution within studies. Incorrect predictions typically occurred at the border of clusters / cell types, which is to be expected (Figure 5C). Generally, wrong predictions were characterized by a lower cell type probability, as visualized by its logit-transformed counterpart (Figure 5C). Thus, incorrect predictions can often by identified in UMPA plots as regions with low probability (by visualising its logit transform). It can be further shown that the prediction accuracy increases when the data is stratified by prediction probability and accuracy computed separately for increasing probability subsets. For example, a cutoff on probability for optimal prediction performance can be identified by computing the prediction accuracy when including progressively lower probability predictions and identifying a level of prediction probability at which the accuracy falls.

Thus, the present work provides an atlas for tumour microenvironment cell type prediction. Additionally, we examined precision and recall of cell type prediction for each study, respectively. Precision measures the fraction of true positives over true positives and false positives, and therewith the ability to correctly distinguish cells, whereas recall is computed as true positives over all positives, and therefore measures how many relevant cells are retrieved. Thus, high precision implies that the reported items are mostly true positives, but fails to account for how many positives were not reported at all. High recall on the other hand implies most relevant items are identified, but this could be trivially achieved by identifying all items without any precision. Hence, combining these two metrics yields a solid evaluation of a predictor. It is to be stressed that not all cell types appear in all studies, but due to training on all except one study, virtually all cell types can be predicted. Nonetheless, the pan-cancer atlas achieved high precision/ recall means across studies, for example 0.96/ 0.90 for B cells, Dendritic 0.76/ 0.82 for dendritic cells, and 0.65/ 0.67 for Endothelial cells.

The present atlas was further validated using publicly available cancer TME CITE-Seq data. CITE-seq data includes both RNA and protein information, allowing for higher confidence cell type annotation. The data was projected onto the atlas to get cell type predictions. These predictions were then compared with the annotations from the authors of the original CITE-Seq data, indicating a good level of matching. The scANVI normalised signals were also compared to the CITE-Seq protein signals, and there were seen to be better correlated than the traditional log1p transformed expression values. Thus, this validates the approach both in terms of accuracy of the annotation and used for data normalisation.

### Discussion

In this study the inventors jointly integrated 13 scRNA-Seq data sets of different cancer indications. An initial trVAE integration of full TME data with subsequent tumour score computation enabled the precise identification of malignant cells. Subsequently, malignant cells were temporarily removed such that the remaining immune and stromal cells could be reliably integrated with CD45+ presorted studies in a second trVAE. Batch effect removal and aggregation additionally allowed for identification of rare cell types and detailed, overall annotation of cell types and states. Consequently, cell type gene signatures with cross-study validity were derived which can aid in future, (semi-)manual annotation workflows. In addition, scANVI models were built and validated on all involved data sets, respectively, leveraging trVAE-derived signatures and resulting in a scRNA-Seq TME atlas, relevant for immuno-oncology. The developed atlas allows for batch effect removal and fully automated cell type annotation of the full TME in novel query studies via projection onto the existing reference. Moreover, this workflow can be expanded to future studies and - independently - the provided atlas can be iteratively extended by the community via architecture surgery. Furthermore, the scANVI model can be deployed for expression value normalization.

Having obtained batch effect free cell type annotation, the inventors show how the integrated data can be further explored to reveal pan cancers similarities and differences. Despite generally high cancer heterogeneity, it could be shown that the TME composition can be driven by cancer indication, however several TMEs of different indications could show higher similarity to each other than to various TMEs of the same indication. Even though the clonal evolution in cancer has irrefutably unique characteristics, specific genes and pathways were substantially impacted in the pan-cancer analysis, highlighting indication independent evolutionary strategies, such as immune evasion by MHC downregulation and active immune suppression via regulatory T cell recruitment.

Even though, all except one study in these analyses were sequenced using the 10x Genomics protocol, thereby raising the question of atlas performance on data from other protocols, prediction performance on study 58 (Singleron sequencing) was in line with other studies. Moreover, robust integration across different sequencing technologies has been previously demonstrated (Lotfollahi et al., 2022). scArches requires the exact same genes to be present in the atlas and in the query data, which can be challenging given that oftentimes the future query data is not known at the moment of atlas generation and only incorporated later via architecture surgery. To overcome this limitation, the inventors concatenated different studies by forming the union of detected genes, imputing missing genes with 0. After atlas generation based on the union of highly variable genes and custom marker genes, the methods stored a single cell as reference, which is later used to map the query studies to the exact same gene space.

Another drawback is the restriction of scArches scANVI to raw count data. That is, frequently publicly available data sets merely contain the normalized expression matrix, rather than raw counts or fastq files, which cannot be used in the model. Given that this is mainly due to patient data protection regulations, the feasibility of decentralized model fine-tuning and solely sharing of the trained weights poses an alternative to circumvent such issues. Moreover, training on unbalanced data might bias the prediction towards more prevalent cell types. However, this can be mitigated by restricting the training input to the same number of cells of each cell type per training epoch. Additionally, synthetic oversampling approaches could be used to increase the count and therewith detection of low frequency cell types (Bej et al., 2021). It is further to be noted that hematological malignancies were excluded in this work to ensure unambiguous labeling of tumour and immune cells. Based on the foundation laid here, future work could build upon our signatures and models and further fine tune the above to also account for hematological cancer data. This could be performed by projecting novel studies onto the atlas by architecture surgery (Lotfollahi et al., 2022). Finally, the presented atlas is not a flawless cell type predictor and incorrect predictions occur. However, most predictions were either correct or close to the true cell type. Prediction accuracy can be further augmented by discarding low probability cells, if desired.

### References

Lotfollahi, M. et al. Mapping single-cell data to reference atlases by transfer learning. Nat. Biotechnol. 40, 121-130 (2022).
Lotfollahi, M., Naghipourfar, M., Theis, F. J. & Wolf, F. A. Conditional out-of-distribution generation for unpaired data using transfer VAE. Bioinformatics 36, i610-i617 (2020).
Xu, C. et al. Probabilistic harmonization and annotation of single-cell transcriptomics data with deep generative models. Mol. Syst. Biol. 17, e9620 (2021).
Duan, Q., Zhang, H., Zheng, J. & Zhang, L. Turning Cold into Hot: Firing up the Tumour Microenvironment. Trends Cancer Res. 6, 605-618 (2020).
Osta, W. A. et al. EpCAM is overexpressed in breast cancer and is a potential target for breast cancer gene therapy. Cancer Res. 64, 5818-5824 (2004).
Wenqi, D. et al. EpCAM is overexpressed in gastric cancer and its downregulation suppresses proliferation of gastric cancer. J. Cancer Res. Clin. Oncol. 135, 1277-1285 (2009).
Massoner, P. et al. EpCAM is overexpressed in local and metastatic prostate cancer, suppressed by chemotherapy and modulated by MET-associated miRNA-200c/205. Br. J. Cancer 111, 955-964 (2014).
van der Gun, B. T. F. et al. Transcription factors and molecular epigenetic marks underlying EpCAM overexpression in ovarian cancer. Br. J. Cancer 105, 312-319 (2011).
Lin, S. Y. et al. Prospective Molecular Profiling of Circulating Tumour Cells from Patients with Melanoma Receiving Combinatorial Immunotherapy. Clin. Chem. 66, 169-177 (2020).
Fang, J. et al. High KRT8 expression promotes tumour progression and metastasis of gastric cancer. Cancer Sci. 108, 178-186 (2017).
Ruffin, A. T. et al. B cell signatures and tertiary lymphoid structures contribute to outcome in head and neck squamous cell carcinoma. Nat. Commun. 12, 3349 (2021).
Li, C. et al. 6-Phosphogluconolactonase Promotes Hepatocellular Carcinogenesis by Activating Pentose Phosphate Pathway. Front Cell Dev Biol 9, 753196 (2021).
Lee, H.-O. et al. Lineage-dependent gene expression programs influence the immune landscape of colorectal cancer. Nat. Genet. 52, 594-603 (2020).
Laughney, A. M. et al. Regenerative lineages and immune-mediated pruning in lung cancer metastasis. Nat. Med. 26, 259-269 (2020).
Kim, E. Y. et al. Early lung carcinogenesis and tumour microenvironment observed by single-cell transcriptome analysis. Transl. Oncol. 15, 101277 (2022).
Wu, F. et al. Single-cell profiling of tumour heterogeneity and the microenvironment in advanced non-small cell lung cancer. Nat. Commun. 12, 2540 (2021).
Borcherding, N. et al. Mapping the immune environment in clear cell renal carcinoma by single-cell genomics. Commun Biol 4, 122 (2021).
Chen, S. et al. Single-cell analysis reveals transcriptomic remodellings in distinct cell types that contribute to human prostate cancer progression. Nat. Cell Biol. 23, 87-98 (2021).
Boufea, K. et al. Single-cell RNA sequencing of human breast tumour-infiltrating immune cells reveals a γδ T-cell subtype associated with good clinical outcome. Life Sci Alliance 4, (2021).
Chen, Z. et al. Single-cell RNA sequencing highlights the role of inflammatory cancer-associated fibroblasts in bladder urothelial carcinoma. Nat. Commun. 11, 5077 (2020).
Zhang, X. et al. Dissecting esophageal squamous-cell carcinoma ecosystem by single-cell transcriptomic analysis. Nat. Commun. 12, 5291 (2021).
Durante, M. A. et al. Single-cell analysis reveals new evolutionary complexity in uveal melanoma. Nat. Commun. 11, 496 (2020).
Ji, A. L. et al. Multimodal Analysis of Composition and Spatial Architecture in Human Squamous Cell Carcinoma. Cell 182, 1661-1662 (2020).
Leland McInnes and John Healy and James Melville. UMAP: Uniform Manifold Approximation and Projection for Dimension Reduction.
Preston, C. C. et al. The ratios of CD8+ T cells to CD4+CD25+ FOXP3+ and FOXP3- T cells correlate with poor clinical outcome in human serous ovarian cancer. PLoS One 8, e80063 (2013).
Liang, W. G. et al. Structural basis for oligomerization and glycosaminoglycan binding of CCL5 and CCL3. Proc. Natl. Acad. Sci. U. S. A. 113, 5000-5005 (2016).
Almatroodi, S. A., McDonald, C. F. & Pouniotis, D. S. Alveolar Macrophage Polarisation in Lung Cancer. Lung Cancer Int 2014, 721087 (2014).
Bej, S., Galow, A.-M., David, R., Wolfien, M. & Wolkenhauer, O. Automated annotation of rare-cell types from single-cell RNA-sequencing data through synthetic oversampling. BMC Bioinformatics 22, 557 (2021).
Vanderbeke et al. Nature Communications volume 12, Article number: 4117 (2021)
MacParland et al. Nature Communications volume 9, Article number: 4383 (2018)
Arora & Pal. Front Oncol. 2021; 11: 596798.
Andrews et al. bioRxivMArch 28, 2021, https://doi.org/10.1101/2021.03.27.436882. Now published in Hepatology Communications doi: 10.1002/hep4.1854
Wang et al. Scientific Reports volume 11, Article number: 19396 (2021)
Merad, Ginhoux & Collin, Nature Reviews Immunology volume 8, pages935-947 (2008)
Wu et al., Nature Communications volume 12, Article number: 2540 (2021)
Poli et al.,bioRxiv Jan 5, 2021. https://doi.org/10.1101/2021.01.04.425268
Cheng et al., Cell Volume 184, Issue 3, 4 February 2021, Pages 792-809.e23
Wang et al., Nature Communications volume 11, Article number: 4239 (2020)
Guslund et al. Front. Immunol., 09 October 2020 https://doi.org/10.3389/fimmu.2020.559555 Rubenstein et al. Scientific Reports volume 10, Article number: 229 (2020)
Nieto et al. Genome Res. 2021 Oct;31(10):1913-1926.

The specific embodiments described herein are offered by way of example, not by way of limitation. Any sub-titles herein are included for convenience only, and are not to be construed as limiting the disclosure in any way.

## Claims

1. A computer-implemented method of analysing a tumour sample comprising tumour cells and immune cells, the method comprising:
(a) obtaining single cell gene expression profiles for a plurality of cells from the tumour sample, the single cell gene expression profiles comprising gene expression measurements for a set of genes;
(b) using a deep learning model to identify a respective latent variable representation of the single cell gene expression profiles in the sample; and
(c) identifying a respective one of one or more latent space clusters of cells that the cells in the sample belong to, wherein the clusters of cells correspond to cells from different cell types and wherein the one or more clusters of cells comprise at least a cluster corresponding to tumour cells and one or more clusters of cells corresponding to different cell types in the tumour microenvironment,
wherein the deep learning model has been obtained by:
obtaining cell type labels associated with one or more clusters of cells in the latent space of a first deep learning model that has been trained to identify a latent variable representation of single cell gene expression profiles from cells in tumour samples that have not been purified to select tumour microenvironment cells, wherein the one or more clusters of cells comprise at least a cluster corresponding to tumour cells,
obtaining cell type labels associated with one or more clusters of cells in the latent space of a second deep learning model that has been trained to identify a latent variable representation of single cell gene expression profiles from cells identified as non-malignant in tumour samples that have not been purified to select tumour microenvironment cells and/or cells from samples comprising purified tumour microenvironment cells, wherein the one or more clusters of cells correspond to different cell types in the tumour microenvironment, and
training a third deep learning model to identify a latent variable representation of single cell gene expression profiles using the cell type labels associated with the one or more clusters of cells in the latent space of the first deep learning model and the one or more clusters of cells in the latent space of the second deep learning model.

2. The method of claim 1, wherein the method comprises:
training the first deep learning model using data comprising the single cell gene expression profiles for the plurality of cells from the tumour sample and single cell gene expression profiles for a plurality of cells from a plurality of tumour samples that have not been purified to select tumour microenvironment cells, and/or
training the second deep learning model using data comprising the single cell gene expression profiles for the plurality of cells from the tumour sample and single cell gene expression profiles for a plurality of cells that have been identified as non-malignant in a plurality of tumour samples that have not been purified to select tumour microenvironment cells and/or for a plurality of cells from samples comprising purified tumour microenvironment cells, and/or
training the third deep learning model using data comprising the single cell gene expression profiles for the plurality of cells from the tumour sample and single cell gene expression profiles for a plurality of cells associated with cell type labels associated with clusters of cells in the latent space of the first and/or second deep learning models.

3. The method of claim 1 or claim 2, wherein the method comprises:
training the first deep learning model using data comprising the single cell gene expression profiles for the plurality of cells from the tumour sample and parameters from a deep learning model that has been trained to identify a latent variable representation from single cell gene expression profiles for a plurality of cells from a plurality of tumour samples that have not been purified to select tumour microenvironment cells, and/or
training the second deep learning model using data comprising the single cell gene expression profiles for the plurality of cells from the tumour sample and parameters from a deep learning model that has been trained to identify a latent variable representation from single cell gene expression profiles for a plurality of cells that have been identified as non-malignant in a plurality of tumour samples and/or for a plurality of cells from samples comprising purified tumour microenvironment cells, and/or
training the third deep learning model using data comprising the single cell gene expression profiles for the plurality of cells from the tumour sample and parameters from a deep learning model that has been trained to identify a latent variable representation from single cell gene expression profiles for a plurality of cells associated with cell type labels associated with clusters of cells in the latent space of the first and/or second deep learning models,
optionally wherein the deep learning model is a deep neural network comprising a set of nodes and weights between nodes, the parameters comprise the weights between the nodes, and training uses transfer learning of a model extending the deep learning models for which weights are provided by including one or more additional input nodes with trainable weights.

4. The method of any preceding claim, wherein the single cell gene expression profiles used to train the first and/or second and/or third deep learning models are from a plurality of samples at least some of which are from different types of cancers, and/or
wherein the single cell gene expression profiles used to train the first and/or second and/or third deep learning models are from a plurality of samples that do not include haematological malignancies.

5. The method of any preceding claim, wherein the deep learning model is a variational autoencoder or a generative adversarial net, wherein the first and/or second deep learning models are unsupervised models and the third deep learning model is a semi-supervised deep learning model, optionally wherein the deep learning model is a conditional variational autoencoder (CVAE), a conditional generative adversarial net (cGAN), a transfer variational autoencoder (trVAE), a single cell variational inference (scVI) model, or a single cell annotation using variational inference (scANVI) model.

6. The method of any preceding claims, wherein the latent space clusters are graph based clusters, optionally wherein the clusters are obtained using the Leiden algorithm, and/or
wherein a cell type refers to a set of cells with a common morphology, physiology and/or function, and/or
wherein a cell type refers to any cell type selected from: malignant cells, non-malignant cells, immune cells, stromal cells, cytotoxic cells, proliferative cells, pro-inflammatory cells, T cells, CD4+ T cells, CD8+ T cells, gamma delta T cells, gamma delta 2 T cells, activated T cells, cd4+ follicular helper T cells, exhausted T cells, exhausted CD4+ T cells, exhausted CD8+ T cells, exhausted regulatory T cells, regulatory T cells, Th17 cells, naïve T cells, naïve CD4+ T cells, naïve CD8+ T cells, proliferative T cells, proliferative CD4+ T cells, proliferative CD8+ T cells, proliferative CD4+ T cells, proliferative CD8+ T cells, recently activated CD4+ T cells, naïve memory CD4+ T cells, terminally exhausted CD8+ T cells, effector memory CD8+ T cells, transitional memory CD4+ T cells, pre-exhausted CD8+ T cells, fibroblasts, B cells, naïve B cells, memory B cells, proliferative B cells, plasma cells, endothelial cells, lymphatic endothelial cells, liver sinusoidal endothelial cells, dendritic cells, plasmacytoid dendritic cells (pDC), cDC1 dendritic cells, dendritic cells expressing CLEC9A, cDC2 dendritic cells, dendritic cells expressing CD1C, cDC3 dendritic cells, dendritic cells expressing LAMP3, myeloid dendritic cells, langerin dendritic cells, follicular dendritic cells, mast cells, natural killer (NK) cells, monocytes, macrophages, tumour associated macrophages (TAM), SPP1 TAMs, M2 TAMs, alveolar macrophages, monocytes, CD14+ monocytes, CD16+ monocytes, erythrocytes, pericytes, keratinocytes, melanocytes, neuronal cells, smooth muscle cells.

7. The method of any preceding claim, wherein the cells identified as non-malignant in tumour samples that have not been purified to select tumour microenvironment cells have been identified based on the latent variable representation from the first deep learning model, and/or
wherein the method further comprises identifying cells as non-malignant in the tumour samples that have not been purified to select tumour microenvironment cells based on the latent representation from the first deep learning model, and/or
wherein the method further comprises identifying one or more cells in the tumour sample as non-malignant cells based on the latent variable representation from the first deep learning model.

8. The method of claim 7, wherein identifying one or more cells in a tumour sample as non-malignant cells based on the latent variable representation from the first deep learning model comprises classifying one or more cells in the tumour sample between a first class corresponding to malignant cells and a second class corresponding to non-malignant cells by assigning cells to one of a plurality of clusters in the latent space of the first deep learning model, each cluster being associated with a malignant state or non-malignant state.

9. The method of claim 8, wherein each cluster is associated with a malignant state or non-malignant state based on a tumour score obtained from expression of a plurality of genes associated with cancer cells and a plurality of genes associated with immune or stromal cells,
optionally wherein the plurality of genes associated with cancer cells are genes overexpressed in cancer and/or wherein the plurality of genes associated with cancer cells comprise one or more of: EPCAM, MLANA and KRT8, and/or
optionally wherein the plurality of genes associated with immune or stromal cells are markers of immune cells, and/or one or more types of stromal cells selected from collagen-producing cells, fibroblasts, pericyte, and/or endothelial origin, and/or wherein the plurality of genes associated with immune or stromal cells comprise one or more of: a marker of immune cells such as PTPRC, markers of collagen producing cells selected from COL1A1, COL1A2, COL5A1 and LUM, a marker of fibroblasts such as FBLN1, markers of pericyte selected from RGS5, CNN1, MYH11, SMTN, ACTA2, TAGLN and CALD1, and markers of endothelial origin selected from VWF and PVLAP.

10. The method of claim 9, wherein the tumour score is obtained by:
computing a single cell tumour score from expression of a plurality of genes associated with cancer cells and a plurality of genes associated with immune or stromal cells,
obtaining a cluster tumour score as a summarised value of the single cell tumour scores for each cluster,
identifying each cluster as malignant or non-malignant based on the cluster tumour score,
obtaining a summarised latent space coordinate for the clusters identified as malignant and a summarised latent space coordinate for the clusters identified as non-malignant, and
associating a cluster with a malignant state or non-malignant state based on a distance between the cluster and the summarised latent space coordinate for the clusters identified as malignant or non-malignant.

11. The method of claim 10, wherein:
the single cell tumour score is obtained by computing, for each cell, the difference between a summarised expression value for the plurality of genes associated with cancer cells and a summarised expression value for the plurality of genes associated with immune or stromal cells, optionally wherein the summarised expression value is the mean or the maximum mean for one of a plurality of subsets of genes, such as subsets of genes that are markers of immune cells or one or more types of stromal cells, and/or
the cluster tumour score is the average of the single cell tumour scores for all the cells assigned to a cluster, and/or
the summarised latent space coordinate for the clusters identified as malignant / non-malignant is the average latent space coordinate vector across clusters identified as malignant / non-malignant, and/or
identifying each cluster as malignant or non-malignant based on the cluster tumour score comprises comparing the cluster tumour score to a threshold identified using the distribution of the single cell tumour scores, optionally wherein the threshold is identified as the positive local minimum of a kernel density estimate of the distribution of single cell tumour scores,
the distance is a Euclidian distance, and/or
associating a cluster with a malignant state or non-malignant state based on a distance between the cluster and the summarised latent space coordinate for the clusters identified as malignant or non-malignant comprises computing the distance between: (i) the average latent space coordinate for the cluster and the summarised latent space coordinate for the clusters identified as malignant and (ii) the average latent space coordinate for the cluster and the summarised latent space coordinate for the clusters identified as non-malignant, and associating the cluster with a malignant state if the distance in (i) is smaller than the distance in (ii).

12. The method of any preceding claim, wherein the method comprises identifying cells as non-malignant in the tumour samples that have not been purified to select tumour microenvironment cells based on the latent representation from the first deep learning model, and identifying remaining malignant cells as cells with high or low tumour potential using a classifier trained to distinguish between normal and non normal cells based on one or more metrics derived from a RNAseq copy number variation analysis, optionally wherein the metrics derived from a RNA seq copy number variation analysis are selected from: a single cell CNV score, a single cell percentile CNV score, or a cluster donor entropy score for clusters obtained in single cell CNV score space.

13. The method of any preceding claim, wherein the cell type labels have been obtained by:
training a first deep learning model to identify a latent variable representation of single cell gene expression profiles from cells in tumour samples that have not been purified to select tumour microenvironment cells,
identifying non-malignant cells and malignant cells based on the latent variable representation from the first deep learning model, optionally using the method of any of claims 8 to 11,
associating a cell type label to any cell identified as a malignant cell,
training a second deep learning model to identify a latent variable representation of single cell gene expression profiles from cells identified as non-malignant cells based on the latent variable representation from the first deep learning model and/or cells from samples comprising purified tumour microenvironment cells,
clustering the latent space representation of single cell gene expression profiles from the second deep learning model, and
associating a cell type label to one or more of the clusters, optionally wherein associating a cell type label is performed based on the level and/or frequency of expression of one or more markers for each cell type label in the cells of a cluster, and/or wherein associating a cell type label to one or more of the clusters comprises re-clustering the one or more clusters to identify further clusters such that the expression of one or more markers is more homogeneous within the further clusters than in the original cluster(s).

14. The method of any preceding claim, further comprising:
(a) classifying the tumour sample between a plurality of classes associated with different tumour burdens, wherein the tumour burden refers to the proportion of cells that are malignant cells vs non-malignant cells in the tumour sample, based on the proportion of cells in the tumour sample assigned to one or more latent space clusters from the first deep learning model corresponding to tumour cells and optionally the proportion of cells in the tumour samples assigned to one or more latent space clusters from the second deep learning model, or based on the proportion of cells in the tumour sample assigned to a latent space cluster from the third deep learning model corresponding to tumour cells, optionally wherein the plurality of classes comprise a class with a higher tumour burden than all other classes, and a class with a lower tumour burden than all other classes, and/or wherein the plurality of classes comprise a class with a high tumour burden and a class with a low tumour burden, and/or wherein the plurality of classes comprise a class with a high tumour burden, a class with an intermediate tumour burden and a class with a low tumour burden,
optionally wherein the plurality of classes were defined by clustering cell type profiles for a plurality of samples, each cell type profile comprising the proportion of cells assigned to one or more latent space clusters from the first deep learning model corresponding to tumour cells and optionally the proportion of cells assigned to one or more latent space clusters from the second deep learning model, wherein one or more of the clusters correspond to the plurality of classes, and classifying the tumour sample comprises clustering a cell type profile for the tumour sample together with cell type profiles for the plurality of samples, or selecting the class associated with the cluster that is closest to the cell type profile for the tumour sample; and/or
(b) identifying the cell type composition of the tumour sample by associating cell type labels with one or more cells in the tumour sample using the third deep learning model, optionally wherein associating cell type labels comprises obtaining a cell type label and prediction confidence for each latent space cluster or cell using the third deep learning model and associating a cell type label to any cell for which the prediction confidence is above a predetermined threshold or to any cell that belongs to a cluster for which the prediction confidence is above a predetermined threshold; and/or
(c) comparing the gene expression values of one or more genes in one or more latent space clusters of the first, second and/or third deep learning model; and/or
(d) using the first, second and/or third deep learning models to obtain batch-corrected single cell gene expression profiles for the sample; and/or
(e) identifying a gene as a biomarker of treatment response, a biomarker of prognosis or a therapeutic target based on the gene expression values of the gene in one or more latent space clusters of the first, second and/or third deep learning model; optionally wherein identifying a gene as a biomarker of treatment response or prognosis comprises correlating expression of the gene in one or more latent space clusters with a metric of treatment response or prognosis; and/or
(f) identifying a therapy for the subject from which the tumour sample has been obtained based on the cell type composition in (b), the expression of one or more genes identified as a biomarker of treatment response in (e) and/or the tumour burden classification in (a); and/or
(g) selecting a subject from which the tumour sample has been obtained for participation in a clinical trial based on the cell type composition in (b), the expression of one or more genes identified as a biomarker of treatment response in (e) and/or the tumour burden classification in (a); and/or
(h) providing a prognosis for the subject from which the tumour sample has been obtained based on the cell type composition in (b), the expression of one or more genes identified as a biomarker of treatment response in (e) and/or the tumour burden classification in (a).

15. A system comprising:
at least one processor; and
at least one non-transitory computer readable medium containing instructions that, when executed by the at least one processor, cause the at least one processor to perform the method of any of claims 1 to 14.

## Patentansprüche

1. Computerimplementiertes Verfahren zum Analysieren einer Tumorprobe, umfassend Tumorzellen und Immunzellen, wobei das Verfahren Folgendes umfasst:
(a) Erhalten von Einzelzell-Genexpressionsprofilen für eine Vielzahl von Zellen aus der Tumorprobe, wobei die Einzelzell-Genexpressionsprofile Genexpressionsmessungen für einen Satz von Genen umfassen;
(b) Verwenden eines Deep-Learning-Modells, um eine jeweilige latente Variablendarstellung der Einzelzell-Genexpressionsprofile in der Probe zu identifizieren; und
(c) Identifizieren eines jeweiligen von einem oder mehreren Latentraum-Zellclustem, zu denen die Zellen in der Probe gehören, wobei die Zellcluster Zellen aus verschiedenen Zelltypen entsprechen und wobei der eine oder die mehreren Zellcluster mindestens einen Cluster, der Tumorzellen entspricht, und einen oder mehrere Zellcluster, die verschiedenen Zelltypen in der Tumormikroumgebung entsprechen, umfassen,
wobei das Deep-Learning-Modell durch Folgendes erhalten wurde:
Erhalten von Zelltyp-Labels, die mit einem oder mehreren Zellclustern in dem Latentraum eines ersten Deep-Learning-Modells assoziiert sind, das trainiert wurde, um eine latente Variablendarstellung von Einzelzell-Genexpressionsprofilen aus Zellen in Tumorproben zu identifizieren, die nicht aufgereinigt wurden, um Tumormikroumgebungszellen auszuwählen, wobei der eine oder die mehreren Zellcluster mindestens einen Cluster, der Tumorzellen entspricht, umfassen,
Erhalten von Zelltyp-Labels, die mit einem oder mehreren Zellclustern in dem Latentraum eines zweiten Deep-Learning-Modells assoziiert sind, das trainiert wurde, um eine latente Variablendarstellung von Einzelzell-Genexpressionsprofilen aus Zellen, die in Tumorproben, die nicht aufgereinigt wurden, um Tumormikroumgebungszellen auszuwählen, als nicht-maligne identifiziert wurden, und/oder Zellen aus Proben, die aufgereinigte Tumormikroumgebungszellen umfassen, zu identifizieren, wobei der eine oder die mehreren Zellcluster verschiedenen Zelltypen in der Tumormikroumgebung entsprechen, und
Trainieren eines dritten Deep-Learning-Modells, um eine latente Variablendarstellung von Einzelzell-Genexpressionsprofilen unter Verwendung der Zelltyp-Labels, die mit dem einen oder den mehreren Zellclustern in dem Latentraum des ersten Deep-Learning-Modells und dem einen oder den mehreren Zellclustern in dem Latentraum des zweiten Deep-Learning-Modells assoziiert sind, zu identifizieren.

2. Verfahren nach Anspruch 1, wobei das Verfahren Folgendes umfasst:
Trainieren des ersten Deep-Learning-Modells unter Verwendung von Daten, umfassend die Einzelzell-Genexpressionsprofile für die Vielzahl von Zellen aus der Tumorprobe und Einzelzell-Genexpressionsprofile für eine Vielzahl von Zellen aus einer Vielzahl von Tumorproben, die nicht aufgereinigt wurden, um Tumormikroumgebungszellen auszuwählen, und/oder
Trainieren des zweiten Deep-Learning-Modells unter Verwendung von Daten, umfassend die Einzelzell-Genexpressionsprofile für die Vielzahl von Zellen aus der Tumorprobe und Einzelzell-Genexpressionsprofile für eine Vielzahl von Zellen, die in einer Vielzahl von Tumorproben, die nicht aufgereinigt wurden, um Tumormikroumgebungszellen auszuwählen, als nicht-maligne identifiziert wurden, und/oder für eine Vielzahl von Zellen aus Proben, die aufgereinigte Tumormikroumgebungszellen umfassen, und/oder
Trainieren des dritten Deep-Learning-Modells unter Verwendung von Daten, umfassend die Einzelzell-Genexpressionsprofile für die Vielzahl von Zellen aus der Tumorprobe und Einzelzell-Genexpressionsprofile für eine Vielzahl von Zellen, die mit Zelltyp-Labels assoziiert sind, die mit Zellclustern in dem Latentraum des ersten und/oder zweiten Deep-Learning-Modells assoziiert sind.

3. Verfahren nach Anspruch 1 oder Anspruch 2, wobei das Verfahren Folgendes umfasst:
Trainieren des ersten Deep-Learning-Modells unter Verwendung von Daten, umfassend die Einzelzell-Genexpressionsprofile für die Vielzahl von Zellen aus der Tumorprobe und Parameter aus einem Deep-Learning-Modell, das trainiert wurde, um eine latente Variablendarstellung von Einzelzell-Genexpressionsprofilen für eine Vielzahl von Zellen aus einer Vielzahl von Tumorproben zu identifizieren, die nicht aufgereinigt wurden, um Tumormikroumgebungszellen auszuwählen, und/oder
Trainieren des zweiten Deep-Learning-Modells unter Verwendung von Daten, umfassend die Einzelzell-Genexpressionsprofile für die Vielzahl von Zellen aus der Tumorprobe und Parameter aus einem Deep-Learning-Modell, das trainiert wurde, um eine latente Variablendarstellung von Einzelzell-Genexpressionsprofilen für eine Vielzahl von Zellen, die in einer Vielzahl von Tumorproben als nicht-maligne identifiziert wurden, und/oder für eine Vielzahl von Zellen aus Proben, die aufgereinigte Tumormikroumgebungszellen umfassen, zu identifizieren, und/oder
Trainieren des dritten Deep-Learning-Modells unter Verwendung von Daten, umfassend die Einzelzell-Genexpressionsprofile für die Vielzahl von Zellen aus der Tumorprobe und Parameter aus einem Deep-Learning-Modell, das trainiert wurde, um eine latente Variablendarstellung von Einzelzell-Genexpressionsprofilen für eine Vielzahl von Zellen, die mit Zelltyp-Labels assoziiert sind, die mit Zellclustern in dem Latentraum des ersten und/oder zweiten Deep-Learning-Modells assoziiert sind, zu identifizieren,
wobei gegebenenfalls das Deep-Learning-Modell ein tiefes neuronales Netzwerk ist, das einen Satz von Knoten und Gewichten zwischen Knoten umfasst, die Parameter die Gewichte zwischen den Knoten umfassen und das Trainieren Transferlernen eines Modells verwendet, das die Deep-Learning-Modelle erweitert, für die Gewichte bereitgestellt werden, indem ein oder mehrere zusätzliche Eingangsknoten mit trainierbaren Gewichten eingeschlossen werden.

4. Verfahren nach einem der vorhergehenden Ansprüche, wobei die Einzelzell-Genexpressionsprofile, die zum Trainieren des ersten und/oder zweiten und/oder dritten Deep-Learning-Modells verwendet werden, aus einer Vielzahl von Proben stammen, von denen mindestens einige von verschiedenen Krebsarten stammen, und/oder wobei die Einzelzell-Genexpressionsprofile, die zum Trainieren des ersten und/oder zweiten und/oder dritten Deep-Learning-Modells verwendet werden, aus einer Vielzahl von Proben stammen, die keine hämatologischen Malignitäten einschließen.

5. Verfahren nach einem der vorhergehenden Ansprüche, wobei das Deep-Learning-Modell ein Variations-Autoencoder oder ein generatives Adversarial-Netz ist, wobei das erste und/oder zweite Deep-Learning-Modell unüberwachte Modelle sind und das dritte Deep-Learning-Modell ein halbüberwachtes Deep-Learning-Modell ist, wobei gegebenenfalls das Deep-Learning-Modell ein konditionaler Variations-Autoencoder (CVAE), ein konditionales generatives Adversarial-Netz (cGAN), ein Transfervariations-Autoencoder (trVAE), ein Einzelzell-Variationsinferenz-(scVI)-Modell oder eine Einzelzell-Annotation unter Verwendung eines Variationsinferenz-(scANVI)-Modells ist.

6. Verfahren nach einem der vorhergehenden Ansprüche, wobei die Latentraum-Cluster graphenbasierte Cluster sind, wobei die Cluster gegebenenfalls unter Verwendung des Leiden-Algorithmus erhalten werden, und/oder
wobei sich ein Zelltyp auf einen Satz von Zellen mit einer gemeinsamen Morphologie, Physiologie und/oder Funktion bezieht, und/oder
wobei sich ein Zelltyp auf einen beliebigen Zelltyp bezieht, ausgewählt aus: malignen Zellen, nicht-malignen Zellen, Immunzellen, Stromazellen, zytotoxischen Zellen, proliferativen Zellen, proinflammatorischen Zellen, T-Zellen, CD4+-T-Zellen, CD8+-T-Zellen, Gamma-Delta-T-Zellen, Gamma-Delta-2-T-Zellen, aktivierten T-Zellen, follikulären CD4+-Helfer-T-Zellen, erschöpften T-Zellen, erschöpften CD4+-T-Zellen, erschöpften CD8+-T-Zellen, erschöpften regulatorischen T-Zellen, regulatorischen T-Zellen, Th17-Zellen, naiven T-Zellen, naiven CD4+-T-Zellen, naiven CD8+-T-Zellen, proliferativen T-Zellen, proliferativen CD4+-T-Zellen, proliferativen CD8+-T-Zellen, proliferativen CD4+-T-Zellen, proliferativen CD8+-T-Zellen, kürzlich aktivierten CD4+-T-Zellen, naiven Gedächtnis-CD4+-T-Zellen, terminal erschöpften CD8+-T-Zellen, Effektor-Gedächtnis-CD8+-T-Zellen, Übergangsgedächtnis-CD4+-T-Zellen, vorerschöpften CD8+-T-Zellen, Fibroblasten, B-Zellen, naiven B-Zellen, Gedächtnis-B-Zellen, proliferativen B-Zellen, Plasmazellen, Endothelzellen, lymphatischen Endothelzellen, Lebersinusoidalendothelzellen, dendritischen Zellen, plasmazytoiden dendritischen Zellen (pDC), dendritischen cDC1-Zellen, dendritischen Zellen, die CLEC9A exprimieren, dendritischen cDC2-Zellen, dendritischen Zellen, die CD1C exprimieren, dendritischen cDC3-Zellen, dendritischen Zellen, die LAMP3 exprimieren, myeloischen dendritischen Zellen, dendritischen Langerhans-Zellen, follikulären dendritischen Zellen, Mastzellen, natürlichen Killer-(NK)-Zellen, Monozyten, Makrophagen, tumorassoziierten Makrophagen (TAM), SPP1-TAMs, M2-TAMs, alveolären Makrophagen, Monozyten, CD14+-Monozyten, CD16+-Monozyten, Erythrozyten, Perizyten, Keratinozyten, Melanozyten, neuronalen Zellen, glatten Muskelzellen.

7. Verfahren nach einem der vorhergehenden Ansprüche, wobei die Zellen, die in Tumorproben, die nicht aufgereinigt wurden, um Tumormikroumgebungszellen auszuwählen, als nicht-maligne identifiziert wurden, basierend auf der latenten Variablendarstellung aus dem ersten Deep-Learning-Modell identifiziert wurden, und/oder
wobei das Verfahren ferner das Identifizieren von Zellen als nicht-maligne in den Tumorproben, die nicht aufgereinigt wurden, um Tumormikroumgebungszellen auszuwählen, basierend auf der latenten Darstellung aus dem ersten Deep-Learning-Modell umfasst, und/oder
wobei das Verfahren ferner das Identifizieren einer oder mehrerer Zellen in der Tumorprobe als nicht-maligne Zellen basierend auf der latenten Variablendarstellung aus dem ersten Deep-Learning-Modell umfasst.

8. Verfahren nach Anspruch 7, wobei das Identifizieren einer oder mehrerer Zellen in einer Tumorprobe als nicht-maligne Zellen basierend auf der latenten Variablendarstellung aus dem ersten Deep-Learning-Modell das Klassifizieren einer oder mehrerer Zellen in der Tumorprobe in eine erste Klasse, die malignen Zellen entspricht, und eine zweite Klasse, die nicht-malignen Zellen entspricht, durch Zuordnen von Zellen zu einem von einer Vielzahl von Clustern im Latentraum des ersten Deep-Learning-Modells umfasst, wobei jedes Cluster mit einem malignen Zustand oder einem nicht-malignen Zustand assoziiert ist.

9. Verfahren nach Anspruch 8, wobei jedes Cluster mit einem malignen Zustand oder einem nicht-malignen Zustand basierend auf einem Tumorscore assoziiert ist, der aus der Expression einer Vielzahl von Genen, die mit Krebszellen assoziiert sind, und einer Vielzahl von Genen, die mit Immun- oder Stromazellen assoziiert sind, erhalten wird,
wobei gegebenenfalls die Vielzahl von Genen, die mit Krebszellen assoziiert sind, Gene sind, die bei Krebs überexprimiert sind, und/oder wobei die Vielzahl von Genen, die mit Krebszellen assoziiert sind, eines oder mehrere von Folgendem umfassen: EPCAM, MLANA und KRT8, und/oder
wobei gegebenenfalls die Vielzahl von Genen, die mit Immun- oder Stromazellen assoziiert sind, Marker von Immunzellen und/oder eine oder mehrere Arten von Stromazellen sind, die aus kollagenproduzierenden Zellen, Fibroblasten, Perizyten und/oder endothelialem Ursprung ausgewählt sind, und/oder wobei die Vielzahl von Genen, die mit Immun- oder Stromazellen assoziiert sind, eines oder mehrere von Folgendem umfassen: einen Marker von Immunzellen wie PTPRC, Marker von kollagenproduzierenden Zellen, die aus COL1A1, COL1A2, COL5A1 und LUM ausgewählt sind, einen Marker von Fibroblasten wie FBLN1, Marker von Perizyten, ausgewählt aus RGS5, CNN1, MYH11, SMTN, ACTA2, TAGLN und CALD1, und Marker endothelialen Ursprungs, ausgewählt aus VWF und PVLAP.

10. Verfahren nach Anspruch 9, wobei der Tumorscore erhalten wird durch:
Berechnen eines Einzelzell-Tumorscores aus der Expression einer Vielzahl von Genen, die mit Krebszellen assoziiert sind, und einer Vielzahl von Genen, die mit Immun- oder Stromazellen assoziiert sind,
Erhalten eines Cluster-Tumorscores als einen zusammengefassten Wert der Einzelzell-Tumorscors für jedes Cluster,
Identifizieren jedes Clusters als malignes oder nicht-malignes basierend auf dem Cluster-Tumorscore,
Erhalten einer zusammengefassten Latentraumkoordinate für die als maligne identifizierten Cluster und einer zusammengefassten Latentraumkoordinate für die als nicht-maligne identifizierten Cluster, und
Assoziieren eines Clusters mit einem malignen Zustand oder einem nicht-malignen Zustand basierend auf einem Abstand zwischen dem Cluster und der zusammengefassten Latentraumkoordinate für die als maligne oder nicht-maligne identifizierten Cluster.

11. Verfahren nach Anspruch 10, wobei:
der Einzelzell-Tumorscore durch Berechnen der Differenz zwischen einem zusammengefassten Expressionswert für die Vielzahl von Genen, die mit Krebszellen assoziiert sind, und einem zusammengefassten Expressionswert für die Vielzahl von Genen, die mit Immun- oder Stromazellen assoziiert sind, für jede Zelle erhalten wird, wobei gegebenenfalls der zusammengefasste Expressionswert der Mittelwert oder der maximale Mittelwert für eine einer Vielzahl von Teilmengen von Genen ist, wie etwa Teilmengen von Genen, die Marker von Immunzellen oder einer oder mehreren Arten von Stromazellen sind, und/oder
der Cluster-Tumorscore der Mittelwert der Einzelzell-Tumorscoree für alle Zellen ist, die einem Cluster zugeordnet sind, und/oder
die zusammengefasste Latentraumkoordinate für die als maligne/nicht-maligne identifizierten Cluster der mittlere Latentraumkoordinatenvektor über als maligne/nicht-maligne identifizierte Cluster ist, und/oder
das Identifizieren jedes Clusters als maligne oder nicht-maligne basierend auf dem Cluster-Tumorscore das Vergleichen des Cluster-Tumorscores mit einem Schwellenwert umfasst, der unter Verwendung der Verteilung der Einzelzell-Tumorscore identifiziert wird, wobei gegebenenfalls der Schwellenwert als das positive lokale Minimum einer Kerndichteschätzung der Verteilung von Einzelzell-Tumorscores identifiziert wird,
der Abstand ein euklidischer Abstand ist, und/oder
das Assoziieren eines Clusters mit einem malignen Zustand oder einem nicht-malignen Zustand basierend auf einem Abstand zwischen dem Cluster und der zusammengefassten Latentraumkoordinate für die als maligne oder nicht-maligne identifizierten Cluster das Berechnen des Abstands zwischen: (i) der mittleren Latentraumkoordinate für den Cluster und der zusammengefassten Latentraumkoordinate für die als maligne identifizierten Cluster und (ii) der mittleren Latentraumkoordinate für den Cluster und der zusammengefassten Latentraumkoordinate für die als nicht-maligne identifizierten Cluster und das Assoziieren des Clusters mit einem malignen Zustand, wenn der Abstand in (i) kleiner als der Abstand in (ii) ist, umfasst.

12. Verfahren nach einem der vorhergehenden Ansprüche, wobei das Verfahren das Identifizieren von Zellen als nicht-maligne in den Tumorproben, die nicht aufgereinigt wurden, um Tumormikroumgebungszellen auszuwählen, basierend auf der latenten Darstellung aus dem ersten Deep-Learning-Modell und das Identifizieren verbleibender maligner Zellen als Zellen mit hohem oder niedrigem Tumorpotential unter Verwendung eines Klassifikators, der trainiert ist, um zwischen normalen und nicht normalen Zellen zu unterscheiden, basierend auf einer oder mehreren Messgrößen, die von einer RNA-seq-Kopienzahlvariationsanalyse abgeleitet sind, umfasst, wobei gegebenenfalls die Messgrößen, die von einer RNA-seq-Kopienzahlvariationsanalyse abgeleitet sind, ausgewählt sind aus: einem Einzelzell-CNV-Score, einem Einzelzell-Perzentil-CNV-Score oder einem Cluster-Donor-Entropie-Score für Cluster, die im Einzelzell-CNV-Score-Raum erhalten werden.

13. Verfahren nach einem der vorhergehenden Ansprüche, wobei die Zelltyp-Labels erhalten wurden durch:
Trainieren eines ersten Deep-Learning-Modells, um eine latente Variablendarstellung von Einzelzell-Genexpressionsprofilen aus Zellen in Tumorproben zu identifizieren, die nicht aufgereinigt wurden, um Tumormikroumgebungszellen auszuwählen,
Identifizieren nicht-maligner Zellen und maligner Zellen basierend auf der latenten Variablendarstellung aus dem ersten Deep-Learning-Modell, gegebenenfalls unter Verwendung des Verfahrens nach einem der Ansprüche 8 bis 11,
Assoziieren eines Zelltyp-Labels mit einer beliebigen Zelle, die als eine maligne Zelle identifiziert wurde,
Trainieren eines zweiten Deep-Learning-Modells, um eine latente Variablendarstellung von Einzelzell-Genexpressionsprofilen aus Zellen, die als nicht-maligne Zellen identifiziert wurden, basierend auf der latenten Variablendarstellung aus dem ersten Deep-Learning-Modell und/oder Zellen aus Proben, die aufgereinigte Tumormikroumgebungszellen umfassen, zu identifizieren,
Clustern der Latentraumdarstellung von Einzelzell-Genexpressionsprofilen aus dem zweiten Deep-Learning-Modell und
Assoziieren eines Zelltyp-Labels mit einem oder mehreren der Cluster, wobei gegebenenfalls das Assoziieren eines Zelltyp-Labels basierend auf dem Niveau und/oder der Häufigkeit der Expression eines oder mehrerer Marker für jedes Zelltyp-Label in den Zellen eines Clusters durchgeführt wird, und/oder wobei das Assoziieren eines Zelltyp-Labels mit einem oder mehreren der Cluster das erneute Clustern des einen oder der mehreren Cluster umfasst, um weitere Cluster zu identifizieren, sodass die Expression eines oder mehrerer Marker innerhalb der weiteren Cluster homogener als in dem/den ursprünglichen Cluster(n) ist.

14. Verfahren nach einem der vorhergehenden Ansprüche, ferner umfassend:
(a) Klassifizieren der Tumorprobe in eine Vielzahl von Klassen, die mit verschiedenen Tumorlasten assoziiert sind, wobei sich die Tumorlast auf den Anteil von Zellen, die maligne Zellen sind, gegenüber nicht-malignen Zellen in der Tumorprobe bezieht, basierend auf dem Anteil von Zellen in der Tumorprobe, die einem oder mehreren Latentraum-Clustern aus dem ersten Deep-Learning-Modell zugeordnet sind, die Tumorzellen entsprechen, und gegebenenfalls dem Anteil von Zellen in den Tumorproben, die einem oder mehreren Latentraum-Clustern aus dem zweiten Deep-Learning-Modell zugeordnet sind, oder basierend auf dem Anteil von Zellen in der Tumorprobe, die einem Latentraum-Cluster aus dem dritten Deep-Learning-Modell zugeordnet sind und Tumorzellen entsprechen, wobei gegebenenfalls die Vielzahl von Klassen eine Klasse mit einer höheren Tumorlast als alle anderen Klassen und eine Klasse mit einer niedrigeren Tumorlast als alle anderen Klassen umfasst, und/oder wobei die Vielzahl von Klassen eine Klasse mit einer hohen Tumorlast und eine Klasse mit einer niedrigen Tumorlast umfasst, und/oder wobei die Vielzahl von Klassen eine Klasse mit einer hohen Tumorlast, eine Klasse mit einer mittleren Tumorlast und eine Klasse mit einer niedrigen Tumorlast umfasst,
wobei gegebenenfalls die Vielzahl von Klassen durch Clustern von Zelltypprofilen für eine Vielzahl von Proben definiert wurde, wobei jedes Zelltypprofil den Anteil von Zellen, die einem oder mehreren Latentraum-Clustern aus dem ersten Deep-Learning-Modell zugeordnet sind und Tumorzellen entsprechen, und gegebenenfalls den Anteil von Zellen, die einem oder mehreren Latentraum-Clustern aus dem zweiten Deep-Learning-Modell zugeordnet sind, umfasst, wobei einer oder mehrere der Cluster der Vielzahl von Klassen entsprechen und das Klassifizieren der Tumorprobe das Clustern eines Zelltypprofils für die Tumorprobe zusammen mit Zelltypprofilen für die Vielzahl von Proben oder das Auswählen der Klasse, die mit dem Cluster assoziiert ist, das dem Zelltypprofil für die Tumorprobe am nächsten ist, umfasst; und/oder
(b) Identifizieren der Zelltypzusammensetzung der Tumorprobe durch Assoziieren von Zelltyp-Labels mit einer oder mehreren Zellen in der Tumorprobe unter Verwendung des dritten Deep-Learning-Modells, wobei gegebenenfalls das Assoziieren von Zelltyp-Labels das Erhalten eines Zelltyp-Labels und einer Vorhersagesicherheit für jeden Latentraum-Cluster oder jede Zelle unter Verwendung des dritten Deep-Learning-Modells und das Assoziieren eines Zelltyp-Labels mit einer beliebigen Zelle, für die die Vorhersagesicherheit über einem vorbestimmten Schwellenwert liegt, oder mit einer beliebigen Zelle, die zu einem Cluster gehört, für das die Vorhersagesicherheit über einem vorbestimmten Schwellenwert liegt, umfasst; und/oder
(c) Vergleichen der Genexpressionswerte eines oder mehrerer Gene in einem oder mehreren Latentraum-Clustern des ersten, zweiten und/oder dritten Deep-Learning-Modells; und/oder
(d) Verwenden des ersten, zweiten und/oder dritten Deep-Learning-Modells, um chargenkorrigierte Einzelzell-Genexpressionsprofile für die Probe zu erhalten; und/oder
(e) Identifizieren eines Gens als einen Biomarker des Ansprechens auf die Behandlung, einen Biomarker der Prognose oder ein therapeutisches Ziel basierend auf den Genexpressionswerten des Gens in einem oder mehreren Latentraum-Clustern des ersten, zweiten und/oder dritten Deep-Learning-Modells; wobei gegebenenfalls das Identifizieren eines Gens als einen Biomarker des Ansprechens auf die Behandlung oder der Prognose das Korrelieren der Expression des Gens in einem oder mehreren Latentraum-Clustern mit einer Messgröße des Ansprechens auf die Behandlung oder der Prognose umfasst; und/oder
(f) Identifizieren einer Therapie für das Individuum, von dem die Tumorprobe erhalten wurde, basierend auf der Zelltypzusammensetzung in (b), der Expression eines oder mehrerer Gene, die in (e) als ein Biomarker des Ansprechens auf die Behandlung identifiziert wurden, und/oder der Tumorlastklassifikation in (a); und/oder
(g) Auswählen eines Individuums, von dem die Tumorprobe erhalten wurde, zur Teilnahme an einer klinischen Studie basierend auf der Zelltypzusammensetzung in (b), der Expression eines oder mehrerer Gene, die in (e) als ein Biomarker des Ansprechens auf die Behandlung identifiziert wurden, und/oder der Tumorlastklassifikation in (a); und/oder
(h) Stellen einer Prognose für das Individuum, von dem die Tumorprobe erhalten wurde, basierend auf der Zelltypzusammensetzung in (b), der Expression eines oder mehrerer Gene, die in (e) als ein Biomarker des Ansprechens auf die Behandlung identifiziert wurden, und/oder der Tumorlastklassifikation in (a).

15. System, umfassend:
mindestens einen Prozessor; und
mindestens ein nichtflüchtiges computerlesbares Medium, das Anweisungen enthält, die, wenn sie von dem mindestens einen Prozessor ausgeführt werden, den mindestens einen Prozessor veranlassen, das Verfahren nach einem der Ansprüche 1 bis 14 durchzuführen.

## Revendications

1. Procédé mis en œuvre par ordinateur d'analyse d'un échantillon de tumeur comprenant des cellules tumorales et des cellules immunitaires, le procédé comprenant :
(a) l'obtention de profils d'expression génique unicellulaire pour une pluralité de cellules de l'échantillon de tumeur, les profils d'expression génique unicellulaire comprenant des mesures d'expression génique pour un ensemble de gènes ;
(b) l'utilisation d'un modèle d'apprentissage profond pour identifier une représentation respective par variables latentes des profils d'expression génique unicellulaire dans l'échantillon ; et
(c) l'identification d'un groupe respectif parmi un ou plusieurs groupes d'espaces latents de cellules auxquels appartiennent les cellules dans l'échantillon, les groupes de cellules correspondant à des cellules de différents types cellulaires et le ou les groupes de cellules comprenant au moins un groupe correspondant aux cellules tumorales et un ou plusieurs groupes de cellules correspondant à différents types cellulaires dans le microenvironnement tumoral,
dans lequel le modèle d'apprentissage profond a été obtenu par :
l'obtention d'étiquettes de types cellulaires associées à un ou plusieurs groupes de cellules dans l'espace latent d'un premier modèle d'apprentissage profond qui a été entraîné pour identifier une représentation par variables latentes de profils d'expression génique unicellulaire parmi les cellules dans des échantillons de tumeurs qui n'ont pas été purifiés pour sélectionner des cellules du microenvironnement tumoral, le ou les groupes de cellules comprenant au moins un groupe correspondant à des cellules tumorales,
l'obtention d'étiquettes de types cellulaires associées à un ou plusieurs groupes de cellules dans l'espace latent d'un deuxième modèle d'apprentissage profond qui a été entraîné pour identifier une représentation par variables latentes de profils d'expression génique unicellulaire parmi les cellules identifiées comme non malignes dans des échantillons de tumeurs qui n'ont pas été purifiés pour sélectionner des cellules du microenvironnement tumoral et/ou des cellules d'échantillons comprenant des cellules du microenvironnement tumoral purifiées, le ou les groupes de cellules correspondant à différents types cellulaires dans le microenvironnement tumoral, et
l'entraînement d'un troisième modèle d'apprentissage profond pour identifier une représentation par variables latentes de profils d'expression génique unicellulaire à l'aide des étiquettes de types cellulaires associées au ou aux groupes de cellules dans l'espace latent du premier modèle d'apprentissage profond et au ou aux groupes de cellules dans l'espace latent du deuxième modèle d'apprentissage profond.

2. Procédé selon la revendication 1, dans lequel le procédé comprend :
l'entraînement du premier modèle d'apprentissage profond à l'aide de données comprenant les profils d'expression génique unicellulaire pour la pluralité de cellules de l'échantillon de tumeur et les profils d'expression génique unicellulaire pour une pluralité de cellules d'une pluralité d'échantillons de tumeurs qui n'ont pas été purifiés pour sélectionner des cellules du microenvironnement tumoral, et/ou
l'entraînement du deuxième modèle d'apprentissage profond à l'aide de données comprenant les profils d'expression génique unicellulaire pour la pluralité de cellules de l'échantillon de tumeur et les profils d'expression génique unicellulaire pour une pluralité de cellules qui ont été identifiées comme non malignes dans une pluralité d'échantillons de tumeurs qui n'ont pas été purifiés pour sélectionner des cellules du microenvironnement tumoral et/ou pour une pluralité de cellules d'échantillons comprenant des cellules du microenvironnement tumoral purifiées, et/ou
l'entraînement du troisième modèle d'apprentissage profond à l'aide de données comprenant les profils d'expression génique unicellulaire pour la pluralité de cellules de l'échantillon de tumeur et les profils d'expression génique unicellulaire pour une pluralité de cellules associées aux étiquettes de types cellulaires associées aux groupes de cellules dans l'espace latent des premier et/ou deuxième modèles d'apprentissage profond.

3. Procédé selon la revendication 1 ou la revendication 2, dans lequel le procédé comprend :
l'entraînement du premier modèle d'apprentissage profond à l'aide de données comprenant les profils d'expression génique unicellulaire pour la pluralité de cellules de l'échantillon de tumeur et de paramètres d'un modèle d'apprentissage profond qui a été entraîné pour identifier une représentation par variables latentes de profils d'expression génique unicellulaire pour une pluralité de cellules d'une pluralité d'échantillons de tumeurs qui n'ont pas été purifiés pour sélectionner des cellules du microenvironnement tumoral, et/ou
l'entraînement du deuxième modèle d'apprentissage profond à l'aide de données comprenant les profils d'expression génique unicellulaire pour la pluralité de cellules de l'échantillon de tumeur et de paramètres d'un modèle d'apprentissage profond qui a été entraîné pour identifier une représentation par variables latentes de profils d'expression génique unicellulaire pour une pluralité de cellules qui ont été identifiées comme non malignes dans une pluralité d'échantillons de tumeurs et/ou pour une pluralité de cellules d'échantillons comprenant des cellules du microenvironnement tumoral purifiées, et/ou
l'entraînement du troisième modèle d'apprentissage profond à l'aide de données comprenant les profils d'expression génique unicellulaire pour la pluralité de cellules de l'échantillon de tumeur et de paramètres d'un modèle d'apprentissage profond qui a été entraîné pour identifier une représentation par variables latentes de profils d'expression génique unicellulaire pour une pluralité de cellules associées aux étiquettes de types cellulaires associées aux groupes de cellules dans l'espace latent des premier et/ou deuxième modèles d'apprentissage profond,
éventuellement dans lequel le modèle d'apprentissage profond est un réseau neuronal profond comprenant un ensemble de nœuds et de poids entre les nœuds, les paramètres comprennent les poids entre les nœuds, et l'entraînement utilise un apprentissage par transfert d'un modèle étendant les modèles d'apprentissage profond pour lesquels les poids sont fournis en incluant un ou plusieurs nœuds d'entrée supplémentaires avec des poids entraînables.

4. Procédé selon une quelconque revendication précédente, dans lequel les profils d'expression génique unicellulaire utilisés pour entraîner les premier et/ou deuxième et/ou troisième modèles d'apprentissage profond proviennent d'une pluralité d'échantillons dont au moins certains sont de différents types de cancers, et/ou
dans lequel les profils d'expression génique unicellulaire utilisés pour entraîner les premier et/ou deuxième et/ou troisième modèles d'apprentissage profond proviennent d'une pluralité d'échantillons qui ne comprennent pas de malignités hématologiques.

5. Procédé selon une quelconque revendication précédente, dans lequel le modèle d'apprentissage profond est un autoencodeur variationnel ou un réseau antagoniste génératif, dans lequel les premier et/ou deuxième modèles d'apprentissage profond sont des modèles non supervisés et le troisième modèle d'apprentissage profond est un modèle d'apprentissage profond semi-supervisé, éventuellement dans lequel le modèle d'apprentissage profond est un autoencodeur variationnel conditionnel (CVAE), un réseau antagoniste génératif conditionnel (cGAN), un autoencodeur variationnel par transfert (trVAE), un modèle d'interférences variationnelles unicellulaire (scVI) ou un modèle d'interférences variationnelles utilisant une annotation unicellulaire (scANVI).

6. Procédé selon une quelconque revendication précédente, dans lequel les groupes d'espaces latents sont des groupes basés sur un graphique, éventuellement dans lequel les groupes sont obtenus à l'aide de l'algorithme de Leiden, et/ou
dans lequel un type cellulaire fait référence à un ensemble de cellules ayant une morphologie, une physiologie et/ou une fonction communes, et/ou
dans lequel un type cellulaire fait référence à un type cellulaire quelconque choisi parmi : les cellules malignes, les cellules non malignes, les cellules immunitaires, les cellules stromales, les cellules cytotoxiques, les cellules prolifératives, les cellules pro-inflammatoires, les lymphocytes T, les lymphocytes T CD4+, les lymphocytes T CD8+, les lymphocytes T gamma-delta, les lymphocytes T gamma-delta 2, les lymphocytes T activés, les lymphocytes T folliculaires helper cd4+, les lymphocytes T épuisés, les lymphocytes T CD4+ épuisés, les lymphocytes T CD8+ épuisés, les lymphocytes T régulateurs épuisés, les lymphocytes T régulateurs, les cellules Th17, les lymphocytes T naïfs, les lymphocytes T CD4+ naïfs, les lymphocytes T CD8+ naïfs, les lymphocytes T prolifératifs, les lymphocytes T CD4+ prolifératifs, les lymphocytes T CD8+ prolifératifs, les lymphocytes T CD4+ prolifératifs, les lymphocytes T CD8+ prolifératifs, les lymphocytes T CD4+ activés récemment, les lymphocytes T CD4+ mémoires naïfs, les lymphocytes T CD8+ terminalement épuisés, les lymphocytes T CD8+ mémoires effecteurs, les lymphocytes T CD4+ mémoires transitionnels, les lymphocytes T CD8+ pré-épuisés, les fibroblastes, les lymphocytes B, les lymphocytes B naïfs, les lymphocytes B mémoires, les lymphocytes B prolifératifs, les plasmocytes, les cellules endothéliales, les cellules endothéliales lymphatiques, les cellules endothéliales sinusoïdales hépatiques, les cellules dendritiques, les cellules dendritiques plasmacytoïdes (pDC), les cellules dendritiques cDC1, les cellules dendritiques exprimant CLEC9A, les cellules dendritiques cDC2, les cellules dendritiques exprimant CD1C, les cellules dendritiques cDC3, les cellules dendritiques exprimant LAMP3, les cellules dendritiques myéloïdes, les cellules dendritiques de Langerhans, les cellules dendritiques folliculaires, les mastocytes, les cellules tueuses naturelles (NK), les monocytes, les macrophages, les macrophages associés à une tumeur (TAM), les TAM SPP1, les TAM M2, les macrophages alvéolaires, les monocytes, les monocytes CD14+, les monocytes CD16+, les érythrocytes, les péricytes, les kératinocytes, les mélanocytes, les cellules neuronales, les cellules musculaires lisses.

7. Procédé selon une quelconque revendication précédente, dans lequel les cellules identifiées comme non malignes dans les échantillons de tumeurs qui n'ont pas été purifiés pour sélectionner des cellules du microenvironnement tumoral ont été identifiées sur la base de la représentation par variables latentes du premier modèle d'apprentissage profond, et/ou
dans lequel le procédé comprend en outre l'identification de cellules comme non malignes dans les échantillons de tumeurs qui n'ont pas été purifiés pour sélectionner des cellules du microenvironnement tumoral sur la base de la représentation par variables latentes du premier modèle d'apprentissage profond, et/ou
dans lequel le procédé comprend en outre l'identification d'une ou plusieurs cellules dans l'échantillon de tumeur comme cellules non malignes sur la base de la représentation par variables latentes du premier modèle d'apprentissage profond.

8. Procédé selon la revendication 7, dans lequel l'identification d'une ou plusieurs cellules dans un échantillon de tumeur comme cellules non malignes sur la base de la représentation par variables latentes du premier modèle d'apprentissage profond comprend la classification d'une ou plusieurs cellules dans l'échantillon de tumeur entre une première classe correspondant à des cellules malignes et une seconde classe correspondant à des cellules non malignes par l'assignation des cellules à l'un parmi une pluralité de groupes dans l'espace latent du premier modèle d'apprentissage profond, chaque groupe étant associé à un état malin ou à un état non malin.

9. Procédé selon la revendication 8, dans lequel chaque groupe est associé à un état malin ou à un état non malin sur la base d'un score tumoral obtenu à partir de l'expression d'une pluralité de gènes associés à des cellules cancéreuses et d'une pluralité de gènes associés à des cellules immunitaires ou stromales,
éventuellement dans lequel la pluralité de gènes associés à des cellules cancéreuses sont des gènes surexprimés dans le cancer et/ou dans lequel la pluralité de gènes associés à des cellules cancéreuses comprennent un ou plusieurs parmi : EPCAM, MLANA et KRT8, et/ou
éventuellement dans lequel la pluralité de gènes associés à des cellules immunitaires ou stromales sont des marqueurs de cellules immunitaires, et/ou un ou plusieurs types de cellules stromales choisies parmi les cellules produisant du collagène, les fibroblastes, un péricyte et/ou d'origine endothéliale, et/ou dans lequel la pluralité de gènes associés à des cellules immunitaires ou stromales comprennent un ou plusieurs parmi : un marqueur de cellules immunitaires tel que PTPRC, les marqueurs de cellules produisant du collagène choisis parmi COL1A1, COL1A2, COL5A1 et LUM, un marqueur de fibroblastes tel que FBLN1, les marqueurs de péricyte choisis parmi RGS5, CNN1, MYH11, SMTN, ACTA2, TAGLN et CALD1, et les marqueurs d'origine endothéliale choisis parmi VWF et PVLAP.

10. Procédé selon la revendication 9, dans lequel le score tumoral est obtenu par :
calcul d'un score tumoral de cellule unique à partir de l'expression d'une pluralité de gènes associés à des cellules cancéreuses et d'une pluralité de gènes associés à des cellules immunitaires ou stromales,
obtention d'un score tumoral de groupe comme valeur résumée des scores tumoraux de cellule unique pour chaque groupe,
identification de chaque groupe comme malin ou non malin sur la base du score tumoral de groupe,
obtention d'une coordonnée d'espace latent résumée pour les groupes identifiés comme malins et d'une coordonnée d'espace latent résumée pour les groupes identifiés comme non malins, et
association d'un groupe à un état malin ou à un état non malin sur la base d'une distance entre le groupe et la coordonnée d'espace latent résumée pour les groupes identifiés comme malins ou non malins.

11. Procédé selon la revendication 10, dans lequel :
le score tumoral de cellule unique est obtenu par calcul, pour chaque cellule, de la différence entre une valeur d'expression résumée pour la pluralité de gènes associés à des cellules cancéreuses et une valeur d'expression résumée pour la pluralité de gènes associés à des cellules immunitaires ou stromales, éventuellement dans lequel la valeur d'expression résumée est la moyenne ou la moyenne maximale pour l'un d'une pluralité de sous-ensembles de gènes, tels que des sous-ensembles de gènes qui sont des marqueurs de cellules immunitaires ou d'un ou plusieurs types de cellules stromales, et/ou
le score tumoral de groupe est la moyenne des scores tumoraux de cellule unique pour toutes les cellules assignées à un groupe, et/ou
la coordonnée d'espace latent résumée pour les groupes identifiés comme malins/non malins est le vecteur de coordonnée d'espace latent moyen sur l'ensemble des groupes identifiés comme malins/non malins, et/ou
l'identification de chaque groupe comme malin ou non malin sur la base du score tumoral de groupe comprend la comparaison du score tumoral de groupe à un seuil identifié à l'aide de la répartition des scores tumoraux de cellule unique, éventuellement dans lequel le seuil est identifié comme le minimum local positif d'une estimation de densité par noyau de la distribution de scores tumoraux de cellule unique,
la distance est une distance euclidienne, et/ou
l'association d'un groupe à un état malin ou à un état non malin sur la base d'une distance entre le groupe et la coordonnée d'espace latent résumée pour les groupes identifiés comme malins ou non malins comprend le calcul de la distance entre : (i) la coordonnée d'espace latent moyenne pour le groupe et la coordonnée d'espace latent résumée pour les groupes identifiés comme malins et (ii) la coordonnée d'espace latent moyenne pour le groupe et la coordonnée d'espace latent résumée pour les groupes identifiés comme non malins, et l'association du groupe à un état malin si la distance de (i) est inférieure à la distance de (ii).

12. Procédé selon une quelconque revendication précédente, dans lequel le procédé comprend l'identification de cellules comme non malignes dans les échantillons de tumeurs qui n'ont pas été purifiés pour sélectionner des cellules du microenvironnement tumoral sur la base de la représentation latente à partir du premier modèle d'apprentissage profond, et l'identification des cellules malignes restantes comme cellules à potentiel tumoral élevé ou faible à l'aide d'un classificateur entraîné pour distinguer entre des cellules normales et non normales sur la base d'une ou plusieurs mesures dérivées d'une analyse de variation du nombre de copies par RNAseq, éventuellement dans lequel les mesures dérivées d'une analyse de variation du nombre de copies par RNAseq sont sélectionnées parmi : un score CNV de cellule unique, un score CNV en centile de cellule unique ou un score d'entropie de donneur de groupe pour des groupes obtenus dans un espace de scores CNV de cellule unique.

13. Procédé selon une quelconque revendication précédente, dans lequel les étiquettes de types cellulaires ont été obtenues par :
entraînement d'un premier modèle d'apprentissage profond pour identifier une représentation par variables latentes de profils d'expression génique unicellulaire de cellules dans des échantillons de tumeurs qui n'ont pas été purifiés pour sélectionner des cellules du microenvironnement tumoral,
identification des cellules non malignes et des cellules malignes sur la base de la représentation par variables latentes à partir du premier modèle d'apprentissage profond, éventuellement à l'aide du procédé selon l'une quelconque des revendications 8 à 11,
association d'une étiquette de type cellulaire à une quelconque cellule identifiée comme cellule maligne,
entraînement d'un deuxième modèle d'apprentissage profond pour identifier une représentation par variables latentes de profils d'expression génique unicellulaire de cellules identifiées comme cellules non malignes sur la base de la représentation par variables latentes à partir du premier modèle d'apprentissage profond et/ou de cellules d'échantillons comprenant des cellules du microenvironnement tumoral purifiées,
le regroupement de la représentation d'espace latent de profils d'expression génique unicellulaire du deuxième modèle d'apprentissage profond, et
l'association d'une étiquette de type cellulaire à un ou plusieurs des groupes, éventuellement dans lequel l'association d'une étiquette de type cellulaire est réalisée sur la base du niveau et/ou de la fréquence d'expression d'un ou plusieurs marqueurs pour chaque étiquette de type cellulaire dans les cellules d'un groupe, et/ou dans lequel l'association d'une étiquette de type cellulaire à un ou plusieurs des groupes comprend un nouveau regroupement du ou des groupes pour identifier d'autres groupes de sorte que l'expression d'un ou plusieurs marqueurs est plus homogène au sein des groupes supplémentaires que dans le ou les groupes initiaux.

14. Procédé selon une quelconque revendication précédente, comprenant en outre :
(a) la classification de l'échantillon de tumeur entre une pluralité de classes associées à différentes charges tumorales, la charge tumorale faisant référence à la proportion de cellules qui sont des cellules malignes par rapport aux cellules non malignes dans l'échantillon de tumeur, sur la base de la proportion de cellules dans l'échantillon de tumeur assignées à un ou plusieurs groupes d'espaces latents du premier modèle d'apprentissage profond correspondant à des cellules tumorales et éventuellement de la proportion de cellules dans les échantillons de tumeurs assignées à un ou plusieurs groupes d'espaces latents du deuxième modèle d'apprentissage profond, ou sur la base de la proportion de cellules dans l'échantillon de tumeur assignées à un groupe d'espace latent du troisième modèle d'apprentissage profond correspondant à des cellules tumorales, éventuellement la pluralité de classes comprenant une classe avec une charge tumorale supérieure à celle de toutes les autres classes, et une classe avec une charge tumorale inférieure à celle de toutes les autres classes, et/ou la pluralité de classes comprenant une classe avec une charge tumorale élevée et une classe avec une charge tumorale faible, et/ou la pluralité de classes comprenant une classe avec une charge tumorale élevée, une classe avec une charge tumorale intermédiaire et une classe avec une charge tumorale faible,
éventuellement la pluralité de classes ayant été définies par regroupement des profils de types cellulaires pour une pluralité d'échantillons, chaque profil de type cellulaire comprenant la proportion de cellules assignées à un ou plusieurs groupes d'espaces latents du premier modèle d'apprentissage profond correspondant à des cellules tumorales et éventuellement la proportion de cellules assignées à un ou plusieurs groupes d'espaces latents du deuxième modèle d'apprentissage profond, un ou plusieurs des groupes correspondant à la pluralité de classes, et la classification de l'échantillon de tumeur comprend le regroupement d'un profil de type cellulaire pour l'échantillon de tumeur conjointement avec les profils de types cellulaires pour la pluralité d'échantillons, ou la sélection de la classe associée au groupe qui est le plus proche du profil de type cellulaire pour l'échantillon de tumeur ; et/ou
(b) l'identification de la composition de type cellulaire de l'échantillon de tumeur par association des étiquettes de types cellulaires à une ou plusieurs cellules dans l'échantillon de tumeur à l'aide du troisième modèle d'apprentissage profond, éventuellement l'association des étiquettes de types cellulaires comprenant l'obtention d'une étiquette de type cellulaire et le niveau de confiance de prédiction pour chaque groupe d'espace latent ou cellule à l'aide du troisième modèle d'apprentissage profond et l'association d'une étiquette de type cellulaire à une quelconque cellule pour laquelle le niveau de confiance de prédiction est supérieur à un seuil prédéterminé ou à une quelconque cellule qui appartient à un groupe pour lequel le niveau de confiance de prédiction est supérieur à un seuil prédéterminé ; et/ou
(c) la comparaison des valeurs d'expression génique d'un ou plusieurs gènes dans un ou plusieurs groupes d'espaces latents du premier, deuxième et/ou troisième modèle d'apprentissage profond ; et/ou
(d) l'utilisation des premier, deuxième et/ou troisième modèles d'apprentissage profond pour obtenir des profils d'expression génique unicellulaire à lots corrigés pour l'échantillon ; et/ou
(e) l'identification d'un gène comme biomarqueur de réponse à un traitement, biomarqueur de pronostic ou cible thérapeutique sur la base des valeurs d'expression génique du gène dans un ou plusieurs groupes d'espaces latents du premier, deuxième et/ou troisième modèle d'apprentissage profond ; éventuellement l'identification d'un gène comme biomarqueur de réponse à un traitement ou de pronostic comprenant la corrélation de l'expression du gène dans un ou plusieurs groupes d'espaces latents avec une mesure de réponse à un traitement ou de pronostic ; et/ou
(f) l'identification d'une thérapie pour le sujet auprès duquel l'échantillon de tumeur a été obtenu sur la base de la composition de type cellulaire de (b), de l'expression d'un ou plusieurs gènes identifiés comme biomarqueur de réponse à un traitement de (e) et/ou de la classification de charge tumorale de (a) ; et/ou
(g) la sélection d'un sujet auprès duquel l'échantillon de tumeur a été obtenu pour une participation à un essai clinique sur la base de la composition de type cellulaire de (b), de l'expression d'un ou plusieurs gènes identifiés comme biomarqueur de réponse à un traitement de (e) et/ou de la classification de charge tumorale de (a) ; et/ou
(h) la fourniture d'un pronostic pour le sujet auprès duquel l'échantillon de tumeur a été obtenu sur la base de la composition de type cellulaire de (b), de l'expression d'un ou plusieurs gènes identifiés comme biomarqueur de réponse à un traitement de (e) et/ou de la classification de charge tumorale de (a).

15. Système comprenant :
au moins un processeur ; et
au moins un support non transitoire lisible par ordinateur contenant des instructions qui, lorsqu'elles sont exécutées par l'au moins un processeur, amènent l'au moins un processeur à réaliser le procédé selon l'une quelconque des revendications 1 à 14.
